# EUROPÄISCHE PATENTSCHRIFT

(11) **EP 1 379 881 B1**
(45) Veröffentlichungstag und Bekanntmachung des Hinweises auf die Patenterteilung: **06.10.2004**
(21) Anmeldenummer: 02735267.3
(22) Anmeldetag: 17.04.2002
(51) Int. Cl.: G01N 33/68

(54) **VERFAHREN ZUR BESTIMMUNG DER SUBSTRATSPEZIFITÄT EINER ENZYMATISCHEN AKTIVITÄT UND VORRICHTUNG HIERZU**
METHOD FOR DETERMINING THE SUBSTRATE SPECIFICITY OF AN ENZYMATIC ACTIVITY AND A DEVICE THEREFOR
PROCEDE DE DETERMINATION DE LA SPECIFICITE DE SUBSTRAT D'UNE ACTIVITE ENZYMATIQUE, ET DISPOSITIF Y RELATIF

(30) Priorität: 17.04.2001 DE 10118774
(43) Veröffentlichungstag der Anmeldung: 14.01.2004
(73) Patentinhaber: Jerini AG, 10115 Berlin (DE)
(72) Erfinder: SCHNEIDER-MERGENER, Jens, 10717 Berlin (DE); SCHUTKOWSKI, Mike, 06268 Zielgelroda (DE); REIMER, Ulf, 10405 Berlin (DE); DONG, Liying, 13355 Berlin (DE); PANSE, Sören, 13351 Berlin (DE); SCHARN, Dirk, 13507 Berlin (DE); OSTERKAMP, Frank, 12489 Berlin (DE); HUMMEL, Gerd, 13357 Berlin (DE); JOBRON, Laurence, 13357 Berlin (DE)
(74) Vertreter: Bohmann, Armin K., Dr.
(86) Internationale Anmeldenummer: PCT/EP2002/004265
(87) Internationale Veröffentlichungsnummer: WO 2002/083933

(56) Entgegenhaltungen:
- WO-A-00/54046
- REINEKE U ET AL: "Applications of peptide arrays prepared by the SPOT-technology." CURRENT OPINION IN BIOTECHNOLOGY. ENGLAND FEB 2001, Bd. 12, Nr. 1, Februar 2001 (2001-02), Seiten 59-64, XP002234261 ISSN: 0958-1669
- MACBEATH G ET AL: "Printing proteins as microarrays for high-throughput function determination." SCIENCE. UNITED STATES 8 SEP 2000, Bd. 289, Nr. 5485, 8. September 2000 (2000-09-08), Seiten 1760-1763, XP002234262 ISSN: 0036-8075
- "C-FIT TM technology A wide range of applications" NANO TYPE, [Online] 3. April 2001 (2001-04-03), Seiten 1-2, XP002234263 Gefunden im Internet: <URL:www.nanotype.de/index.php3?sid=b5e0ff 37ef1www.nanotype.de/index.php3?sxx_site=t echnology&sid=12c79e89f28f4e5a2082216175c5 2613> [gefunden am 2003-03-11]
- ARENKOV P ET AL: "Protein microchips: use for immunoassay and enzymatic reactions." ANALYTICAL BIOCHEMISTRY. UNITED STATES 15 FEB 2000, Bd. 278, Nr. 2, 15. Februar 2000 (2000-02-15), Seiten 123-131, XP002234264 ISSN: 0003-2697
- LEMIEUX G A ET AL: "Chemoselective ligation reactions with proteins, oligosaccharides and cells." TRENDS IN BIOTECHNOLOGY. ENGLAND DEC 1998, Bd. 16, Nr. 12, Dezember 1998 (1998-12), Seiten 506-513, XP002234265 ISSN: 0167-7799 in der Anmeldung erwähnt
- BRIAN B HAAB ET AL.: "Protein microarrays for highly parallel detection and quantitation of specific proteins and antibodies in complex solutions" GENOME BIOLOGY , [Online] Bd. 2, Nr. 2, 22. Januar 2001 (2001-01-22), Seiten 1-13, XP002234266 Gefunden im Internet: <URL:genomebiology.com/2001/2/2/research/0 004/> [gefunden am 2003-03-11]

## Beschreibung

Die vorliegende Erfindung betrifft Anordnungen von einer Vielzahl von Aminosäuresequenzen auf einer Oberfläche, diese umfassende Träger sowie Trägeranordnungen, Verfahren zur Herstellung einer derartigen Anordnung, Verfahren zur Bestimmung der Substratspezifität einer enzymatischen Aktivität, Verwendung des Verfahrens zur Bestimmung des Musters der enzymatischen Aktivität einer Probe.

Mit der zunehmenden Verfügbarkeit von Sequenzinformationen aus den verschiedenen Genomprojekten gewann die Anordnung von Nukleinsäurefragmenten mit hoher Dichte auf einem Trägermaterial, sogenannten Chips oder Biochips, eine große Bedeutung, deren volles Potential jedoch erst mit der Verfügbarkeit neuerer Synthesetechniken sowie der Miniaturisierung ausgeschöpft werden konnte und zu einer Vielzahl von Anwendungen führte. Neben Nukleinsäuren wurden Naturstoffe bzw. Bibliotheken davon, aber auch Anordnungen von Oligopeptiden und Proteinen auf derartige Chips aufgebracht. Als Trägermaterialien für diese Anordnungen wurden dabei Zellulose, Glas, Nitrozellulose, PTFE-Membranen und Spezialagar verwendet.

Mit der zunehmenden Bedeutung von Proteomics und deren biotechnologische Anwendung rücken Peptide und Proteine in den Vordergrund des Interesses. Generell sind es Proteine und meistens deren enzymatische Aktivitäten, die nahezu alle biochemischen Reaktionen innerhalb und außerhalb der Zelle ermöglichen. Die Verwendung von Anordnungen von Nukleinsäuren, mit denen entweder die Messenger-RNA (mRNA), die durch in der Zelle gerade aktive Gene generiert wurden, oder aber DNA-Kopien dieser mRNA nachgewiesen werden, sind zwar von großer Bedeutung, jedoch ist die damit erhältliche Information aus einer Reihe von Gründen nicht ausreichend, um die Vorgänge sowohl intrazellulärer wie auch extrazellulärer Prozesse zü verstehen und hiervon im Rahmen verschiedener biotechnologischer Anwendungen Gebrauch zu machen. Ein Grund dafür besteht darin, dass die Menge an mRNA in einer Zelle oft nicht mit der entsprechenden, in der Zelle produzierten Proteinmenge korreliert. Außerdem können einmal hergestellte Proteine durch geringfügige chemische Modifikationen in der Zelle (posttranslationale Modifikationen) erheblich in ihrer enzymatischen Aktivität (und damit in ihrer biologischen Funktion) beeinflußt werden. Somit besteht die Notwendigkeit, eine parallele Analyse der enzymatischen Aktivität möglichst vieler Proteine, insbesondere Enzyme, durchzuführen. Ein derartiger Ansatz erlaubt u. a. die sehr schnelle Bestimmung der Substratspezifität eines definierten Enzyms, welche wiederum eine wichtige Voraussetzung für das Design von Wissensbasierten Inhibitoren ist, oder die selektive Prüfung von Pharmaka bzw. Pharmaka-Kandidaten, insbesondere im Rahmen der Voraussage von Nebenwirkungen.

Im Stand der Technik wurden Anordnungen von Peptiden bzw. Proteinen auf verschiedenen Oberflächen wie Glas (J. Robles, M. Beltran, V. Marchan, Y. Perez, I. Travesset, E. Pedroso, A. Grandas; 1999, Towards nucleopeptides containing any trifunctional amino acid, *Tetrahedron,* **55**, 13251-13264), Zellulose (D.R. Englebretsen, D.R.K. Harding; 1994, High yield, directed immobilization of a peptide-ligand onto a beaded cellulose support, *Pept. Res.,* **7**, 322-326,), Nitrozellulose (S.J. Hawthorne, M. Pagano, P. Harriott, D.W. Halton, B. Walker; 1998, The synthesis and utilization of 2,4-dinitrophenyl-labeled irreversible peptidyl diazomethyl ketone inhibitors, *Anal. Biochem*., **261**, 131-138), PTFE-Membranen (T.G. Vargo, E.J. Bekos, Y.S. Kim, J.P. Ranieri, R. Bellamkonda, P. Aebischer, D.E. Margevich, P.M. Thompson, F.V. Bright, J.A. Gardella; 1995, Synthesis and characterization of fluoropolymeric substrata with immobilized minimal peptide sequences for cell adhesion studies 1., *J*. *Biomed. Mat. Res*., **29**, 767-778), Titanoxid (SJ. Xiao, M. Textor, ND. Spencer, M. Wieland, B. Keller, H. Sigrist; 1997, Immobilization ofthe cell-adhesive peptide ARG-GLY-ASP-CYS (RGDC) on titanium surfaces by covalent chemical attachment, *J. Materials Science-Materials in Medicine*, **8**, 867-872), Siliziumoxid (T. Koyano, M. Saito, Y. Miyamoto, K. Kaifu, M. Kato; 1996, Development of a technique for microimmobilization of proteins on silicon wafers by a streptavidin-biotin reaction, *Biotech. Progress.,* **12**, 141-144) oder Gold (B.T. Houseman, M. Meksich; 1998, Efficient solid-phase synthesis of peptide-substituted alkanethiols for the preparation of substrates that support the adhesion of cells, *J. Org. Chem.,* **63**, 7552-7555) immobilisiert oder direkt auf einer entsprechenden Glasoberflächen (S.P.A. Fodor, J.L.Read, M.C.Pirrung, L.Stryer, A.T.Lu, D.Solas; 1991, Light-directed, spatially addressable parallel chemical synthesis, *Science,* **251**, J.P. Pellois, W. Wang, X.L. Gao; 2000, Peptide synthesis based on *t*-Boc chemistry and solution photogenerated acids, *J. Comb. Chem.,* **2**, 355-360) oder auf Zellulose (R.Frank, 1992, Spot synthesis: an easy technique for the positionally addressable, parallel chemical synthesis on a membrane support, *Tetrahedron,* **48**, 9217-9232; A.Kramer und J. Schneider-Mergener, Methods in Molecular Biology, vol 87: Combinatorial Peptide Library Protocols, S. 25-39, edited by: S. Cabilly; Humana Press Inc., Totowa, NJ; Töpert, F., Oires, C., Landgraf, C., Oschkinat, H. and Schneider-Mergener, J., 2001, Synthesis of an array comprising 837 variants of the hYAP WW protein domain, *Angew. Chem*. *Int*. *Ed*., **40**, 897-900) oder auf Polypropylen (M. Stankova, S. Wade, K.S. Lam, M. Lebl; 1994, Synthesis of combinatorial libraries with only one representation of each structure, *Pept.* *Res.,* **7**, 292-298, F. Rasoul, F. Ercole, Y. Pham, C.T. Bui, Z.M. Wu, S.N. James, R.W. Trainor, G. Wickham, N.J. Maeji; 2000, Grafted supports in solid-phase synthesis, *Biopolymers,* **55**, 207-216, H. Wenschuh, R. Volkmer-Engert, M. Schmidt, M. Schulz, J. Schneider-Mergener, U. Reineke; 2000, Coherent membrane supports for parallel microsynthesis and screening of bioactive peptides, *Biopolymers,* **55**, 188-206) oder auf Chitin (W. Neugebauer, R.E. Williams, J.R. Barbier, R. Brzezinski, G. Willick; 1996, Peptide synthesis on chitin, *Int. J. Pept. Prot. Res.,* **47**, 269-275) oder auf Sepharose (W. Tegge, R. Frank, 1997, Peptide synthesis on Sepharose beads, *J. Peptides Res.,* **49**, 355-362, R. Gast, J. Glokler, M. Hoxter, M. Kiess, R. Frank, W. Tegge; 1999, Method for determining protein kinase substrate specificities by the phosphorylation of peptide libraries on beads, phosphate-specific staining, automated sorting, and sequencing, *Anal. Biochem*., **276**, 227-241) die schrittweise Synthese der Peptide durchgeführt.

Reinecke et al., Current Opinion in Biotechnology 2001, 12, 1, 59 - 64 offenbart darüberhinaus die Verwendung von Membranen auf Zellulosebasis, die alle poröse Oberflächen darstellen und in MacBeath et al., Science 2000, 289, 5485, 1760 - 1763 werden Peptide beschrieben, die auf einer Schicht aus BSA-Molekülen immobilisiert sind, wobei die BSA Moleküle nachfolgend chemisch aktiviert werden, um eine Kopplung der Peptide oder kleinen Proteine zu ermöglichen

Der vorliegenden Erfindung liegt somit die Aufgabe zugrunde, ein Mittel für das Testen von Substratspezifitäten von enzymatischen Aktivitäten bereitzustellen, welches zum einen geeignet ist, in einem System mit hohem Durchsatz verwendet zu werden, und zum anderen mit extrem geringen Mengen der enzymatischen Aktivität bzw. Probenvolumina durchgeführt werden kann. Dabei ist es insbesondere eine Aufgabe, dass das Mittel ein gegenüber den Mitteln nach dem Stand der Technik, insbesondere den darin beschriebenen Peptid- und Protein-Anordnungen, dort auch als "Arrays" bezeichnet, verbessertes Signal-Rausch-Verhältnis aufweist.

Eine weitere Aufgabe der vorliegenden Erfindung besteht darin, ein Verfahren zur Herstellung derartiger Mittel bereitzustellen sowie Verfahren zur Bestimmung der Substratspezifität einer enzymatischen Aktivität und Verfahren zur Bestimmung der Selektivität eines Wirkstoffes.

Diese Aufgabe wird erfindungsgemäß gelöst durch Verfahren zur Bestimmung der Substratspezifität einer enzymatischen Aktivität umfassend die folgenden Schritte:
- Bereitstellen einer Anordnung umfassend eine Vielzahl von Aminosäuresequenzen auf einer planaren Oberfläche eines Trägermaterials, wobei die Aminosäuresequenzen gerichtet immobilisiert sind,
- Kontaktieren und/oder Inkubieren einer enzymatischen Aktivität mit der Anordnung, und
- Nachweis einer Reaktion zwischen einer der auf der Anordnung immobilisierten Aminosäuresequenzen und der enzymatischen Aktivität,
wobei vorgesehen ist, dass bei der Umsetzung der enzymatischen Aktivität mit der Anordnung eine Änderung des Molekulargewichts mindestens einer der Aminosäuresequenzen erfolgt.

In einer Ausführungsform ist vorgesehen, dass der Nachweis der Reaktion an der oder unter Verwendung der auf der Oberfläche des Trägermaterials immobilisierten Aminosäuresequenz erfolgt.

In einer weiteren Ausführungsform ist vorgesehen, dass die Änderung des Molekulargewichts durch Ausbildung oder Spaltung einer kovalenten Bindung an einer der Aminosäuresequenzen erfolgt, bevorzugterweise an derjenigen Aminosäuresequenz, die mit der enzymatischen Aktivität reagiert.

In einer noch weiteren Ausführungsform ist vorgesehen, dass der Nachweis der Reaktion durch Detektion der Änderung des Molekulargewichts erfolgt.

Schließlich ist in einer Ausführungsform vorgesehen, dass der Nachweis durch ein Nachweisverfahren erfolgt, das ausgewählt ist aus der Gruppe, die Autoradiographie, Plasmonresonanzspektroskopie und Fluoreszenzspektroskopie umfasst.

In einer Ausführungsform ist vorgesehen, dass mindestens eine der Aminosäuresequenzen ein Substrat für eine enzymatische Aktivität ist.

In einer weiteren Ausführungsform ist vorgesehen, dass die Anordnung von Aminosäuresequenzen für mindestens zwei verschiedene enzymatische Aktivitäten jeweils mindestens ein Substrat aufweist.

In einer bevorzugten Ausführungsform ist vorgesehen, dass die enzymatische Aktivität ausgewählt ist aus der Gruppe, die Kinasen, Sulfotransferasen, Glycosyltransferasen, Acetyltransferasen, Farnesyltransferasen, Palrnityltransferasen, Phosphatasen, Sulfatasen, Esterasen, Lipasen, Acetylasen und Proteasen umfasst.

In einer weiteren Ausführungsform ist vorgesehen, dass der Nachweis einer Reaktion zwischen den auf der Anordnung immobilisierten Aminosäuresequenzen und der enzymatischen Aktivität mehrfach, bevorzugterweise in zeitlichen Abständen, wiederholt wird.

In einer noch weiteren Ausführungsform ist vorgesehen, dass die enzymatische Aktivität in einer Probe bestimmt wird und die Probe bevorzugterweise ausgewählt ist aus der Gruppe, die Urin, Liquor, Sputum, Stuhl, Lymphflüssigkeit, Zellysate, Gewebelysate, Organlysate, Extrakte, Rohextrakte, gereinigte Präparate und ungereinigte Präparate umfasst.

Zur erfindungsgemässen Lösung der Aufgaße ist es notwending, dass die Oberfläche eine nicht-poröse Oberfläche ist.

In einer weiteren Ausführungsform ist vorgesehen, dass das Trägermaterial Glas ist.

In einer noch weiteren Ausführungsform ist vorgesehen, dass die Aminosäuresequenz über eine Schwefel umfassende Gruppe an der Oberfläche immobilisiert ist.

In einem zweiten Aspekt wird die Aufgabe gelöst durch eine Anordnung von einer Vielzahl von Aminosäuresequenzen auf einer Oberfläche, bevorzugterweise auf der Oberfläche eines Festphasenträgers, wobei die Aminosäuresequenzen gerichtet auf der planaren Oberfläche eines Trägermaterials immobilisiert sind, wobei mindestens eine der Aminosäuresequenzen ein Substrat für eine enzymatische Aktivität darstellt, wobei am Substrat durch die enzymatische Aktivität eine Änderung des Molekulargewichts erfolgt.

In einer Ausführungsform ist vorgesehen, dass die Änderung des Molekulargewichts durch Ausbildung oder Spaltung einer kovalenten Bindung am Substrat erfolgt ist.

In einer weiteren Ausführungsform ist vorgesehen, dass die Anordnung von Aminosäuresequenzen für mindestens zwei verschiedene enzymatische Aktivitäten jeweils mindestens ein Substrat aufweist.

In einer noch weiteren Ausführungsform ist vorgesehen, dass die planare Oberfläche eine nicht-poröse Oberfläche ist.

In einer Ausführungsform ist vorgesehen, dass das Trägermaterial ausgewählt ist aus der Gruppe, die Silikate, Keramik, Glas, Metalle und organische Trägermaterialien umfasst.

In einer weiteren Ausführungsform ist vorgesehen, dass die Aminosäuresequenzen ausgewählt sind aus der Gruppe, die Peptide, Oligopeptide, Polypeptide und Proteine sowie deren jeweilige Derivate umfasst.

In einer noch weiteren Ausführungsform ist vorgesehen, dass eine jede Aminosäuresequenz oder Gruppe von Aminosäuresequenzen relativ zu einer anderen Aminosäuresequenz oder Gruppen von Aminosäuresequenzen definiert angeordnet ist.

In einem weiteren Aspekt wird die Aufgabe erfindungsgemäß gelöst durch einen Träger umfassend eine erfindungsgemäße Anordnung.

In einer Ausführungsform ist vorgesehen, dass der Träger ein Basisträgermaterial umfasst.

In einer weiteren Ausführungsform ist vorgesehen, dass die Anordnung einer Vielzahl von Aminosäuresequenzen auf einer oder mehreren der Oberflächen des Trägers angeordnet ist.

In einem weiteren Aspekt wird die Aufgabe gelöst durch eine Trägeranordnung umfassend mindestens zwei der erfindungsgemäßen Träger, wobei jeweils zwei Träger durch einen Spalt getrennt sind.

In einer Ausführungsform ist vorgesehen, dass zumindest eine Anordnung auf einem ersten Träger mindestens einer Anordnung auf einem zweiten Träger zugewandt ist.

In einer weiteren Ausführungsform ist vorgesehen, dass der Spalt eine Breite etwa von 0,01 mm bis 10 mm, bevorzugterweise etwa von 0,1 mm bis 2 mm und bevorzugtererweise etwa von 0,5 mm bis 1 mm aufweist.

In einem noch weiteren Aspekt wird die Aufgabe erfindungsgemäß gelöst durch die Verwendung der erfindungsgemäßen Anordnung und/oder eines erfindungsgemäßen Trägers und/oder einer erfindungsgemäßen Trägeranordnung in einem der erfindungsgemäßen Verfahren.

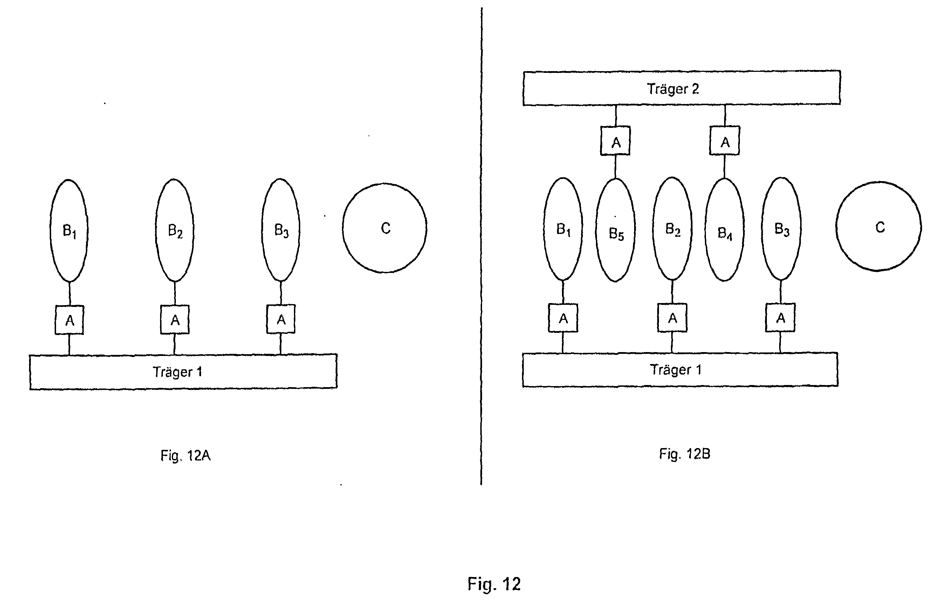

Der vorliegenden Erfindung liegt die überraschende Erkenntnis zugrunde (vgl. Fig. 12A), dass mit einer Anordnung von einer Vielzahl von Aminosäuresequenzen (Fig. 12A, B₁-B₃) auf einer Oberfläche, wobei insbesondere vorgesehen ist, dass die Aminosäuresequenzen gerichtet auf der Oberfläche immobilisiert sind und die Oberfläche eine planare Oberfläche ist, bei Kontaktieren der Anordnung mit einer Probe, die einen potentiellen Wechselwirkungspartner (Fig. 12A, C) für eine oder mehrere der in der Anordnung umfassten Aminosäuresequenzen enthält, sehr geringe Mengen des potentiellen Wechselwirkungspartners, ausgedrückt als internationale Einheiten / Flüssigkeitsvolumen, ausreichen, um ein Bindungsereignis zwischen einer oder mehreren der Aminosäuresequenzen und dem potentiellen Wechselwirkungspartner nachweisen zu können. Bei dem potentiellen Wechselwirkungspartner handelt es sich bevorzugterweise um eine enzymatische Aktivität und bei dem Bindungsereignis um die Ausbildung des für eine katalytische Reaktion erforderlichen Komplexes aus enzymatisch aktivem Protein und - otentiellem - Substrat. Mit anderen Worten, die erfindungsgemäße Anordnung erlaubt eine Verbesserung des Signal-Rausch-Verhältnisses um mehrere Größenordnungen gegenüber den Anordnungen nach dem Stand der Technik, die auf der speziellen Kombination der Merkmale der gerichteten Immobilisierung dem Vorhandensein einer planaren Oberfläche beruht.

Bei der Verwendung von porösen Oberflächen, wie dies beispielsweise bei der Verwendung von Zellulose oder porösem Glas der Fall ist, kann zwar eine sehr große Menge an Material, im vorliegenden Falle von Aminosäuresequenzen pro Flächeneinheit immobilisiert werden, was zu guten Signal-Intensitäten und großen Bereichen mit einem proportionalen Messignal führt, jedoch wird gleichzeitig mit der Verfügbarkeit der großen Oberfläche eine unspezifische Wechselwirkung der Aminosäuresequenzen mit dem Trägermaterial bedingt, was zu höheren Hintergrundsignalen führt. Weiterhin erfordern derartige poröse Oberflächen wesentlich mehr Material für die Ausgestaltung der Anordnung bzw. für die Beschichtung eines die Anordnung tragenden Trägermaterials, d.h. größere Mengen an einer jeden der verschiedenen Aminosäuresequenzen. Ebenfalls bedingt durch die poröse Oberfläche wird mehr Probenmaterial für den eigentlichen Analyseprozeß benötigt. Bei dem Probenmaterial handelt es sich um solches Material, das einen möglichen Wechselwirkungspartner für eine oder mehrere der Aminosäuresequenzen enthält. Dieses Mehr an Probenmaterial ist jedoch nicht in einem jeden Fall dadurch zu kompensieren, dass eine größeres Probenvolumen aufgegeben wird, sondern vielmehr kann es erforderlich sein, dass die spezifische Menge des potentiellen Wechselwirkungpartners in der Probe, die mit der Anordnung kontaktiert wird oder kontaktiert werden soll, erhöht werden muß. Dies würde ein Aufreinigen des zu analysierenden Probenmaterials erfordern, wobei jedoch bei derartigen Aufreinigungen oftmals nicht unerhebliche Verluste auftreten, so dass die Verwendung von porösen Oberflächen bei Anordnungen von Molekülen auf Oberflächen nicht geeignet ist, Wechselwirkungspartner nachzuweisen, deren Konzentration in einer Probe vergleichsweise gering ist. Handelt es sich bei dem potentiellen Wechselwirkungspartner um eine enzymatische Aktivität (was hierin Enzyme und jegliche katalytisch aktiven Moleküle, bspw. auch katalytisch aktive Nukleinsäuren, allgemein einschließt), so kann sich unter dem Einfluß der bei Verwendung von Anordnungen nach dem Stand der Technik erforderlichen Aufreinigung oder Konzentrierung des Probenmaterials oder des Wechselwirkungspartners, d.h. der spezifischen enzymatischen Aktivität, die Situation ergeben, dass bestimmte enzymatische Aktivitäten nicht ermittelt werden können. Dies stellt insoweit eine erhebliche Beschränkung der Verwendung von Aminosäuresequenzen umfassenden Anordnungen dar, als dass oftmals jene enzymatischen Aktivitäten von zentraler biologischer Bedeutung sind, die mengenmäßig in einer Probe nicht notwendigerweise vorherrschend sind. Mit der erfindungsgemäßen Anordnung lassen sich somit beispielsweise Zellaufschlüsse ohne weitere Bearbeitung im Sinne einer Aufarbeitung analysieren und darin enthaltene enzymatischen Wechselwirkungspartner mit einer geringen spezifischen Aktivität nachweisen.

Ein weiterer Nachteil der Verwendung von porösen Oberflächen besteht darin, dass dort zwangsläufig Kapillarkräfte wirken, die einer Miniaturisierung, wie sie insbesondere für high throughput-Systeme erforderlich ist, entgegenstehen. Mit anderen Worten, bei Verwendung von porösen Trägersystemen ist nur eine bestimmte Dichte von Aminosäuresequenzen in einer Anordnung realisierbar. Derzeit scheint diese Grenze infolge der der Porosität zugrundeliegenden physikochemischen Eigenschaften im Fall von Zellulose bei 100/cm² zu liegen.

Andererseits ist jedoch die Verwendung von planaren Oberflächen alleine wiederum nicht geeignet, eine Anordnung bereitzustellen, die in den Bereich der mit der erfindungsgemäßen Anordnung erzielbaren Signalintensitäten und insbesondere den Signal-Rausch-Verhältnissen vorzustoßen, da hier die Beladungskapazität oftmals der limitierende Faktor ist. Versuche, diese Limitierungen zu umgehen, indem auf die planare, nicht poröse Oberfläche, Polyacrylamidgele mit definierter Porenweite aufgebracht wurden, führten nicht zu dem erwünschten Erfolg, da hier die genannten Nachteile der porösen Membranen nachträglich wieder eingeführt wurden.

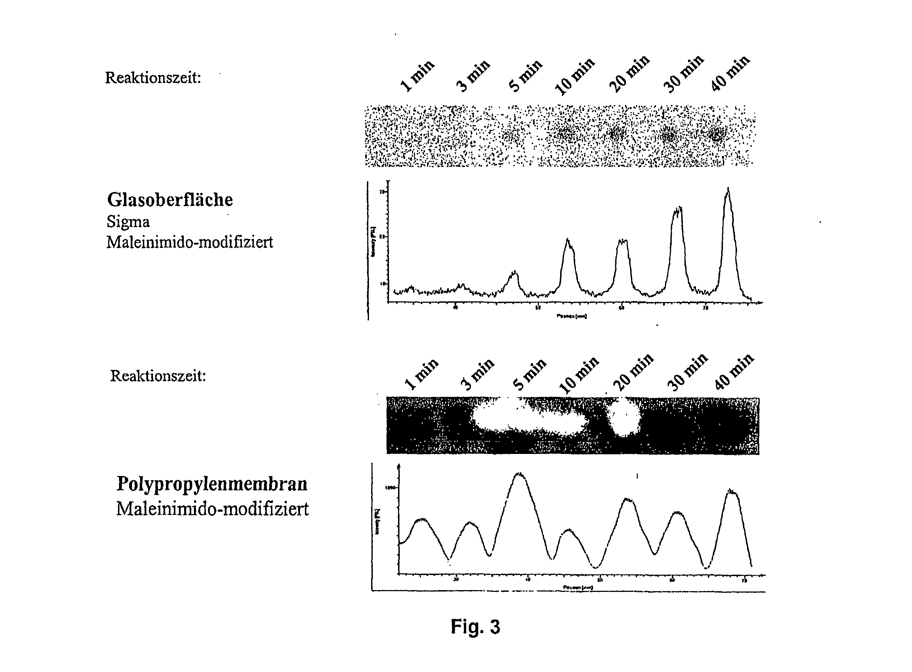

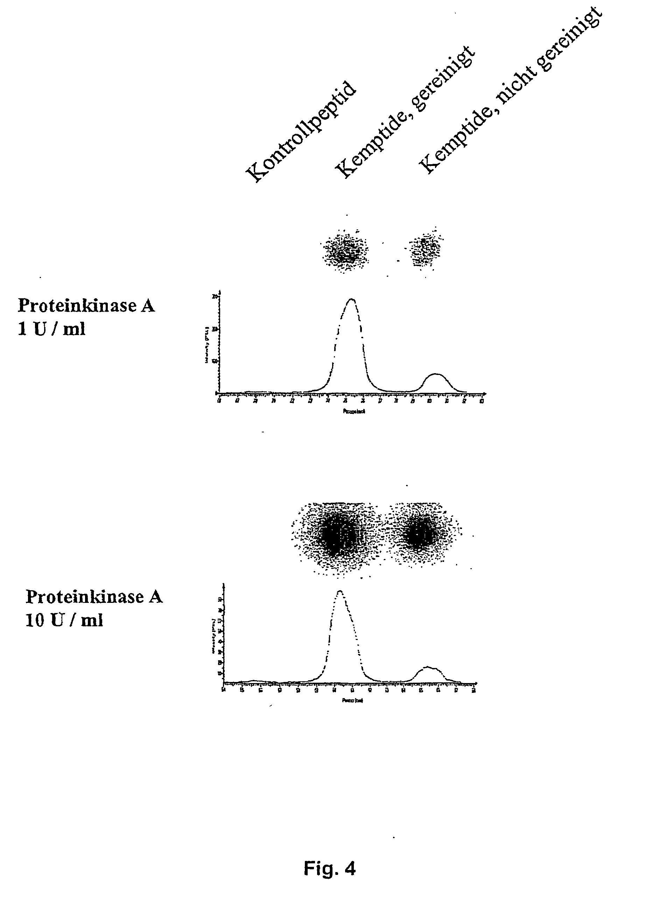

Mit der vorliegenden Erfindung wird ein Weg bei der Konstruktion von Anordnungen von einer Vielzahl von Aminosäuresequenzen auf einer Oberfläche beschritten, der nicht nur auf den Oberflächenaspekt, sondern auch auf die spezifische Art der Immobilisierung der auf der Anordnung enthaltenden Aminosäuresequenzen abstellt und damit deren überraschende Leistungsfähigkeit begründet. Dabei erfordert die planare Oberfläche lediglich eine vergleichsweise geringe Menge der verschiedenen Aminosäuresequenzen, die darüber hinaus infolge ihrer gerichteten Immobilisierung auf der Oberfläche optimale Wechselwirkungspartner, insbesondere Substrate für enzymatische Aktivitäten, darstellen, so dass trotz der infolge der glatten, d.h. bevorzugterweise nicht-porösen Oberfläche vergleichsweise geringen Beladungskapazität dennoch signifikante Signale erzielt werden und, ebenfalls bedingt durch die planare Oberfläche, es zu keiner unspezifischen Absorption und damit zu einer Verschlechterung des Signal-Rausch-Verhältnisses kommt. Wie anhand der in den Beispielen angegebenen Modellrechnung dargestellt wird, kommt es durch die Kombination dieser beiden Merkmale der erfindungsgemäßen Anordnung zu einer Verbesserung des Signal-Rausch-Verhältnisses um den Faktor 3000, wie dies auch aus den Fig. 3 und 4 ersichtlich ist.

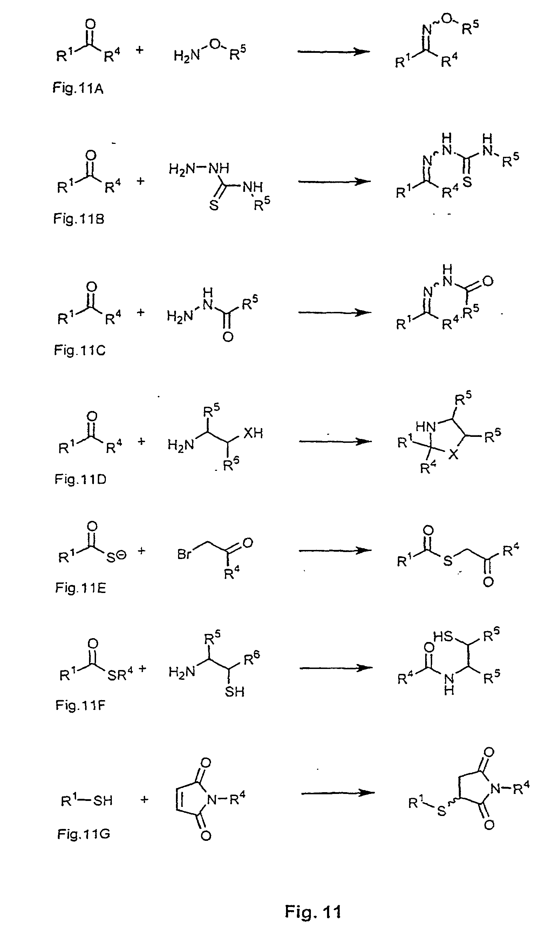

Bei der erfindungsgemäßen Anordnung einer Vielzahl von Aminosäuresequenzen auf einer Oberfläche fungiert die Oberfläche gewissermaßen als Substrat, auf dem die Vielzahl von Aminosäuresequenzen immobilisiert ist. Die Immobilisierung kann dabei so erfolgen, dass sie kovalent erfolgt. Neben der kovalenten Immobilisierung sind jedoch andere Formen der Immobilisierung, insbesondere der adsorptiven Immobilisierung oder der Immobilisierung über-spezifische Wechselwirkungssysteme möglich. Bei der Immobilisierung besonders bevorzugt ist die kovalente Immobilisierung, wobei hier eine chemoselektive Bindung der Aminosäuresequenz an die Oberfläche des Trägermaterials erfolgt. Hierzu können eine Reihe von Reaktionen verwendet werden, die dem Fachmann auf diesem Gebiet als solche bekannt sind (Lemieux, G.A. & Bertozzi, C.R., 1998, Chemoselective ligation reactions with proteins, oligosaccharides and cells, *TIBTECH*, **16**, 506-513, vgl. hierzu Fig. 11). Grundsätzlich soll mit Blick auf die erforderliche gerichtete Immobilisierung sichergestellt werden, dass unter den jeweiligen Reaktionsbedingungen im wesentlichen nur eine spezielle Verbindung zwischen der Aminosäuresequenz und der Oberfläche hergestellt wird (Fig 12, Linker A). Die Auswahl der reaktiven Gruppe auf seiten der Aminosäuresequenzen wird somit im wesentlichen von der Einzelsequenz abhängen. Alternativ ist es im Rahmen der vorliegenden Erfindung, dass eine allen Aminosäuresequenzen einheitliche terminale Struktur vorgesehen wird und diese terminale Struktur für die spezifische Reaktion mit der Oberfläche, insbesondere einer aktivierten Oberfläche, zur Verfügung gestellt wird (Fig 12, Linker A). Typischerweise werden bei den chemoselektiven Reaktionen in der Aminosäuresequenz enthaltene Amino- bzw. Carboxylgruppen nicht beeinträchtigt. Beispiele für geeignete Reaktionen sind die Bildung von Thioethern aus Halo-Carbonsäuren und Thiolen, von nen sind die Bildung von Thioethern aus Halo-Carbonsäuren und Thiolen, von Thioethern aus Thiolen und Maleinimiden, von Amidbindungen aus Thioestern und 1,2-Aminothiolen, von Thioarnidbindungen aus Dithioestern und 1,2-Aminothiolen, von Thiazolidinen aus Aldehyden und 1,2-Aminothiolen, von Oxazolidinen aus Aldehyden/Ketonen und 1,2-Aminoalkoholen, von Imidazolen aus Aldehyden/Ketonen und 1,2-Diaminen, (vgl. hierzu auch Fig. 11) von Thiazolen aus Thioamiden und alpha-Halo-Ketonen, von Aminothiazolen aus Amino-oxy-Verbindungen und alpha-Isothiocyanato-Ketonen, von Oximen aus Amino-oxy-Verbindungen und Aldehyden, von Oximen aus Amino-oxy-Verbindungen und Ketonen, von Hydrazonen aus Hydrazinen und Aldehyden, von Hydrazonen aus Hydraziden und Ketonen. Dabei können die in Fig. 11 aufgezeigten Reste R1-R5 bzw. die Reste in den oben aufgeführten chemoselektiven Reaktionen Alkyl-, Alkenyl-, Alkinyl-, Cycloalkyl- oder Aryl-Reste, bzw. Heterocyclen sein, wobei Alkyl für verzweigte und unverzweigte C₁₋₂₀-Alkyl, C₃₋₂₀-Cycloalkyl, bevorzugt für verzweigte und unverzweigte C₁₋₁₂-Alkyl, C₃₋₁₂-Cycloalkyl und besonders bevorzugt für verzweigte und unverzweigte C₁₋₆-Alkyl, C₃₋₆-Cycloalkyl-Reste steht. Alkenyl steht für verzweigte und unverzweigte C₂₋₂₀-Alkenyl, verzweigte und unverzweigte C₁₋₂₀-Alkyl-O-C₂₋₂₀-alkenyl, C₁₋₂₀(-O/S-C₂₋₂₀)₂₋₂₀alkenyl, Aryl-C₂₋₂₀-alkenyl, verzweigte und unverzweigte Heterocyclyl- C₂₋₂₀-alkenyl, C₃₋₂₀-Cycloalkenyl, bevorzugt für verzweigte und unverzweigte C₂₋₁₂-Alkenyl, verzweigte und unverzweigte C₁₋₁₂ (-O/S-C₂₋₁₂)₂₋₁₂alkenyl, besonders bevorzugt für verzweigte und unverzweigte C₂₋₆-Alkenyl, verzweigte und unverzweigte C₁₋₆ (-O/S-C₂₋₈)₂₋₈alkenyl-Reste; Alkinyl steht für verzweigte und unverzweigte C₂₋₂₀-Alkinyl, verzweigte und unverzweigte C₁₋₂₀ (-O/S-C₂₋₂₀)₂₋₂₀alkinyl, bevorzugt für verzweigte und unverzweigte C₂₋₁₂-Alkinyl, verzweigte und unverzweigte C₁₋₁₂ (-O/S-C₂₋₁₂)₂₋₁₂alkinyl, besonders bevorzugt für verzweigte und unverzweigte C₂₋₆-Alkinyl, verzweigte und unverzweigte C₁₋₆ (-O/S-C₂₋₈)₂₋₈alkinyl-Reste; Cycloalkyl steht für überbrückte und nicht-überbrückte C₃₋₄₀-Cycloalkyl, bevorzugt für überbrückte und nicht-überbrückte C₃₋₂₆-Cycloalkyl, besonders bevorzugt für überbrückte und nicht-überbrückte C₃₋₁₅-Cycloalkyl-Reste, Aryl steht für substituierte und unsubstituierte mono-oder multi-verknüpfte Phenyl-, Pentalenyl-, Azulenyl-, Anthracenyl-, Indacenyl-, Acenaphtyl, Fluorenyl, Phenalenyl, Phenanthrenyl, bevorzugtfür substituierte und unsubstituierte mono-oder multi-verknüpfte Phenyl-, Pentalenyl-, Azulenyl-, Anthracenyl-, Indenyl-, Indacenyl-, Acenaphtyl-, Fluorenyl, besonders bevorzugt fiir substituierte und unsubstituierte mono-oder multi-verknüpfte Phenyl-, Pentalenyl-, Anthracenyl-Reste, sowie deren teilhydrierte Derivate. Heterocyclen können sein ungesättigte und gesättigte 3-15-gliedrige mono-bi und tricyclische Ringe mit 1-7 Heteroatomen, bevorzugt 3-10-gliedrige mono-bi und tricyclische Ringe mit 1-5 Heteroatomen und besonders bevorzugt: 5-, 6- und 10-gliedrige mono-, bi und tricyclische Ringe mit 1-3 Heteroatomen.

Zusätzlich kann am Alkyl, Alkenyl, Alkinyl, Cycloalkyl, A. , ryl, Heteroatome, Heterocyclen, Biomoleküloder Naturstoff 0 bis 30 (bervorzugt 0 bis 10, besonders bevorzugt 0 bis 5) der folgende Substituent einzeln oder in Kombination untereinander auftreten; Fluor, Chlor, Brom, Iod, Hydroxyl, Amid, Ester, Säure, Amin, Acetal, Ketal, Thiol, Ether, Phosphat, Sulfat, Sulfoxyd, Peroxyd, Sulfonsäure, Thioether, Nitril, Harnstoffe, Carbamat, wobei folgende bevorzugt sind: Fluor, Chlor, Brom, Hydroxyl, Amid, Ester, Säure, Amin, Ether, Phosphat, Sulfat, Sulfoxyd, Thioether, Nitril, Harnstoffe, Carbamat,und besonders bevorzugt: Chlor, Hydroxyl, Amid, Ester, Säure, Ether, Nitril

Unter gerichteter Immobilisierung soll hierin insbesondere verstanden werden, dass j ede Aminosäuresequenz über eine definierte reaktive Gruppe oder Ansammlung reaktiver Gruppen an die Oberfläche gebunden wird. Infolge dieser Bindungsspezifität wird erreicht, dass sich im Rahmen der üblichen Entropien die einzelnen Aminosäuresequenzen in einem energetisch bevorzugten Zustand befinden werden, so dass die solchermaßen immobilisierten Aminosäuresequenzen in weitgehend ähnlichen Sekundär- und Tertiärstrukturen vorliegen.

Unter dem Begriff "Anordnung von einer Vielzahl von Aminosäuresequenzen" wird hierin insbesondere verstanden, dass eine jede Aminosäuresequenz an einem bestimmten Ort auf einer Oberfläche immobilisiert ist. Bevorzugterweise kann dabei jeder dieser Orte identifiziert werden. Bei den Orten handelt es sich somit um distinkte Orte, an denen jeweils im wesentlichen eine Spezies von Aminosäuresequenz immobilisiert ist. Mit anderen Worten, es existiert eine Karte, aus der die Lage einer jeden der immobilisierten Aminosäuresequenzen auf der Oberfläche abgeleitet werden kann. Die einzelne Aminosäuresequenz kann dabei eine Vielzahl von Molekülen darstellen, die jedoch hinsichtlich ihrer Aminosäuresequenz, d.h. der Art und Abfolge der sie aufbauenden Aminosäuren im wesentlichen identisch sind. Die Identität der Aminosäuresequenzen wird im wesentlichen durch das Herstellungsverfahren der Aminosäuresequenzen bestimmt. Es ist im Rahmen der vorliegenden Erfindung, dass die Aminosäuresequenzen *in situ* auf der Oberfläche der Anordnung synthetisiert werden, wobei hier alle möglichen Formen denkbar sind, d. h. sequentielles Anfügen der die Aminosäuresequenz aufbauenden einzelnen Aminosäuren, ebenso wie die Verwendung von Block-Synthesetechniken, bei denen Gruppierungen von Aminosäuren zusammengefügt werden und dann die einzelnen Blöcke sequentiell aneinandergereiht werden und die Blöcke oder Aneinanderreihungen davon sodann immobilisiert werden bzw. an bereits immobilisierte Aminosäuresequenzen angefügt werden.

Es ist für den Fachmann verständlich, dass infolge der nicht immer vollständigen Ausbeuten der einzelnen Syntheseschritte bzw. Kopplungsschritte gewisse Heterogenitäten in den verschiedenen Aminosäuresequenzen im vorstehend beschriebenen Sinne entstehen können. Insbesondere bei Synthesen, die viele Reaktionsschritte erfordern, wie dies bei der Synthese von Amiosäuresequenzen der Fall ist (pro Aminosäurebaustein eine Kupplungsreaktion und eine Schutzgruppen-Abspaltung und am Ende der Synthese im allgemeinen eine Reaktion für die simultane Abspaltung aller Schutzgruppen der Seitenkettenfunktionen) kann das ein Problem darstellen. So ist zum Beispiel bei der Synthese einer Aminosäuresequenz, die aus 20 Aminosäurebausteinen oder 40 Aminosäurebausteinen besteht, und einer angenommenen durchschnittlichen Ausbeute von 95% für die nötigen 41 bzw. 81 Reaktionsschritte die zu erwartente theoretische Ausbeute nur noch 0.95⁴¹ = 0.122 (12.2 %) bzw. 0.95⁸¹ = 0.0157 (1.57 %). Selbst bei einer angenommen durchschnittlichen Ausbeute von 99 % ergeben sich für die oben aufgeführten Beispiele nur 66.2 % bzw. 44.3 %. Somit wird klar, daß bei den zu untersuchenden enzymatischen Umsetzungen infolge dieser Limitierungen in großer Vielzahl neben der gewünschten Aminosäuresequenz weitere Aminosäuresequenzen vorhanden sind, die sich durch das Fehlen einer oder mehrerer Aminosäurebausteine auszeichnen. Genau diese, dem Fachmann als Fehl- bzw. Rumpfsequenzen bekannten Nebenprodukte, können unter Umständen das Ergebnis der Inkubation mit einer, die auf der Oberfläche angeordneten Aminosäuresequenzen modifizierenden, enzymatischen Aktivität stark verfälschen bzw. die Interpretation der Ergebnisse erschweren. Zum Beispiel können bei der Immobilisierung eines Substrates für eine Kinase auf oder an einer Oberfläche unter Nutzung der innerhalb dieses Substrates enthaltenen Aminogruppen mehrere (abhängig von der Anzahl der in der zu immobilisierenden Verbindung vorhandenen Aminogruppen) Möglichkeiten der Reaktion bestehen und damit der endgültigen Orientierung der Verbindung auf der Oberfläche. Werden dabei bei der nachgeschalteten Inkubation dieser immobilisierten Verbindung (Kinasesubstrat) mit einer, mindestens eine Kinaseaktivität enthaltenden, biologischen Flüssigkeit eben eine oder mehrere dieser Aminogruppen für die effektive Ausbildung eines Emzym/Substrat/Komplexes benötigt, kann eine solche unspezifische Immobilisierung dazu führen, dass nur eine geringe Population der immobilisierten Substrate in der richtigen Art und Weise verankert ist und damit das Meßsignal unterhalb der Nachweisgrenze liegt. Damit ist eine spezifische oder auch gerichtete Immobilisierung von großem Vorteil. Hier erfolgt in einem Immobilisierungsereignis der Kontakt zwischen der zu immobilisierenden Verbindung und der Oberfläche, auf bzw. an der die Verbindnung immobilisiert wird, jeweils in der gleichen Art und Weise und alle Verbindungen sind in einer definierten und vorhersagbaren Orientierung auf oder an der Oberfläche gebunden.

Die Vielzahl von Aminosäuresequenzen besteht aus mindestens zwei unterschiedlichen Aminosäuresequenzen. Es kann vorgesehen sein, dass die an einer distinkten Stelle immobilisierte Aminosäuresequenz sich an anderer Stelle der Oberfläche wiederfindet. Dies kann beispielsweise zu Kontrollzwecken erfolgen.

Bei der planaren Oberfläche kann es sich um eine solche handeln, die im wesentlichen zweidimensional ausgerichtet ist. Insbesondere ist gemäß der vorliegenden Erfindung nicht vorgesehen, dass die eine Vielzahl von Aminosäuresequenzen tragende Oberfläche eine sphärische Oberfläche darstellt oder einen wesentlichen Teil einer solchen. Bei der Ausgestaltung der planaren Oberfläche ist dabei bevorzugt, dass die distinkten Orte, an denen jeweils eine einzelne Aminosäuresequenz immobilisiert ist, nicht oder zumindest nicht wesentlich durch eine dreidimensionale Struktur von einem anderen distinkten Ort auf der Oberfläche getrennt ist.

Als Materialien für die Oberfläche bzw. als Trägermaterialien, die die erfindungsgemäßen Anordnungen tragen können, können im Rahmen der vorliegenden Erfindung alle bioverträglichen, funktionalisierten bzw. funktionalisierbaren Materialien verwendet werden. Diese Materialien können beispielsweise als feste Trägerplatten (monolithische Blöcke), Membranen, Filme oder Laminate vorliegen. Geeignete Materialien sind dabei Polyolefine, wie z. B. Polyethylen, Polypropylen, halogenierte Polyolefine (PVDF, PVC, etc.) sowie Polytetrafluoroethylen. Auf seiten der anorganischen Materialien können beispielsweise Keramik, Silikate, Silizium und Glas verwendet werden. Obwohl nicht-metallische Trägerplatten bevorzugt sind, ist es jedoch auch im Umfang der vorliegenden Erfindung, metallische Trägermaterialien zu verwenden, trotz deren Tendenz zur Ausbildung potentiell unspezifischer Adsorptionseffekte. Beispiele für derartige Materialien sind Gold oder Metalloxide, wie beispielsweise Titanoxid.

Unabhängig von dem tatsächlich ausgewählten Material, wobei Glas besonders bevorzugt ist, ist es für die vorliegende Erfindung essentiell, dass die Oberfläche nicht-poröser Natur ist und Kapillarkräfte an der Oberfläche nicht bzw. im wesentlichen nicht auftreten.

Bei der Ausgestaltung der Anordnung bestehen bei der Gestaltung der Oberfläche im eigentlichen Sinne, d.h. der die Vielzahl von Aminosäuresequenzen tragenden planaren Oberfläche, eine Reihe von Möglichkeiten. Es ist grundsätzlich möglich, dass die Oberfläche, an der die gerichtete Immobilisierung der Aminosäuresequenzen erfolgt, gleichzeitig das Trägermaterial darstellt. Es ist jedoch auch möglich, dass die reaktive Oberfläche verschieden ist von dem Trägermaterial. Ein derartiges Szenario ist beispielsweise dann gegeben, wenn das die (planare) Oberfläche ausbildende Material in Form eines Films vorliegt, der dann, nicht zuletzt zu Stabilisierungszwecken, auf einem weiteren Basisträgermaterial aufgebracht ist.

Zu Zwecken der gerichteten Immobilisierung, insbesondere wenn diese durch kovalente Bindung der Aminosäuresequenzen auf einem Trägermaterial erfolgt, kann die Oberfläche der Trägerplatte funktionalisiert werden. Grundsätzlich sind mehrere aufeinanderfolgende Funktionalisierungen möglich, es kann jedoch in Abhängigkeit vom ausgewählten Trägermaterial auch eine Funktionalisierung unterbleiben.

Eine erste Funktionalisierung, welche bereits geeignet ist, eine kovalente Bindung der Aminosäuresequenzen an die Oberfläche zu bewerkstelligen, kann in der Bereitstellung von Aminound/oder Carboxylgruppen als reaktive Gruppen erfolgen. Eine derartige Funktionalisierung wird, unabhängig von der chemischen Natur der aufgebrachten reaktiven Gruppen, hierin auch als erste Funktionalisierung bezeichnet. Die Erzeugung von Carboxylgruppen kann, beispielsweise, ausgehend von Polyolefinen, als das die Oberfläche bereitstellendes Material, durch Oxidation mit Chromsäure erfolgen. Alternativ kann dies beispielsweise durch Hochdruckreaktion mit Oxalylchlorid sowie Plasmaoxidation, radikalische oder Licht-induzierte Addition von Acrylsäure und dergleichen bewerkstelligt werden. Halogenierte Materialien wie halogenierte Polyolefine können durch Basen-katalysierte Eliminationsprozesse, die zu Doppelbindungen an der Oberfläche führen, sowohl zur Erzeugung von Amino- als auch Carboxy-reaktiven Gruppen führen, wobei anschließend die reaktiven Doppelbindungen Carboxy- oder Amino-funktionalisiert werden.

Keramik, Gläser Siliziumoxyd und Titanoxid können einfach mit den in einer Vielzahl kommerziell erhältlicher substituierter Silane, wie z. B. Aminopropyltriethoxysilan, funktionalisiert werden. Trägerplatten mit Hydroxylgruppen auf der Oberfläche sind durch eine Vielzahl von Reaktionen zu modifizieren. Besonders vorteilhaft sind Umsetzungen mit Biselektrophilen, wie zum Beispiel die direkte Carboxymethylierung mit Bromessigsäure; Acylierung mit einem entsprechenden Aminosäureabkömmling, wie z. B. die Dimethylaminopyridin-katalysierte Carbodiimid-Kupplung mit Fluorenylmethoxycarbonyl-3-aminopropansäure oder die Generation von Iso(thio-)cyanaten durch Mono-Umsetzung mit entsprechenden Bis-Iso(thio)cyanaten. Eine besonders vorteilhafte Methode ist die Reaktion mit Carbonyldiimidazol oder Phosgen oder Triphosgen oder p-Nitrophenyl-Chloroformiat bzw. Thiocarbonyldiimidazol, gefolgt durch die Reaktion mit Diamin oder einfach geschützten Diaminen, um Aminofunktionen über eine stabile Urethanbindung auf der Oberfläche zu den Trägermaterialien anzubringen.

Gemäß der vorliegenden Erfindung kann vorgesehen sein, dass die auf der Oberfläche immobilisierten Aminosäuresequenzen einen Abstandhalter aufweisen. Derartige Abstandhalter sind besonders dann bevorzugt, wenn die Aminosäuresequenzen das Substrat für enzymatische Aktivitäten sind, die eine bestimmte räumliche Struktur einnehmen sollen, um damit für die enzymatische Aktivität zugänglich zu sein. Infolge der Verwendung derartiger Abstandhalter, hierin auch als "spacer" bezeichnet, gewinnen die Aminosäuresequenzen, die die eigentlichen Substrate für die besagte enzymatische Aktivität oder Aktivitäten darstellen sollen, an zusätzlichen Freiheitsgraden und es werden Oberflächenphänomene wie Adsorption, Änderung der thermodynamischen Freiheitsgrade etc. auftreten. Ein Abstandhalter kann im wesentlichen jedes Molekül sein, insbesondere ein jedes biokompatible Molekül, das mindestens zwei funktionelle bzw. funktionalisierbare Gruppen enthält. Der Abstandhalter ist im verwendeten Zustand als Element zwischen der Oberfläche und der Aminosäuresequenz eingebaut.

Als Abstandhalter eignen sich die folgenden Verbindungsklassen:
- Alkane, verzweigt oder unverzweigt, insbesondere solche mit einer Kettenlänge von C2 bis C30, insbesondere C4 bis C8;
- Polyether, d. h. Polymere des Polyethylenoxids oder des Polypropylenoxids, wobei die Polyether bevorzugt aus 1 bis 5 Polyethylenoxideinheiten bzw. Polypropylenoxideinheiten bestehen.
- Polyalkohole, verzweigt oder unverzweigt, wie Polyglycol und Derivate davon, wie beispielsweise O,O'-Bis(2-Aminopropyl)-polyethylenglycol 500 und 2,2'-(Ethylendioxid)-Diethylamin
- Polyurethane, Polyhydroxysäuren, Polycarbonate, Polyimide, Polyamide, Polyester, Polysulfone, insbesondere solche bestehen aus 1-100 Monomereinheiten, ganz besonders bevorzugt bestehend aus 1-10 Monomereinheiten.
- Kombinationen der vorstehend genannten Alkane mit den vorstehend genannten Polyethern; Polyurethanen, Polyhydroxysäure, Polycarbonaten, Polyimiden, Polyamiden, Polyaminosäuren, Polyestern und Polysulfonen
- Diaminoalkane, verzweigt oder unverzweigt, bevorzugterweise solche mit einer Kettenlänge von C2 bis C30, ganz besonders bevorzugt solche mit einer Kettenlänge von C2 bis C8; beispielhaft seien hier genannt 1,3-Diaminopropan, 1,6-Diaminohexan und 1,8-Diaminooctan, sowie deren Kombinationen mit Polyethern, bevorzugterweise mit den vorstehend genannten Polyethem; wie zum Beispiel 1,4-bis-(3-Aminopropoxy)butan.
- Dicarbonsäuren und deren Derivate, wie zum Beispiel Hydroxy-, Mercapto- und Aminodicarbonsäuren, gesättigt oder ungesättigt, verzweigt oder unverzweigt, insbesondere C2 bis C30 Dicarbonsäuren, bevorzugterweise solche mit einer Kettenlänge von C2 bis C10, ganz besonders bevorzugt solche mit einer Kettenlänge von C2 bis C6; wie beispielsweise Bernsteinsäure und Glutarsäure; und
- Aminosäuren und Peptide, bevorzugterweise mit einer Länge von 1-20 Aminosäureresten, ganz besonders bevorzugt mit einer Läne von 1-3 Aminosäureresten, beispielsweise Trimere von Lysin, Dimere von 3-Aminopropionsäure und monomere 6-Aminocapronsäure.

Grundsätzlich ist es, infolge der Tatsache, dass der Abstandhalter zwei funktionale Enden aufweist, möglich, diese Funktionalitäten so auszuwählen, dass die auf der Oberfläche zu immobilisierenden Aminosäuresequenzen entweder über ihren C-Terminus oder ihren N-Terminus oder über eine andere funktionelle Gruppierung innerhalb der zu immobilisierenden Aminosäuresequenz immobilisiert werden. Soll eine Immobilisierung über den C-Terminus erfolgen, wird die am C-Terminus angreifende funktionale Gruppe des Abstandhalters bevorzugterweise eine Aminogruppe sein. Sollen die Aminosäuresequenzen vermittels des N-Terminus an die Oberfläche immobilisiert werden, ist die entsprechende funktionelle Gruppe des Abstandhalters eine Carboxylgruppe.

Bei der erfindungsgemäßen Anordnung kann vorgesehen sein, das der Abstandhalter ein verzweigter Abstandshalter ist. Derartige verzweigte Abstandshalter werden auch als dendrimere Strukturen oder kurz Dendrimere bezeichnet, und sind dem Fachmann auf dem Gebiet bekannt. Dendrimere Strukturen zur Immobilisierung von Nukleinsäuren sind beispielsweise beschrieben in Beier, M. & Hoheisel, J.D., 1999, Versatile derivatisation of solid support media for covalent bonding on DNA-microchips, 9, 1970-1977. Die Funktion derartiger dendrimerer Strukturen besteht darin, die Anzahl der reaktiven Gruppen pro Flächeneinheit der Oberfläche und damit die Signalintensität zu erhöhen. Dendrimere Strukturen können mit fast allen funktionellen oder funktionalisierbaren Gruppen versehen werden, die sodann eine Immobilisierung der Aminosäuresequenzen erlauben. Infolge der Verwendung derartiger dendrimerer Strukturen kann die Anzahl der reaktiven Gruppen pro Flächeneinheit der planaren Oberfläche um den Faktor 2 bis 100, bevorzugterweise um den Faktor 2 bis 20 und bevorzugtererweise um den Faktor 2 bis 10 gesteigert werden.

Der Aufbau einer dendrimerem Struktur kann beispielsweise im Falle des Versehens der Oberfläche mit einer Aminofunktionalität durch eine Reaktionsfolge bewirkt werden, die aus einer Acylierung mit Acrylsäure bzw. Acrylsäureabkömmlingen wie beispielsweise Acrylsäurechlorid, oder alpha-Brom-Carbonsäuren bzw. alpha-Brom-Carbonsäureabkömmlingen wie beispielsweise Bromacetylbromid, Michael-Addition geeigneter Polyamine wie beispielsweise Tetraethylenpentamin, anschließende erneute Acylierung mit Acrylsäure bzw. Acrylsäureabkömmlingen wie beispielsweise Acrylsäurechlorid oder alpha-Brom-Carbonsäuren bzw. alpha-Brom-Carbonsäureabkömmlingen wie beispielsweise Bromacetylbromid und erneute Michael-Addition von Polyaminen hergestellt werden. Die Polyamine werden bevorzugterweise so ausgewählt, dass sie selbst hydrophil sind, um die Hydrophilie der Oberfläche zu erhöhen. Ein Beispiel für ein derartiges Polyamin ist 1,4-bis-(3-Aminopropoxy)butan.

Neben der ersten Funktionalisierung der Oberfläche kann eine zweite Funktionalisierung erfolgen, die auf der ersten Funktionalisierung aufbaut. Mit anderen Worten, die reaktive Gruppe der Oberfläche wird durch zusätzliche Maßnahmen weitergehend funktionalisiert. Diese zweite Funktionalisierung kann dabei direkt an der funktionalisierten Oberfläche, an der mit einem Abstandshalter versehenen Oberfläche oder an einer dendrimeren Struktur erfolgen.

Ein Grund für die zweite Funktionalisierung kann darin gesehen werden, dass infolge der auch in den Aminosäuresequenzen vorhandenen Amino- und Carboxylgruppen, Thiolfunktionen, Imidazolfunktionen und Guanidofunktionen keine einheitliche Immobiliserung bezogen auf die Orientierung der Aminosäuresequenz auf der Oberfläche zu erzielen ist. Eine zweite Funktionalisierung bietet den Zugang zu weiteren chemoselektiven Reaktionen, um eine gerichtete Immobilisierung zu erreichen.

Für diese zweite Funktionalisierung eignen sich all jene Verbindungen, die sich durch ein Vorhandensein von nicht-proteinogenen funktionellen Gruppen auszeichnen. Beispielhaft seien die folgenden Verbindungen genannt: Maleinimido-Verbindungen wie Maleinimido-amine oder Maleinimido-carbonsäuren; alpha-Halo-Ketone wie Brombrenztraubensäure oder 4-Carboxyalpha-Brom-Acetophenon, alpha-Isothiocyanato-Ketone wie 4-Carboxy-alpha-Isothiocyanato-Acetophenon, Aldehyde wie Carboxybenzaldehyd, Ketone wie Lävulinsäure, Thiosemicarbazide, Thioamide wie Bemsteinsäuremonothioamid, alpha-Brom-Carbonsäuren wie Bromessigsäure, Hydrazine wie 4-Hydrazinobenzoesäure, O-Alkylhydroxylamine wie Amino-oxy-essigsäure, und Hydrazide wie Glutarsäuremonohydrazid.

Als weitere Maßnahme kann bei der Ausgestaltung der erfindungsgemäßen Anordnung vorgesehen sein, dass diejenigen Stellen oder Bereiche der Oberfläche, die nicht mit einer Aminosäuresequenz versehen sind, blockiert sind. Durch die Blockierung wird gewährleistet, dass während oder nach der chemoselektiven Reaktion der Aminosäuresequenzen mit den ggf. funktionalisierten Oberflächen noch nicht umgesetzte, aber noch reaktive Gruppierungen oder Gruppen auf der Oberfläche inaktiviert werden. Diese Blockierungsreaktion ist notwendig, da sonst die zugesetzte enzymatische Aktivität oder andere Bestandteile der eingesetzten biologischen Probe unspezifisch mit diesen, noch nicht blockierten, reaktiven Gruppen auf der Oberfläche reagieren und damit eventuell für ein großes Hintergrundsignal sorgen können. Solche unspezifischen Reaktionen mit Oberflächen sind eine häufige Ursache für ungünstige Signal-Rausch-Verhältnisse bei biochemischen Analysen. Für dieses Blockieren eignen sich solche Verbindungen, die sterisch nicht anspruchsvoll sind, sehr gut mit den zu blockierenden Gruppen reagieren und möglichst günstige Oberflächeneigenschaften generieren. Die Auswahl dieser Verbindungen wird in Abhängigkeit von der Art der Probe bzw. des Wechselwirkungspartners, der mit einer der Aminosäuresequenz in Wechselwirkung tritt, abhängen. Die Verbindung wird hydrophil ausgestaltet werden, wenn bekannt ist, daß die enzymatische Aktivität bevorzugt an hydrophoben Oberflächen unspezifisch bindet und hydrophob, wenn bekannt ist, daß die enzymatische Aktivität bevorzugt an hydrophoben Oberflächen unspezifisch bindet. So ist es dem Fachmann bekannt, dass ein Biomolelül, wie zum Beispiel ein Protein, eine dreidimensionale, genau definierte Struktur für die korrekte biologische Funktion benötigt. Diese Tertiärstruktur ist deutlich von der Umgebung abhängig. So besitz ein Protein in Wasser, als hydrophilem Lösungsmittel, die Tendenz, alle oder besser gesagt möglichst viele hydrophobe Gruppierungen im Inneren zu verbergen. Gelangt ein solches Protein in eine mehr hydrophobe Umgebung (hydrophobe Oberfläche) , kann es deshalb zum Um- bzw. Auffalten des Proteins und damit zur Inaktivierung kommen. Auf der anderen Seite sind Proteine bekannt, die in ihrer natürlichen Vorkommensweise innerhalb von (hydrophoben) Biomembranen vorliegen. Solche Proteine würden sich beim Inkontaktkommen mit einer hydrophilen Oberfläche umfalten und dabei denaturieren bzw. inaktiviert werden. In einem solchen Fall ist eine hydrophobe Oberfläche wünschenswert.

Die Bestandteile der Aminosäuersequenzen der erfindungsgemäßen Anordnung sind Aminosäuren, die bevorzugterweise ausgewählt sind aus der Gruppe, die L- und D- Aminosäuren umfasst. Weiterhin können die Aminosäuren ausgewählt sein aus der Gruppe die natürliche und nichtnatürliche Aminosäuren umfasst. Eine bevorzugte Gruppe innerhalb einer jeden der vorstehenden Gruppen von Aminosäuren sind die entsprechenden alpha-Aminosäuren. Die Aminosäuresequenzen können aus einer Abfolge von Aminosäuren aus einer jeden beliebigen der vorstehenden Gruppe bestehen. So ist beispielsweise eine Kombination aus D- und L-Aminosäuren im Umfang der Erfindung ebenso wie Aminosäuresequenzen, die entweder ausschließlich aus D-oder aus L- Aminosäuren bestehen. Die Bestandteile der Aminosäuresequenzen können darüberhinaus auch andere Moleküle umfassen als Aminosäuren Beispiele hierfür sind Thioxo-Aminosäuren, Hydroxy-Säuren, Mercapto-Säuren, Dicarbonsäuren, Diamine, Dithioxocarbonsäuren, Säuren und Amine. Eine weitere Form von derivatisierten Aminosäuresequenzen sind die sogenannten PNAs (engl. peptide nucleic acids)

Die Dichte der Aminosäuresequenzen beträgt 1/cm² bis 2000/cm², wobei die Dichte bevorzugt 5/cm² bis 1000/cm² und ganz besonders bevorzugt 10/cm² bis 400/cm² beträgt. Derartige Dichten von distinkten Orten auf einer Oberfläche, die jeweils eine Aminosäurespecies enthalten können unter Verwendung verschiedener Techniken erreicht werden, so beispielsweise mit piezoelektrisch betriebenen Pipettier-Robotem, mit feinen Nadeln aus verschiedenen Materialien wie Polypropylen, Edelstahl oder Wolfram bzw. entsprechender Legierungen, mit sogenannten "Pin-tools", die entweder geschlitzte Nadeln darstellen oder aufgebaut sind aus einem Ring, der die zu applizierende Substanzmischung enthält, und einer Nadel, die durch die in diesem Ring enthaltene Substanzmischung hindurch diese auf der entsprechenden Oberfläche absetzt. Aber auch mit einer motorgetriebenen Spritze verbundene Kapillaren sind dafür geeignet (Spotter). Eine weitere Möglichkeit besteht darin, die zu immobilisierenden Proben durch geeignete Stempelchen aufzubringen. Aber auch das Auftragen der zu immobilisierenden Aminosäuresequenzen mittels geeigneter Pipetten bzw. sogenannter Multipetten per Hand ist möglich. Weiterhin ist es möglich, die oben angegebenen Dichten von distinkten Orten durch die direkte *in situ* Synthese der Aminosäuresequenzen auf der Oberfläche zu erzeugen. (M. Stankova, S. Wade, K.S. Lam, M. Lebl; 1994, Synthesis of combinatorial libraries with only one representation of each structure, *Pept. Res.,* **7**, 292-298, F. Rasoul, F. Ercole, Y. Pham, C.T. Bui, Z.M. Wu, S.N. James, R.W. Trainor, G. Wickham, N.J. Maeji; 2000, Grafted supports in solid-phase synthesis, *Biopolymers,* **55**, 207-216, H. Wenschuh, R. Volkmer-Engert, M. Schmidt, M. Schulz, J. Schneider-Mergener, U. Reineke; 2000, Coherent membrane supports for parallel microsynthesis and screening of bioactive peptides, *Biopolymer.,* **55**, 188-206, R.Frank, 1992, Spot synthesis: an easy technique for the positionally addressable, parallel chemical synthesis on a membrane support, *Tetrahedron*, **48**, 9217-9232; A.Kramer und J. Schneider-Mergener, Methods in Molecular Biology, vol 87: Combinatorial Peptide Library Protocols, S. 25-39, edited by: S. Cabilly; Humana Press Inc., Totowa, NJ; Töpert, F., Oires, C., Landgraf, C., Oschkinat, H. and Schneider-Mergener, J., 2001, Synthesis of an array comprising 837 variants of the hYAP WW protein domain, *Angew. Chem. Int. Ed.,* **40**, 897-900, S.P.A. Fodor, J.L.Read, M.C.Pirrung, L.Stryer, A.T.Lu, D.Solas; 1991, Light-directed, spatially addressable parallel chemical synthesis, *Science,* **251**, J.P. Pellois, W. Wang, X.L. Gao; 2000, Peptide synthesis based on *t*-Boc chemistry and solution photogenerated acids, *J. Comb. Chem.,* **2**, 355-360).

In einer bevorzugten Ausführungsform der erfindungsgemäßen Anordnung und deren verschiedenen Verwendungen und Anwendungen ist vorgesehen, dass die verschiedenen Aminosäuresequenzen Substrate oder mögliche Substrate von enzymatischen Aktivitäten sind, die als Wechselwirkungspartner in Proben enthalten sind, denen gegenüber die erfindungsgemäße Anordnung exponiert wird. Unter enzymatischen Aktivitäten sind hierin allgemein solche enzymatischen Aktivitäten verstanden, die sich dadurch auszeichnen, dass sie eine Atomgruppe, ein Molekül oder eine Molekülgruppe auf ein Molekül übertragen. Dabei sind unter enzymatischen Aktivitäten hierin insbesondere Kinasen, Sulfotransferasen, Glycosyltransferasen, Acetyltransferasen, Farnesyltransferasen, Palmityltransferasen, Phosphatasen, Sulfatasen, Esterasen, Lipasen, Acetylasen und Proteasen zu verstehen. Die enzymatische Aktivität wird entsprechend gegebenenfalls eine oder mehrere der Aminosäuresequenzen der Anordnung, also eine oder mehrere der Aminosäuren auf dem Chip, hinsichtlich des Molekulargewichtes verändern. Eine derartige Veränderung des Molekulargewichts kann in einer Verringerung oder Erhöhung desselben bestehen und weitere Änderungen der physikochemischen Eigenschaften der Aminosäuresequenzen oder der distinkten Orte, an denen sich jeweils eine Species von Aminosäuresequenz befindet, nach sich ziehen.

Der Nachweis darüber, dass an einer oder mehreren der verschiedenen Aminosäuresequenzspecies ein Bindungsereignis und, sofern der Wechselwirkungspartner der Aminosäuresequenz eine enzymatische Aktivität ist oder diese trägt, eine enzymatische Umsetzung an der jeweiligen Aminosäuresequenz erfolgt ist, kann dabei unter Verwendung von verschiedenen Techniken erfolgen, die dem Fachmann bekannt sind. So ist es möglich, eine Spaltungsreaktion, z. B. vermittelt durch eine Protease, durch Änderung der Fluoreszenz eines geeigneten, die Oberfläche gebundenen Substratmoleküles zu verfolgen. Prinzipiell können alle Reaktionen, bei denen das an die Oberfläche gebundene Molekül durch Übertragung von weiteren Molekülen (Co-Substraten) im Molekolargewicht verändert wird, durch Einbringen einer radioaktiven Markierung in das Co-Substrat verfolgt werden. Dazu muß nach der erfolgten Reaktion die in das an die Oberfläche gebundene, modifizierte Molekül eingebaute Radioaktivität quantifiziert werden. Mit Hilfe solcher radioaktiven Markierungen können alle Transferasen, wie beispielsweise Kinasen, Acetyltransferasen, Famesyltransferasen und Glycosyltransferasen, bezüglich der enzymatischen Aktivität charakterisiert werden. Alternativ können aus der jeweiligen enzymatische Umsetzung an der jeweiligen Aminosäuresequenz hervorgegangene, vorher nicht vorhandene reaktive Gruppen mittels nachgeschalteter spezifischer Reaktionen nachgewiesen werden. Zum Beispiel läßt sich eine nach enzymatische Umsetzung erhaltende Mercaptofunktion mittels einer nachgeschaltete Reaktion mit Ellman-Reagenz nachweisen.

Es ist im Rahmen der vorliegenden Erfindung, dass die Anordnung eine gewisse Anzahl von verschiedenen Aminosäurespecies umfasst. Dabei kann vorgesehen sein, dass die gleiche Aminosäurespecies an mehreren distinkten Orten auf der Oberfläche bzw. dem Trägermaterial vorhanden ist. Damit kann zum einen ein interner Standard realisiert werden, zum anderen aber auch mögliche Randeffekte dargestellt und erfasst werden.

Eine weitere Ausgestaltung der erfindungsgemäßen Anordnung sieht vor, dass mindestens zwei oder mehrere der Anordnungen so aneinandergefügt werden, dass zwischen den beiden Anordnungen nur mehr ein sehr kleiner Spalt verbleibt, in den sich die Aminosäuresequenzen der beiden Anordnungen hinein erstrecken (vgl. Fig. 12B). Diese Ausgestaltung wird hierin auch als Trägeranordnung bezeichnet. Damit wird eine Möglichkeit eröffnet, mit einem sehr kleinen Probenvolumen eine Vielzahl von Tests durchzuführen. Die Breite des Spaltes beträgt dabei 2 mm, bevorzugterweise 0.5 mm und bevorzugtererweise weniger als 0.1 mm. Damit ergibt sich ein Flüssigkeitsvolumen weniger als 100 nL bezogen auf eine Grundfläche von 1 mm². Es ist im Rahmen der vorliegenden Erfindung, dass die Anordnungen, die die Trägeranordnung ausbilden verschieden sind hinsichtlich der Ausgestaltung. Diese Unterschiede können darin bestehen, dass die Aminosäuresequenzen insgesamt oder teilweise verschieden sind. Weiterhin ist es möglich, dass die Aminosäuresequenzen bei den verschiedenen Anordnungen vollständig oder teilweise an anderen distinkten Orten angeordnet sind.

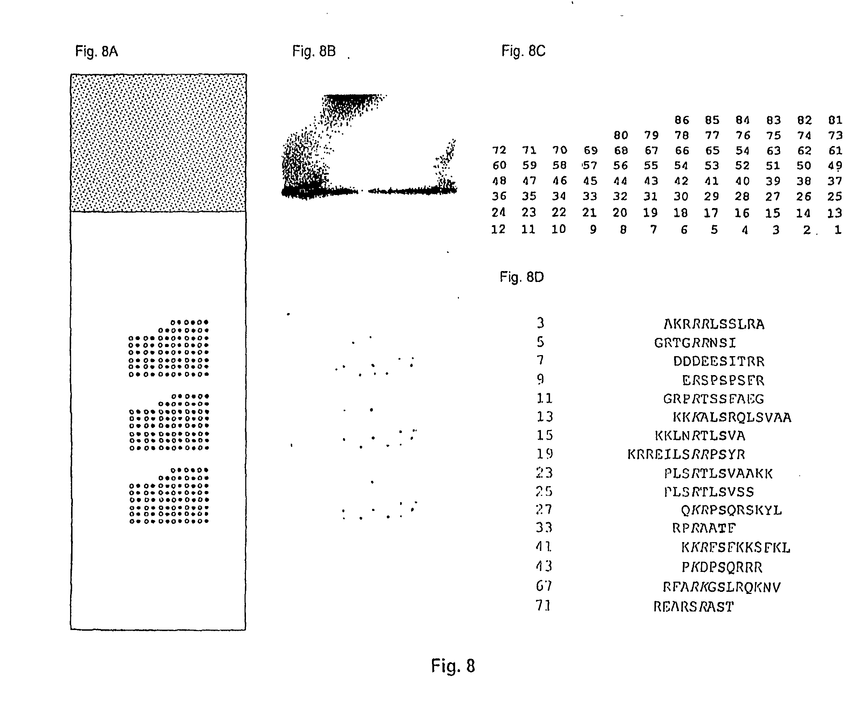

Die erfindungsgemäße Anordnung bietet eine Reihe von Anwendungsmöglichkeiten. Eine derartige Anwendung stellt die Bestimmung der Substratspezifität einer enzymatischen Aktivität (Fig. 12, Verbindung C) dar. Dabei wird so vorgegangen, dass in einem ersten Schritt eine erfindungsgemäße Anordnung oder Chip bereitgestellt wird und dieser mit einer Probe, die die jeweilige enzymatische Aktivität enthält, kontaktiert und gegebenenfalls inkubiert. Anschließend erfolgt der Nachweis der Reaktion zwischen einer oder mehreren auf der Anordnung vorhandenen Aminosäuresequenzen (Fig. 12A, Verbindungen B₁-B₃, oder Fig. 12 B, Verbindungen B₁-B₅) und der enzymatischen Aktivität (Fig. 12, Verbindung C), wobei auf die oben beschriebenen Nachweisverfahren zurückgegriffen werden kann. Infolge der Anordnung der verschiedenen Aminosäuresequenzspecies an distinkten Orten kann somit ein an einem bestimmten Ort erfolgendes Reaktionsgeschen einer bestimmten Aminosäuresequenz oder Aminosäuresequenzspecies (beide Begriffe werden hierin synonym gebraucht) eindeutig zugeordnet und daraus die Substratspezifität der enzymatischen Aktivität bestimmt werden (vgl. hierzu Fig. 8, 9 und 10).

Ausgehend von der Substratspezifität kann dann beispielsweise auch der Einfluß verschiedener Substanzen auf die jeweilige Reaktion untersucht werden. Beispielsweise kann in Abhängigkeit vom jeweiligen Substrat eine enzymatische Aktivität eine bestimmte Modifizierung durch einen dem die enzymatische Aktivität enthaltenden Reaktionsansatz zugesetzten Faktor, beispielsweise eine niedermolekulare Verbindung, erfahren.

Eine weitere Anwendung findet die erfindungsgemäße Anordnung bei der Darstellung der differenziellen Analyse der enzymatischen Aktivitäten einer Probe. Eine besonders bedeutsame Probe in diesem Sinne stellt das Proteom einer Zelle dar, anhand dessen dieser Aspekt der Erfindung im folgenden erläutert werden wird. Dabei wird im Unterschied zu der vorstehend beschriebenen Anwendung nicht auf die Spezifität einer einzelnen enzymatischen Aktivität hinsichtlich der auf der Oberfläche vorhandenen Aminosäurespecies abgestellt, sondern vielmehr gewissermaßen eine Momentaufnahme der enzymatischen Aktivitäten in einer Probe hinsichtlich der verschiedenen Aminosäuresequenzspecies der Anordnung. Diese Momentaufnahme erfolgte unter bestimmten Bedingungen, die zu dem Zeitpunkt herrschten, als die Probe genommen wurde. Im Falle des Proteoms kann dies beispielsweise der Zustand nach Exposition der Zelle, von der die Probe gewonnen wurde, gegenüber einer bestimmten Verbindung sein. Es werden dann eine oder mehrere weitere Proben genommen werden, wobei die Bedingungen, die zum Zeitpunkt der Probenahme herrschten, geändert werden, beispielsweise die Zelle gegenüber der besagten Verbindung nun nicht mehr exponiert war, und sodann eine Analyse durchgeführt werden. Je nach gewählten Nachweisverfahren wird man sodann das Ergebnis des Reaktionsgeschehens unter den verschiedenen Bedingungen vergleichen und daraus erkennen, ob und wenn ja in welchem Umfang sich das Muster aus dem jeweiligen Reaktionsgeschehen geändert hat. Andererseits kann eine solche Anordnung von Aminosäuresequenzen auch dazu genutz werden, biologische Proben wie zum Beispiel Zell-Lysate oder biologische Flüssigkeiten einer Species oder verschiedener Species über eine Mustererkennung miteinander zu vergleichen bzw. diese biologischen Proben mittels des erhaltenen Musters zu katalogisieren. Ein solches Muster dient dann im übertragenen Sinne als Fingerabdruck der untersuchten biologischen Probe. Somit kann das erfindungsgemäße Verfahren zur Identifizierung oder Individualisierung darstellen. Die Identifizierung kann dabei auf verschiedenen systematischen Ebenen erfolgen, d.h. die Zuordnung einer entsprechend untersuchten Probe zu einem Stamm, einer Klasse, einer Ordnung, einer Familie, einer Gattung, einer Art. Weiterhin kann die Identifizierung auch auf der Ebene der Art zwischen Individuen der gleichen Art oder Rasse erfolgen. Beispielhaft kann dieses Verfahren in der Forensik verwendet werden. Eine weitere Anwendung des Verfahrens kann darin gesehen werden, pathologische Zustände wie Krebs oder ein gegenüber der Norm geändertes Muster an enzymatischer Aktivität (sowohl quantitativ wie auch qualitativ) zu bestimmen, zu diagnostizieren oder zu prognostizieren.

Die vorliegende Erfindung wird nun anhand der folgenden Figuren und Beispiele weiter erläutert werden, wobei sich daraus weitere Merkmale, Ausführungsbeispiele und Vorteile ergeben können. Dabei zeigt:
- Fig. 1: das Resultat der Inkubation einer unterschiedlich modifizierten Glasoberfläche mit verschiedenen Kinasen;
- Fig. 2: das Resultat der Inkubation unterschiedlicher, modifizierter Oberflächen mit Proteinkinase A;
- Fig. 3: das Resultat der zeitabhängigen Inkubation unterschiedlicher modifizierter Oberflächen mit Proteinkinase A;
- Fig. 4: das Resultat der Inkubation einer unterschiedlich modifizierten Glasoberfläche mit verschiedenen Konzentrationen von Proteinkinase A
- Fig. 5: das Resultat der Inkubation einer modifizierten Glasoberfläche mit Proteinkinase A;
- Fig. 6: das Resultat der Inkubation einer modifizierten Glasoberfläche (11760 Spots) mit einer Kinase;
- Fig. 7: das Resultat der Inkubation einer modifizierten Glasoberfläche (960 Spots) mit einer Kinase;
- Fig. 8: das Resultat der Inkubation einer mit einem Satz potentieller Substratpeptide und entsprechenden Kontrollpeptiden modifizierten Glasoberfläche mit Proteinkinase C;
- Fig. 9: das Resultat der Inkubation einer mit einem Satz potentieller Substratpeptide und entsprechenden Kontrollpeptiden modifizierten Glasoberfläche mit Proteinkinase A;
- Fig. 10: das Resultat der Inkubation einer mit einem Satz potentieller Substratpeptide modifizierten Glasoberfläche mit Proteinkinase A;
- Fig. 11: eine Übersicht verschiedener chemoselektiver Reaktionen; und
- Fig. 12: den schematischen Aufbau verschiedener Ausführungsformen einer Anordnung von gerichtet auf einer Trägeroberfläche immobilisierten Verbin. dungen.

Es folgt eine genauere Beschreibung der Figuren.

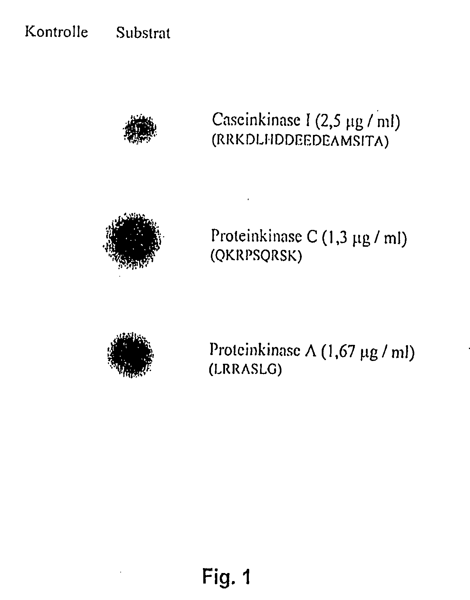

**Fig 1.** Die in den Klammern angegebenen Kinasesubstrate (am N-Terminus mit dem Dipeptid Cysteinyl-β-alanin modifiziert) wurden durch eine Michael-Addition an eine maleinimidofunktionalisierte Glasoberfläche (Beispiel 1) gekoppelt. Als Negativ-Kontrollen wurden Peptide, bei denen die zu phosphorylierende Aminosäure Serin durch die nicht phosphorylierbare Aminosäure Alanin ausgetauscht wurde, verwendet. Die Glasoberfläche wurde zunächst mit 10ml 100µM ATP-Lösung in 50 mM Natriumphosphatpuffer pH 7.5 für 10 Minuten vorinkubiert. Anschließend wurden die entsprechenden Kinasen zusammen mit ATP/γ³²P-ATP-Mischung aufgespottet (jeweils 1 µL, 5 U/mL) und 30 Minuten bei 25°C inkubiert (Beispiel 29). Die Phosphorylierung der entsprechenden Peptide wurde mittels eines PhosphorImagers von FUJIFILM detektiert.

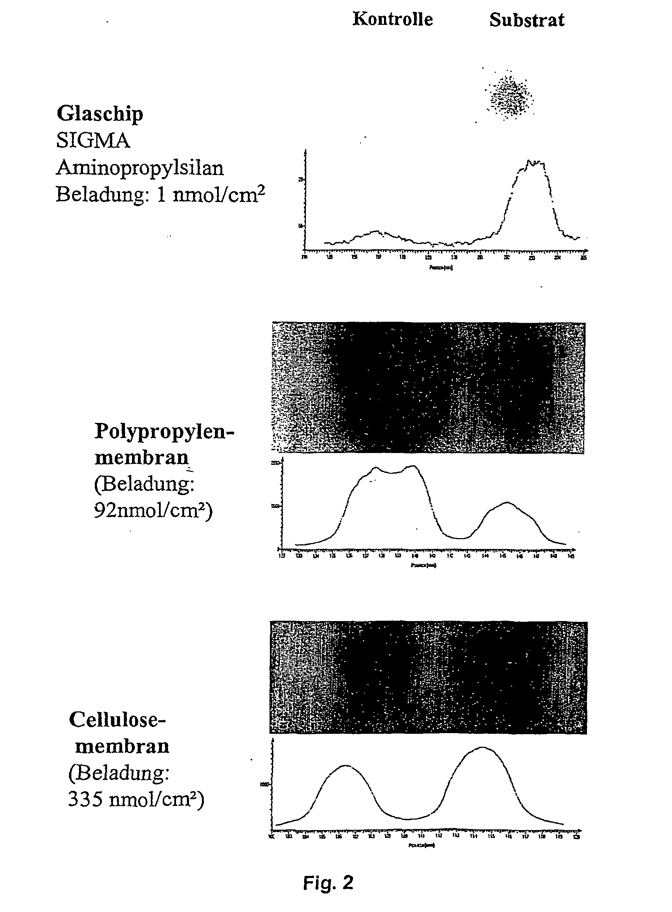

**Fig. 2.** Das Peptid Leu-Arg-Arg-Ala-Ser-Leu-Gly-NH₂ und das Kontrollpeptid Leu-Arg-Arg-Ala-Ser-Leu-Gly-NH₂, jeweils am N-Terminus mit dem Dipeptid Cysteinyl-β-alanin modifiziert, wurden durch eine Michael-Addition an eine maleinimidofunktionalisierte Oberfläche gekoppelt (Beispiel 1; maleinimidobutyryl-β-alaninin-funktionalisierte Cellulose sowie maleinimidobutyryl-β-alaninin-funktionalisierte, modifizierte Polypropylen-Membranen). Die so modifizierten Oberflächen wurden zunächst mit 10ml 100µM ATP-Lösung in 50 mM Natriumphosphatpuffer pH 7.5 für 10 Minuten vorinkubiert. Anschließend wurde Proteinkinase A zusammen mit ATP/γ³²P-ATP-Mischung (100 µM/mL; 100µCi/mL) aufgespottet (jeweils 1 µL, 2 U/mL) und 30 Minuten bei 25°C inkubiert (Beispiel 30). Die Phosphorylierung der entsprechenden Peptide wurde mittels eines PhosphorImagers von FUJIFILM detektiert. Unter den Abbildungen ist die Signalintensität der jeweiligen Spots angegeben. Es wird deutlich, daß unter den gewählten Versuchsbedingungen im Fall der modifizierten Cellulose bzw. Polypropylenoberfächen im Prinzip nur unspezifische Bindung von ATP bzw. Kinase an die Peptide gemessen wird. Im Fall der modifizierten Glasoberflächen dagegen ist das Signal für die Substrataminosäuresequenz um den Faktor 4-5 größer als das Signal für die entsprechende Kontrollaminosäuresequenz.

**Fig. 3.** Das Peptid Leu-Arg-Arg-Ala-Ser-Leu-Gly-NH₂, am N-Terminus mit dem Dipeptid Cysteinyl-β-alanin modifiziert, wurde durch eine Michael-Addition an maleinimidofunktionalisierte Oberflächen gekoppelt (maleinimido-funktionalisierte Glasoberfläche, Sigma, Silane-Prep™, S4651; sowie maleinimidobutyryl-β-alaninin-funktionalisierte, modifizierte Polypropylen-Membranen). Die modifizierten Glas-Oberflächen wurden zunächst mit 20ml 100µM ATP-Lösung in 50 mM Natriumphosphatpuffer pH 7.5 für 10 Minuten vorinkubiert. Anschließend wurde Proteinkinase A zusammen mit ATP/γ³²P-ATP-Mischung (100 µM/mL; 100µCi/mL) aufgespottet (jeweils 1 µL, 2 U/mL) und für die angegebene Zeit bei 25°C inkubiert (Beispiel 31). Die Phosphorylierung der entsprechenden Peptide wurde mittels eines PhosphorImagers von FUJIFILM detektiert. Unter den Abbildungen ist die Signalintensität der jeweiligen Spots angegeben. Es wird deutlich, daß unter den gewählten Versuchsbedingungen im Fall der modifizierten Polypropylenoberfächen im Prinzip nur unspezifische Bindung von ATP bzw. Kinase an das Peptid gemessen wird. Im Fall der modifizierten Glasoberflächen dagegen ist eine deutliche Zeitabhängigkeit für den Kinase-vermittelten Einbau von Radioaktivität in die Substrataminosäuresequenz zu erkennen.

**Fig. 4.** Das Kontrollpeptid Leu-Arg-Arg-Ala-Ala-Leu-Gly-NH₂, das Peptid Leu-Arg-Arg-Ala-Ala-Leu-Gly-NH₂ nach HPLC-Reinigung und das Synthese-Rohprodukt Leu-Arg-Arg-Ala-Ala-Leu-Gly-NH₂, jeweils am N-Terminus mit dem Dipeptid Cysteinyl-β-alanin modifiziert, wurden durch eine Michael-Addition an eine maleinimidofunktionalisierte Glas-Oberfläche (Beispiel 1) gekoppelt. Die modifizierten Glasoberfläche wurde zunächst mit 10ml 100µM ATP-Lösung in 50 mM Natriumphosphatpuffer pH 7.5 für 10 Minuten vorinkubiert. Anschließend wurde die modifizierte Glasoberfläche mit einem Deckgläschen abgedeckt und in den entstandenen Zwischenraum Proteinkinase A (1 U/mL bzw. 10U/mL) zusammen mit ATP/γ³²P-ATP-Mischung (100 µM/mL; 100µCi/mL) mittels Kapillarkraft eingebracht (Beispiel 25). Nach 30 minütiger Inkubation bei 25°C wurde die Phosphorylierung der entsprechenden Peptide mittels eines PhosphorImagers von FUJIFILM detektiert. Unter den Abbildungen ist die Signalintensität der jeweiligen Spots angegeben. Es wird deutlich, daß unter den gewählen Versuchsbedingungen die Signalintensität für die gereinigte Aminosäuresequenz um 500% höher ist als für das Syntheserohprodukt. Die Signalintensität für die gereinigte Substrat-Aminosäuresequenz ist ca. 300 mal höher als die für die entsprechenden Kontrollaminosäuresequenz. Zusammen mit der ca. 10fach geringeren Aktivitätsmenge, die für ein vergleichbares Signal (im Vergleich zu Celluloseoberflächen) benötigt wird ergibt sich somit eine Signalverbesserung um den Faktor 3000.

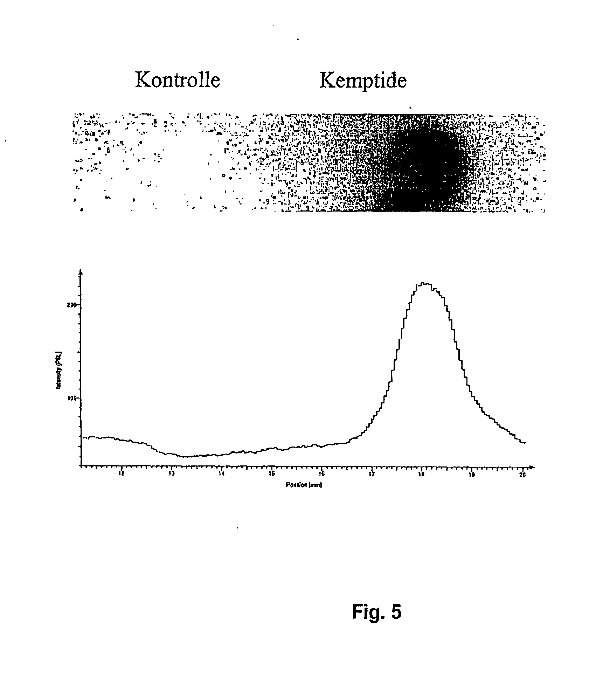

**Fig. 5.** Das Kontrollpeptid Leu-Arg-Arg-Ala-Ala-Leu-Gly-NH₂ und das Peptid Leu-Arg-Arg-Ala-Ala-Leu-Gly-NH₂, jeweils am N-Terminus mit dem Dipeptid Cysteinyl-β-alanin modifiziert, wurden durch eine Michael-Addition an eine maleinimidofunktionalisierte Glas-Oberfläche (Beispiel 1) gekoppelt. Die modifizierte Glas-Oberfläche wurden zunächst mit 10ml 100µM ATP-Lösung in 50 mM Natriumphosphatpuffer pH 7.5 für 10 Minuten vorinkubiert. Anschließend wurde die modifizierte Glas-Oberfläche mit einem Deckgläschen abgedeckt und in den entstandenen Zwischenraum Proteinkinase A (10U/mL) zusammen mit ATP/γ³²P-ATP-Mischung (100 µM/mL; 100µCi/mL) mittels Kapillarkraft eingebracht. Nach 30 minütiger Inkubation bei 25°C wurde die Phosphorylierung der entsprechenden Peptide mittels eines PhosphorImagers von FUJIFILM detektiert. Unter den Abbildungen ist die Signalintensität der jeweiligen Spots angegeben. Es wird deutlich, daß unter den gewählen Versuchsbedingungen die Signalintensität für die Substrat-Aminosäuresequenz um 800% höher ist als für das entsprechende Kontrollpeptid.

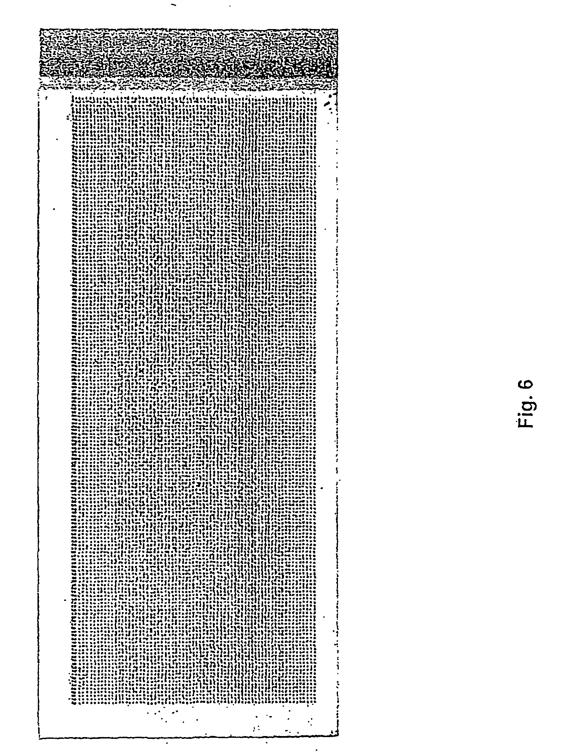

**Fig. 6.** Eine mit dem Peptid Leu-Arg-Arg-Ala-Ser-Leu-Gly-thioamid modifizierte Glasoberfläche (vgl. Beispiel 24) (Aminosäuresequenz wurde in 200 mM Natrium-Phosphatpuffer pH 5.5 gelöst und bei RT wurde jeweils 1 nL dieser Lösung in einer Anordnung von 70 Reihen und 168 Spalten (gesamt 11760 Spots) auf die bromketon-funktionalisierten Glasoberflächen (Beispiel 10) mittels eines NanoPlotters der Firma Gesim aufgebracht. Dabei betrug der Spot-zu-Spot-Abstand 0.3 mm. Anschließend wurden die so behandelten Glasoberflächen 2 min mit einer Mikrowelle behandelt und dann 3 Stunden bei RT inkubiert. Anschließend wurde zunächst mit 10ml 100µM ATP-Lösung in 50 mM Natriumphosphatpuffer pH 7.5 für 10 Minuten vorinkubiert. Anschließend wurde die modifizierte Glas-Oberfläche mit einer zweiten Glasoberfläche abgedeckt und in den entstandenen Zwischenraum Proteinkinase A (10U/mL) zusammen mit ATP/γ³²P-ATP-Mischung (100 µM/mL; 100µCi/mL) mittels Kapillarkraft eingebracht (vgl. Beispiel 31). Nach 30 minütiger Inkubation bei 25°C wurde die Phosphorylierung der entsprechenden Peptide mittels eines PhosphorImagers von FUJIFILM detektiert. Es wird deutlich, das einerseits die Verknüpfung des immobilisierten Kinasesubstrates von der Proteinkinase A toleriert wird und das andererseits die Modifikation der Glasoberflächen gleichmäßig und ohne größere Schwankungen in der Immobilisierungsdichte verläuft. Weiterhin ist klar, das die Auflösung des hier verwendeten PhosphorImagers ausreichend ist, um selbst mehr als 11000 Meßpunkte pro Biochip zu analysieren.

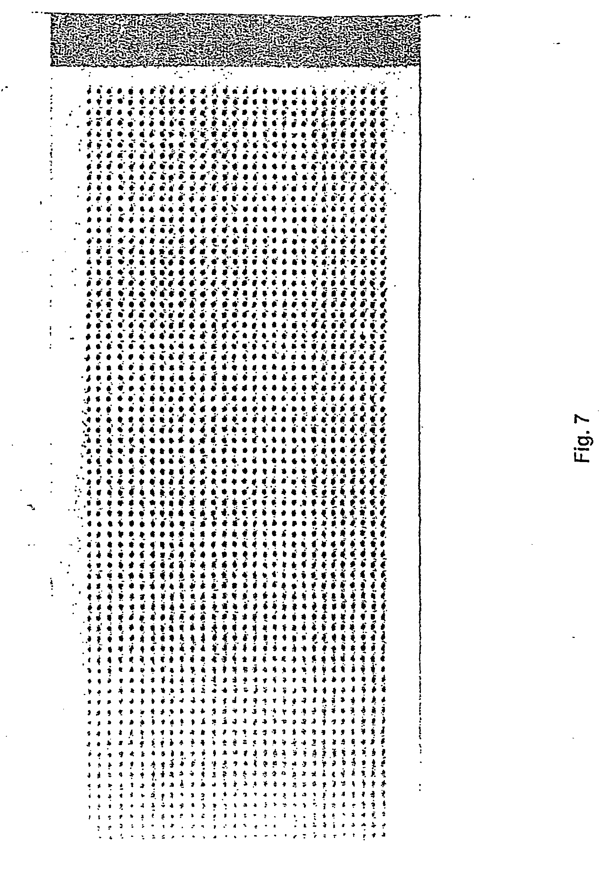

**Fig. 7.** Eine mit dem Peptid Dpr(Aoa)-Leu-Arg-Arg-Ala-Ser-Leu-Gly-NH₂ modifizierte Glasoberfläche (vgl. Beispiel 22) wurde zunächst mit 10ml 100µM ATP-Lösung in 50 mM Natriumphosphatpuffer pH 7.5 für 10 Minuten vorinkubiert. Anschließend wurde die modifizierte Glas-Oberfläche mit einer zweiten Glasoberfläche abgedeckt und in den entstandenen Zwischenraum Proteinkinase A (10U/mL) zusammen mit ATP/γ³²P-ATP-Mischung (100 µM/mL; 100µCi/mL) mittels Kapillarkraft eingebracht. Nach 30 minütiger Inkubation bei 25°C wurde die Phosphorylierung der entsprechenden Peptide mittels eines PhosphorImagers von FUJIFILM detektiert (vgl. Beispiel 14). Es wird deutlich, das einerseits die Verknüpfung des immobilisierten Kinasesubstrates von der Proteinkinase A toleriert wird und das andererseits die Modifikation der Glasoberflächen gleichmäßig und ohne größere Schwankungen in der Immobilisierungsdichte verläuft. Weiterhin ist klar, das die Auflösung des hier verwendeten PhosphorImagers ausreichend st, um mehr als 950 Meßpunkte pro Biochip zu analysieren.

**Fig. 8.** Genau 43 serin- und/oder threoninhaltige Peptide (potentielle Substratpeptide für Kinasen) und die entsprechenden Kontrollpeptide, jeweils am N-Terminus mit dem Dipeptid Cysteinyl-β-alanin modifiziert, wurden durch eine Michael-Addition an eine maleinimidofunktionalisierte Glas-Oberfläche gekoppelt (Beispiel 18). In den Kontrollpeptiden wurden die Serinund/oder Threoninreste bei ansonsten gleichbleibender Sequenz durch Alanin ersetzt. Die Aufbringung erfolgte mittels eines NanoPlotters der Firma Gesim. Dabei betrug der Spot-zu-Spot-Abstand 1 mm, pro Spot wurden 0.8 nL einer Peptidlösung in 100 mM PBS Puffer pH 7.8, die 20 % Glycerin enthielt, aufgebracht. Die Anordnung der Peptide ist in Fig. 8A dargestellt. Dabei stellt ein gefüllter Kreis ein serin und/oder threoninhaltiges potentielles Subtratpeptid dar, ein offener Kreis steht für ein Kontrollpeptid. Es wurden drei identische Subarrays auf die Glasoberfläche aufgebracht. Die Nummerierung der Spots ist in Fig. 8C ersichtlich, die Sequenzen der genutzten Peptide gehen aus Beispiel 18 hervor. Die modifizierte Glas-Oberfläche wurde nach dem Aufbringen der Peptide zunächst mit 10mL 100µM ATP-Lösung in 50 mM Natriumphosphatpuffer pH 7.5 für 10 Minuten vorinkubiert. Anschließend wurde die modifizierte Glas-Oberfläche mit einem Deckgläschen abgedeckt und in den entstandenen Zwischenraum Proteinkinase C (10U/mL) zusammen mit ATP/γ³²P-ATP-Mischung (100 µM/mL; 100µCi/mL) mittels Kapillarkraft eingebracht. Nach 30 minütiger Inkubation bei 25°C wurde die Phosphorylierung der entsprechenden Peptide mittels eines PhosphorImagers von FUJIFILM detektiert (Beispiel 38). Das resultierende Bild ist in Fig. 8B dargestellt. Die Spots mit hoher Signalintensität in allen drei Subarrays wurden den entsprechenden von der Proteinkinase C phosphorylierten Peptiden zugeordnet. Deren Primärstrukturen sind in Fig. 8D gezeigt. Es wird deutlich, dass als Proteinkinase C Substrate bekannte (Substratpeptid Nr. 3, 23, 27, 41, 43) und weitere, nicht als Substrate für Proteinkinase C beschriebene Peptide von dieser Kinase auf der modifizierten Glasoberfläche erkannt und phosphoryliert werden. Damit wird deutlich, dass eine solche Anordnung geeignet ist, die Substratspezifität einer Kinase, wie zum Beispiel Proteinkinase C, zu charakterisieren.

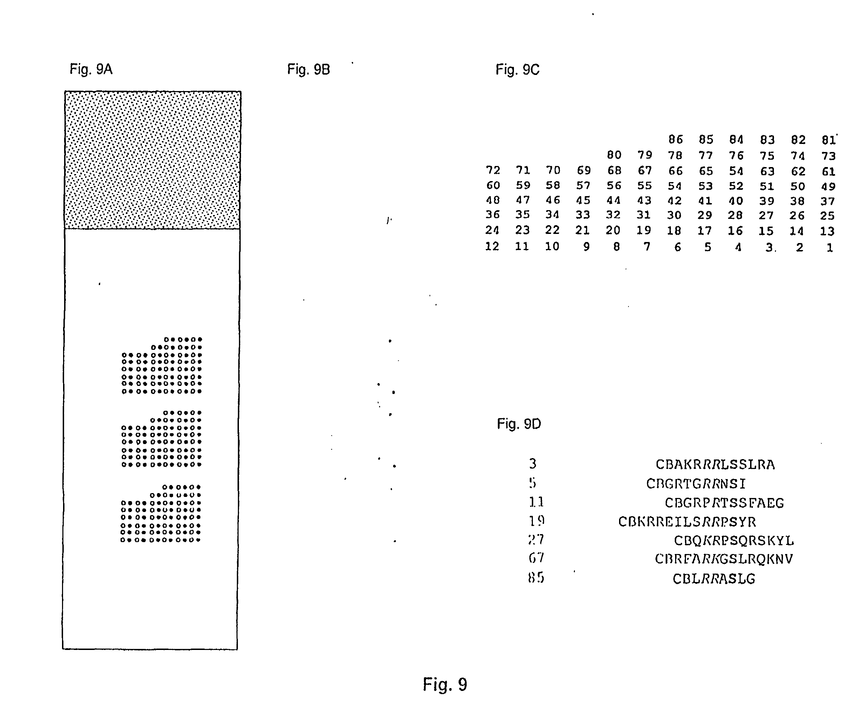

**Fig. 9.** Genau 43 serin- und/oder threoninhaltige Peptide (potentielle Substratpeptide für Kinasen) und die entsprechenden Kontrollpeptide, jeweils am N-Terminus mit dem Dipeptid Cysteinyl-β-alanin modifiziert, wurden durch eine Michael-Addition an eine maleinimidofunktionalisierte Glas-Oberfläche gekoppelt (Beispiel 18). In den Kontrollpeptiden wurden die Serinund/oder Threoninreste bei ansonsten gleichbleibender Sequenz durch Alanin ersetzt. Die Aufbringung erfolgte mittels eines NanoPlotters der Firma Gesim. Dabei betrug der Spot-zu-Spot-Abstand 1 mm, pro Spot wurden 0.8 nL einer Peptidlösung in 100 mM PBS Puffer pH 7.8, die 20 % Glycerin enthielt, aufgebracht. Die Anordnung der Peptide ist in Fig. 9A dargestellt. Dabei stellt ein gefüllter Kreis ein serin und/oder threoninhaltiges potentielles Subtratpeptid dar, ein offener Kreis steht für ein Kontrollpeptid. Es wurden drei identische Subarrays auf die Glasoberfläche aufgebracht. Die Nummerierung der Spots ist in Fig. 9C ersichtlich, die Sequenzen der genutzten Peptide gehen aus Beispiel 18 hervor. Die modifizierte Glas-Oberfläche wurde nach dem Aufbringen der Peptide zunächst mit 10mL 100µM ATP-Lösung in 50 mM Natriumphosphatpuffer pH 7.5 für 10 Minuten vorinkubiert. Anschließend wurde die modifizierte Glas-Oberfläche mit einem Deckgläschen abgedeckt und in den entstandenen Zwischenraum Proteinkinase A (10U/mL) zusammen mit ATP/γ³²P-ATP-Mischung (100 µM/mL; 100µCi/mL) mittels Kapillarkraft eingebracht. Nach 30 minütiger Inkubation bei 25°C wurde die Phosphorylierung der entsprechenden Peptide mittels eines PhosphorImagers von FUJIFILM detektiert (Beispiel 39). Das resultierende Bild ist in Fig. 9B dargestellt. Die Spots mit hoher Signalintensität in allen drei Subarrays wurden den entsprechenden von der Proteinkinase A phosphorylierten Peptiden zugeordnet. Deren Primärstrukturen sind in Fig. 9D gezeigt. Es wird deutlich, daß mit einer Ausnahme alle Peptide auf der modifizierten Glasoberfläche von Proteinkinase A erkannt und phosphoryliert werden, die in Position -2 und -3 (N-terminal) zum Serin zwei Argininreste tragen. Dieses Sequenzmotiv RRxS ist als bevorzugtes Substratmotiv für Proteinkinase A beschrieben (A. Kreegipuu, N. Blom, S. Brunak, J. Jarv, 1998, Statistical analysis of protein kinase specificity determinants, *FEBS Lett.,* **430**, 45-50). Das Peptid 83 wird wahrscheinlich wegen der zu sehr N-terminalen Lokalisation des Substratmotives nicht phosphoryliert. Somit wird deutlich, dass eine solche Anordnung geeignet ist, die Substratspezifität einer Kinase, wie zum Beispiel Proteinkinase A, zu charakterisieren.

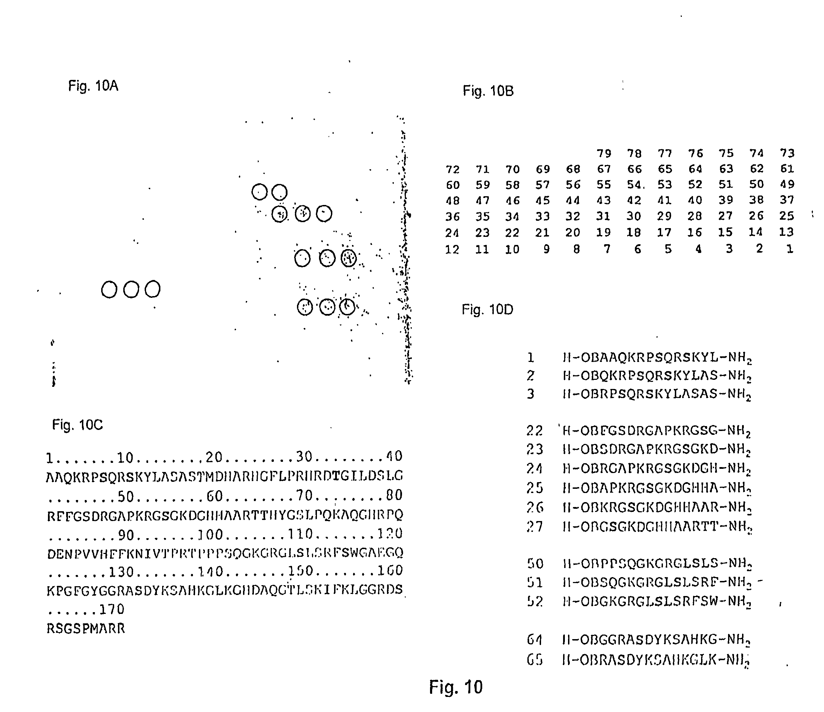

**Fig. 10.** Genau 79 Peptide, jeweils am N-Terminus mit dem Dipeptid Amino-oxyessigsäure-β-alanin modifiziert, wurden an eine aldehydfunktionalisierte Glas-Oberfläche gekoppelt (Beispiel 20). Die Aufbringung erfolgte mittels eines NanoPlotters der Firma Gesim. Dabei betrug der Spot-zu-Spot-Abstand 1.5 mm, pro Spot wurden 1 nL einer Peptidlösung in DMSO aufgebracht. Die 13meren Peptide überlappen mit jeweils 11 Aminosäureresten und decken zusammen vollständig die Primarstruktur von MBP ab, dass heißt, sie stellen zusammen einen Scan durch das Myelin basic Protein (MBP) aus *bos taurus* (SWISSPROT Zugangsnummer P02687) dar. Die Primässtruktur des MBP ist in Fig. 10C dargestellt. Für die durch Fettdruck hervorgehobenen Reste wurde eine Phosphorylierung durch Proteinkinase A im Stand der Technik beschrieben (A. Kishimoto, K. Nishiyama, H. Nakanishi, Y. Uratsuji, H. Nomura, Y. Takeyama, Y. Nishizuka, 1985, Studies on the phosphorylation of myelin basic protein by protein kinase C and adenosine 3':5'-monophosphate-dependent protein kinase, *J*. *Biol*. *Chem*., **260**, 12492-12499). Die 13 meren Peptide in dem Scan weisen eine Sequenzverschiebung von 2 Aminosäuren auf. Die Anordnung der Peptide ist in Fig. 10B dargestellt. Somit repräsentiert das Peptid Nr. 1 die Aminosäuresequenz 1-13 der Primärstruktur von MBP, das Peptid Nr. 2 die Aminosäuresequenz 3-15 der Primärstruktur von MBP usw. Es wurden drei identische Subarrays auf die Glasoberfläche aufgebracht. In Fig. 10A ist eines dieser Subarrays dargestellt. Die modifizierte Glas-Oberfläche wurde nach dem Aufbringen der Peptide zunächst mit 10mL 100µM ATP-Lösung in 50 mM Natriumphosphatpuffer pH 7.5 für 10 Minuten vorinkubiert. Anschließend wurde die modifizierte Glas-Oberfläche mit einem Deckgläschen abgedeckt und in den entstandenen Zwischenraum Proteinkinase A (10U/mL) zusammen mit ATP/γ³²P-ATP-Mischung (100 µM/mL; 100µCi/mL) mittels Kapillarkraft eingebracht (vgl. Beispiel 40). Nach 30 minütiger Inkubation bei 25°C wurde die Phosphorylierung der entsprechenden Peptide mittels eines PhosphorImagers von FUJIFILM detektiert. Das resultierende Bild ist in Fig. 10A dargestellt. Die Spots mit hoher Signalintensität in allen drei Subarrays wurden den entsprechenden von der Proteinkinase A phosphorylierten Peptiden zugeordnet. Deren Primärstrukturen sind in Fig. 10D gezeigt. Es wird deutlich, daß die meisten Peptide auf der modifizierten Glasoberfläche von Proteinkinase A erkannt und phosphoryliert werden, die auch in dem in Lösung durchgeführten Experiment gefunden werden konnten (A. Kishimoto, K. Nishiyama, H. Nakanishi, Y. Uratsuji, H. Nomura, Y. Takeyama, Y. Nishizuka, 1985, Studies on the phosphorylation of myelin basic protein by protein kinase C and adenosine 3':5'-monophosphate-dependent protein kinase, *J*. *Biol*. *Chem*., **260**, 12492-12499).

**Fig. 11.** Gezeigt ist eine Übersicht verschiedener chemoselektiver Reaktionen nach dem Stand der Technik: A) Aldehyde (R⁴=H) oder Ketone (R⁴ nicht H) und Amino-oxy-Verbindungen reagieren zu Oximen, B) Aldehyde (R⁴=H) oder Ketone (R⁴ nicht H) und Thiosemicarbazide reagieren zu Thiosemicarbazonen, C) Aldehyde (R⁴=H) oder Ketone (R⁴ nicht H) und Hydrazide reagieren zu Hydrazonen, D) Aldehyde (R⁴=H) oder Ketone (R⁴ nicht H) und 1,2-Aminothiole reagieren zu Thiazolinen (X=S) bzw. 1,2-Aminoalkohole zu Oxazolinen (X=O) bzw. 1,2-Diamine reagieren zu Imidazolinen (X=NH), E) Thiocarboxylate und α-Halocarbonyle reagieren zu Thioestern, F) Thioester und β-Aminothiole reagieren zu β-Mercaptoamiden, F) Mercaptane und Maleinimide reagieren zu Succinimiden. Der Rest R¹ stellt dabei Alkyl-, Alkenyl-, Alkinyl-, Cycloalkyl- oder Aryl-Reste, bzw. Heterocyclen oder Oberflächen dar und die Reste R⁴-R⁶ stellen dabei Alkyl-, Alkenyl-, Alkinyl-, Cycloalkyl- oder Aryl-Reste, bzw. Heterocyclen oder Oberflächen oder H, D, bzw. T dar, wobei Alkyl für verzweigte und unverzweigte C₁₋₂₀-Alkyl, C₃₋₂₀-Cycloalkyl, bevorzugt für verzweigte und unverzweigte C₁₋₁₂-Alkyl, C₃₋₁₂-Cycloalkyl und besonders bevorzugt für verzweigte und unverzweigte C₁₋₆-Alkyl, C₃₋₆-Cycloalkyl-Reste steht. Alkenyl steht für verzweigte und unverzweigte C₂₋₂₀-Alkenyl, verzweigte und unverzweigte C₁₋₂₀-Alkyl-O-C₂₋₂₀-alkenyl, C₁₋₂₀(-O/S-C₂₋₂₀)₂₋₂₀alkenyl, Aryl-C₂₋₂₀-alkenyl, verzweigte und unverzweigte Heterocyclyl- C₂₋₂₀-alkenyl, C₃₋₂₀-Cycloalkenyl, bevorzugt für verzweigte und unverzweigte C₂₋₁₂-Alkenyl, verzweigte und unverzweigte C₁₋₁₂ (-O/S-C₂₋₁₂)₂₋₁₂alkenyl, besonders bevorzugt für verzweigte und unverzweigte C₂₋₆-Alkenyl, verzweigte und unverzweigte C₁₋₆ (-O/S-C₂₋₈)₂₋₈alkenyl-Reste; Alkinyl steht für verzweigte und unverzweigte C₂₋₂₀-Alkinyl, verzweigte und unverzweigte C₁₋₂₀ (-O/S-C₂₋₂₀)₂₋₂₀alkinyl, bevorzugt für verzweigte und unverzweigte C₂₋₁₂-Alkinyl, verzweigte und unverzweigte C₁₋₁₂ (-O/S-C₂₋₁₂)₂₋₁₂alkinyl, besonders bevorzugt für verzweigte und unverzweigte C₂₋₆-Alkinyl, verzweigte und unverzweigte C₁₋₆ (-O/S-C₂₋₈)₂₋₈alkinyl-Reste; Cycloalkyl steht für überbrückte und nicht-überbrückte C₃₋₄₀-Cycloalkyl, bevorzugt für überbrückte und nicht-überbrückte C₃₋₂₆-Cycloalkyl, besonders bevorzugt für überbrückte und nicht-überbrückte C₃₋₁₅-Cycloalkyl-Reste, Aryl steht für substituierte und unsubstituierte mono-oder multi-verknüpfte Phenyl-, Pentalenyl-, Azulenyl-, Anthracenyl-, Indacenyl-, Acenaphtyl, Fluorenyl, Phenalenyl, Phenanthrenyl, bevorzugtfür substituierte und unsubstituierte mono-oder multi-verknüpfte Phenyl-, Pentalenyl-, Azulenyl-, Anthracenyl-, Indenyl-, Indacenyl-, Acenaphtyl-, Fluorenyl, besonders bevorzugt für substituierte und unsubstituierte mono-oder multi-verknüpfte Phenyl-, Pentalenyl-, Anthracenyl-Reste, sowie deren teilhydrierte Derivate. Heterocyclen können sein ungesättigte und gesättigte 3-15-gliedrige mono-bi und tricyclische Ringe mit 1-7 Heteroatomen, bevorzugt 3-10-gliedrige mono-bi und tricyclische Ringe mit 1-5 Heteroatomen und besonders bevorzugt: 5,6 und 10-gliedrige mono-bi und tricyclische Ringe mit 1-3 Heteroatomen.

Zusätzlich können am Alkyl, Alkenyl, Alkinyl, Cycloalkyl, Aryl, Heteroatome, Heterocyclen, Biomolekül oder Naturstoff 0 bis 30 (bervorzugt 0 bis 10, besonders bevorzugt 0 bis 5) der folgende Substituenten einzeln oder in Kombination untereinander auftreten; Fluor, Chlor, Brom, Iod, Hydroxyl, Amid, Ester, Säure, Amin, Acetal, Ketal, Thiol, Ether, Phosphat, Sulfat, Sulfoxyd, Peroxyd, Sulfonsäure, Thioether, Nitril, Harnstoffe, Carbamat, wobei folgende bevorzugt sind: Fluor, Chlor, Brom, Hydroxyl, Amid, Ester, Säure, Amin, Ether, Phosphat, Sulfat, Sulfoxyd, Thioether, Nitril, Harnstoffe, Carbamat, und besonders bevorzugt: Chlor, Hydroxyl, Amid, Ester, Säure, Ether, Nitril.

Fig. 12. Schematisch gezeigt sind verschiedene Ausführungsformen der Inkubation einer Anordnung von Verbindungen (B₁-B₅) auf der Oberfläche eines Trägers mit einem Agens (enzymatische Aktivität, C), das in der Lage ist, unter den gegebenen Bedingungen bei einer oder mehreren Verbindungen im immobilisierten Zustand das Molekulargewicht zu verringern oder zu erhöhen. Dabei sollte die Verbindung zwischer der Oberfläche und den immobilisierten Verbindungen (A) kovalent und regioselektiv sein. Fig. A zeigt eine Ausführungsform, bei der das Agens C auf die Oberfläche aufgegeben wird. Fig. B dagegen zeigt eine Ausführungsform, bei der das Agens C auf zwischen zwei einander zugewandte Oberflächen, die entweder gleiche oder aber ungleiche Anordnungen von immobilisierten Verbindungen beeinhalten können, gegeben wird.

Die nachfolgenden Beispiele betreffen die Funktionalisierung von Glas, dessen Oberfläche als Oberfläche für eine Immobilisierung erforderlich ist (Beispiele 1 bis 14), die Immobilisierung von verschiedenen mit einer reaktiven Gruppe versehenen Peptiden an eine Oberfläche (Beispiele 15 bis 24) und die Analyse von Kinase vermittelter Peptidmodifikation unter Verwendung der erfindungsgemäß immobilisierten Peptide (Beispiele 25-34). Dabei wurden die nachfolgend aufgelisteten Abkürzungen verwendet:
- Ala, A: L-Alanin
- Aoa, O: Aminooxyessigsäure
- Arg, R: L-Arginin
- Asn, N: L-Asparagin
- Asp, D: L-Asparaginsäure
- ATP: Adenosin-5'-triphosphat
- βAla, B, BAL: β-Alanin, 3-Aminopropionsäure
- Boc: tertiär-Butoxycarbonyl
- Cit: L-Citrullin
- Cys, C: L-Cystein
- DCM: Dichlormethan
- DIC: *N*,*N*'-Diisopropylcarbodiimid
- DIPEA: *N*,*N*-Diisopropylethylamin
- DMF: *N*,*N'*-Dimethylformamid
- DMF: *N*,*N*'-Dimethylformamid
- DMSO: Dimthylsulfoxid
- EGTA: Ethylenglycol-bis-(2-aminoethyl)-*N,N,N',N'*-tetraessigsäure
- Et: Ethyl
- Fmoc: 9-Fluorenylmethoxycarbonyl
- Gln, Q: Glutamin
- Glu, E: L-Glutaminsäure
- Gly, G: Glycin
- HBTU: O-(Benzotriazol-1-yl)-*N,N,N',N'*-tetramethyluroniumhexafluorophosphat
- His, H: L-Histidin
- HPLC: high-performance liquid chromatography
- Ile, I: L-Isoleucin
- L: Liter
- Leu, L: L-Leucin
- Lys, K: L-Lysin
- M: molar
- MBHA: Methylbenzhydrylamin
- MBP: myelin basic protein
- MeOH: Methanol
- Met, M: L-Methionin
- mL: Milliliter
- mM: millimolar
- mRNA: messenger RNA
- nL: Nanoliter
- Phe, F: L-Phenylalanin
- Pbf: 2,2,4,6,7-Pentamethyl-dihydrobenzofuran-5-sulfonyl
- Pro, P: L-Prolin
- PTFE: Polytetrafluorethylen
- - PVC: Polyvinylchlorid
- PVDF: Polyvinyldifluorid
- RNA: ribonucleic acid
- RP: reversed-phase
- RT: Raumtemperatur
- SDS: Natriumlaurylsulfat
- Ser, S: L-Serin
- tBu: tertiär-Butyl
- TFA: Trifluoressigsäure
- THF: Tetrahydrofuran
- Thr, T: L-Threonin
- Tris: 2-Amino-2-hydroxymethyl-1,3-propandiol
- Trp, W: L-Tryptophan
- Tween20: Polyoxyethylen-Sorbitant-Monolaurat (Wz. der Fa. Atlas Chemie)
- Tyr, Y: L-Tyrosin
- U: unit
- Val, V: L-Valin

Dabei wurden die folgenden Reagenzien und Lösungsmittel benutzt:
Brom, tert.-Butyl-Methyl-Ether, 1,3-Diisopropylcarbodiimid, N,N-Diisopropylethylamin Eisessig, Glycerol, Harnstoff, 40%ige Hydroxylaminlösung, Piperidin, Triethylamin, Dichlormethan, Diethylether, N,N-Dimethylformamid, Ethanol, Methanol, und Tetrahydrofuran stammen von Merck Eurolab (Darmstadt, Deutschland). Oxalylchlorid, Natriumthiocyanat, Trifluoressigsäure, Dimethylsulfoxid, Thioacetamid, Lawessons Reagenz, Ameisensäure, und Thioharnstoff wurden von Fluka (Deisenhofen, Deutschland) bezogen. Adenosin-5'-triphosphat, 2-Amino-2-hydroxymethyl-1,3-propandiol-hydrochlorid, Natriumchlorid, Magnesiumchlorid, 1,4-dithio-DL-threitol, Natriumlaurylsulfat, Polyoxyethylen-Sorbitant-Monolaurat, und Ethylenglycol-bis-(2-aminoethyl)-N,N,N',N'-tetraessigsäure stammen von Sigma (Taufkirchen, Deutschland). Das Rink-Amid-MBHA-Harz, (Benzotriazol-1-yl)-N,N,N',N'tetramethyluroniumhexafluorophosphat, sowie alle Fmoc-Aminosäure-pentafluorphenylester wurde bei der Firma Novabiochem (Bad Soden, Deutschland) bezogen.
Zur SPOT-Synthese wurden Zellulose Membranen "Whatman 50" (Whatman Maidstone, UK) verwendet.

### Chromatographie und physikalische Daten:

RP-18-HPLC-MS-Analysen wurden durch Chromatographie unter Verwendung eines Hewlett Packard Serie 1100-Systems (Entgaser G1322A, Quaternäre Pumpe G1311A, Automatischer Probengeber G1313A, Thermostatisiertes Säulenfach G 1316A, Variabler UV-Detektor G1314A) und gekoppelter ESI-MS (Finnigan LCQ Ion-Trap-Massenspektrometer) durchgeführt. Die Trennung erfolgte an RP-18-Säulenmaterial (Vydac 218 TP5215, 2.1x150 mm, 5 µm, C18, 300 A mit Vorsäule) bei 30°C und einem Fluß von 0.3 mL/min unter Anwendung eines linearen Gradienten für alle Chromatogramme (5-95% B innerhalb von 25 min, wobei A: 0.05% TFA in Wasser und B: 0.05% TFA in CH₃CN). Die UV-Detektion erfolgte bei λ=220 nm.

Präparative HPLC wurde mit einem System der Firma Merck/Hitachi (Quaternäre Pumpe L-6250, Variabler UV-Detektor L-/400, Interface D-7000, Software: HPLC Systemmanager D-7000 für NT 4.0) unter Verwendung einer Säule der Firma Merck Eurolab (LiChrospher 100, RP18, 10x250 mm) bei einem Lösungsmittelfluß von 6.0 mL/min durchgeführt. Das verwendete Lösungsmittelsystem setzte sich aus den Komponenten A (H₂O/0.1 Vol-% TFA) und B (CH₃CN/0.1·Vol-% TFA) zusammen.

### Geräte zur Herstellung der löslichen Peptide:

Die zur Immobilisierung verwendeten Peptide wurden als C-terminale Peptidamide mit Hilfe eines parallelen Syntheseautomaten "Syro" (MultiSynTech, Witten, Deutschland) nach dem Standard Fmoc-Protokoll an Rink-Amid-MBHA-Harz synthetisiert. Alle erhaltenen Peptide wurden, nach Spaltung vom Harz und Abspaltung aller Schutzgruppen, mittels HPLC-MS analysiert und zeigten die gewünschten Molekülionensignale. Nach anschließender HPLC-Reinigung wurden die Peptide lyophilisiert und bei -20°C gelagert.

Die zur Imobilisierung verwendeten Peptide (13mere Peptide der Proteine MBP, Casein, Histon H1) wurden nach der Standard-SPOT-Synthesemethode automatisch mit dem Gerät Autospot AMS 222 (Abimed, Langenfeld, Deutschland) unter Anwendung der Steuersoftware Autospot XL Ver. 2.02 durchgeführt. Die Waschschritte erfogten in Edelstahlschalen (Merck Eurolab), die auf einem Wipptisch bewegt wurden.

### Beispiele

### Beispiel 1: Maleinimido-Funktionalisierung aminopropylsilylierter Glasoberflächen

4-Maleinimidobuttersäure (Fluka, 63174) wurde 0.3 M in DMF gelöst. Die resultierende Mischung wurde durch die Zugabe von einem halben Äquivalent Di-iso-propylcarbodiimid (DIC) 15 min bei Raumtemperatur (RT) aktiviert. Mit Druckluft gereinigte aminopropylsilylierte Glasoberflächen (2.5 x 7.5 cm; Sigma, Silane-Prep™, S4651) wurden mit der so erhaltenen Maleinimidobuttersäureanhydrid-Lösung überschichtet und drei Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL Dichlormethan (DCM) bei RT wurden die Glasoberflächen getrocknet und bis zur weiteren Verwendung bei 4°C gelagert.

### Beispiel 2: Maleinimido-Funktionalisierung poly-lysinmodifizierter Glasoberflächen

6-Maleinimidocapronsäure (Fluka, 63176) wurde 0.3 M in DMF gelöst. Die resultierende Mischung wurde durch die Zugabe von einem halben Äquivalent DIC 15 min bei Raumtemperatur (RT) aktiviert. Mit Druckluft gereinigte poly-lysinmodifizierte Glasoberflächen (Sigma, Poly-Prep™, P0425, 2.5 x 7.5 cm) wurden mit der so erhaltenen Maleinimidocapronsäureanhydrid-Lösung drei Stunden bei RT inkubiert. Dabei wurden 60 µL dieser Lösung durch Kapillarkräfte in den Spalt, der durch zwei aufeinanderliegende modifizierte Glasoberflächen gebildet wird, aufgegeben. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 3: Aldehyd-Funktionalisierung aminopropylsilylierter Glasoberflächen

4-Carboxybenzaldehyd (Fluka, 21873) wurde 0.3 M in DMF gelöst. Die resultierende Mischung wurde durch die Zugabe von einem halben Äquivalent DIC 15 min bei RT aktiviert. Mit Druckluft gereinigte aminopropylsilylierte Glasoberflächen (2.5 x 7.5 cm; Sigma, Silane-Prep™, S4651) wurden mit der so erhaltenen aktivierten Carboxybenzaldehyd-Lösung überschichtet und drei Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 4: Keton-Funktionalisierung aminopropylsilylierter Glasoberflächen

Lävulinsäure (Fluka, 61380) wurde 0.3 M in DMF gelöst. Die resultierende Mischung wurde durch die Zugabe von einem halben Äquivalent DIC 15 min bei RT aktiviert. Mit Druckluft gereinigte aminopropylsilylierte Glasoberflächen (2.5 x 7.5 cm; Sigma, Silane-Prep™, S4651) wurden mit der so erhaltenen Lävulinsäureanhydrid-Lösung überschichtet und drei Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 5: Bromacetylierung aminopropylsilylierter Glasoberflächen

Bromessigsäuresäure wurde 0.4 M in DMF gelöst. Die resultierende Mischung wurde durch die Zugabe von einem halben Äquivalent DIC 15 min bei RT aktiviert. Mit Druckluft gereinigte aminopropylsilylierte Glasoberflächen (2.5 x 7.5 cm; Sigma, Silane-Prep™, S4651) wurden mit der so erhaltenen Bromessigsäureanhydrid-Lösung überschichtet und drei Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 6: 4-Bromomethylbenzoesäure-Funktionalisierung aminopropylsilylierter Glasoberflächen

4-Bromomethylbenzoesäure wurde 0.3 M in DMF gelöst. Die resultierende Mischung wurde durch die Zugabe von einem halben Äquivalent DIC 15 min bei RT aktiviert. Mit Druckluft gereinigte aminopropylsilylierte Glasoberflächen (2.5 x 7.5 cm; Sigma, Silane-Prep™, S4651) wurden mit der so erhaltenen 4-Bromomethylbenzoesäureanhydrid-Lösung überschichtet und drei Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 7: Phenylthioharnstoff-Funktionalisierung aminopropylsilylierter Glasoberflächen

4-Carboxyphenylthioharstoff (Lancaster, 13047) wurde 0.2 M in DMF gelöst. Die resultierende Mischung wurde durch die Zugabe von einem halben Äquivalent DIC 15 min bei RT aktiviert. Mit Druckluft gereinigte aminopropylsilylierte Glasoberflächen (2.5 x 7.5 cm; Sigma, Silane-Prep™, S4651) wurden mit der so erhaltenen aktivierten Lösung überschichtet und drei Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 8: Thioamid-Funktionalisierung aminopropylsilylierter Glasoberflächen

Bemsteinsäure-mono-thioamid wurde 0.2 M in DMF gelöst. Die resultierende Mischung wurde durch die Zugabe von einem halben Äquivalent DIC 15 min bei RT aktiviert. Mit Druckluft gereinigte aminopropylsilylierte Glasoberflächen (2.5 x 7.5 cm; Sigma, Silane-Prep™, S4651) wurden mit der so erhaltenen modifizierten Bernsteinsäureanhydrid-Lösung überschichtet und drei Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 9: Bromoketon-Funktionalisierung aminopropylsilylierter Glasoberflächen

1,4-Dibrom-2,3-Diketobutan (Aldrich, D3,916-9) wurde 0.2 M in DMF 0.1% Triethylamin gelöst. Mit Druckluft gereinigte aminopropylsilylierte Glasoberflächen (2.5 x 7.5 cm; Sigma, Silane-Prep™, S4651) wurden mit Lösung überschichtet und sieben Stunden bei RT inkubiert. Anschließend wurden die so behandelten Glasoberflächen fünfmal mit jeweils 30 mL DMF je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 10: Bromoketon-Funktionalisierung thioamidmodifizierter Glasoberflächen

Die in diesem Beispiel dargestellten Strukturen ermöglichen eine einfache und mit guten Ausbeuten verlaufende Oberflächenmodifikation

Mit Bernsteinsäure-mono-thioamid umgesetzte aminopropylsilylierte Glasoberflächen (2.5 x 7.5 cm; Sigma, Silane-Prep™, S4651) (vgl. Beispiel 8) wurden mit einer 0.1 M 1,4-Dibrom-2,3-Diketobutan-Lösung (Aldrich, D3,916-9) in Ethanol überschichtet und drei Stunden bei RT inkubiert. Anschließend wurden die so behandelten Glasoberflächen fünfmal mit jeweils 30 mL Ethanol je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 11: Bromoketon-Funktionalisierung phenylthioharstoffmodifizierter Glasoberflächen

Die in diesem Beispiel dargestellten Strukturen ermöglichen eine einfache und mit guten Ausbeuten verlaufende Oberflächenmodifikation

Mit 4-Carboxyphenylthioharstoff umgesetzte aminopropylsilylierte Glasoberflächen (2.5 x 7.5 cm; Sigma, Silane-Prep™, S4651) wurden mit einer 0.1 M 1,4-Dibrom-2,3-Diketobutan-Lösung in Ethanol überschichtet und drei Stunden bei RT inkubiert. Anschließend wurden die so behandelten Glasoberflächen fünfmal mit jeweils 30 mL Ethanol je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 12: Brombrenztraubensäure-Funktionalisierung aminopropylsilylierter Glasoberflächen

Diese Oberflächenmodifikation zeigt, daß es möglich, auch sehr kleine Strukturen zur Umwandlung einer aminofunktionalisierten in eine bromketonfunktionalisierte Glasoberfläche zu benutzten. Dabei stellt die Brombrenztraubensäure die kleinste mögliche Verbindung dar, die sowohl die für die Amidbindungsknüpfung notwendige Carboxyl-Funktion als auch die für die nachfolgende Immobilisierung des Biomoleküls notwendige alpha-Brom-Keto-Funktion enthält.

Natriumpyruvat wurde mit Oxalylchlorid in in das entsprechende Säurechlorid überfuhrt. Mit Druckluft gereinigte aminopropylsilylierte Glasoberflächen (2.5 x 7.5 cm; Sigma, Silane-Prep™, S4651) wurden mit der so erhaltenen Lösung überschichtet und 5 Stunden bei RT inkubiert. Anschließend wurden die so behandelten Glasoberflächen fünfmal mit jeweils 30 mL DMF je 3 Minuten bei RT gewaschen. Nach jeweils dreimaligem Waschen je drei Minuten mit jeweils 30 mL Methanol und DCM bei RT wurden die Glasoberflächen getrocknet. Die so erhaltenen Brenztraubensäure-modifizierten Glasoberflächen wurden durch einstündige Behandlung mit einer Lösung von 0.1 mL Brom in 10 mL Eisessig in die Brombrenztraubensäure-modifizierten Glasoberflächen überführt. Nach jeweils dreimaligem Waschen je drei Minuten mit jeweils 30 mL Methanol und DCM bei RT wurden die Glasoberflächen getrocknet und bis zur weiteren Verwendung bei 4°C gelagert.

### Beispiel 13: Bromacetophenon-Funktionalisierung aminopropylsilylierter Glasoberflächen

4-Acetylbenzoesäure wurde 0.3 M in DMF gelöst. Die resultierende Mischung wurde durch die Zugabe von einem halben Äquivalent DIC 15 min bei RT aktiviert. Mit Druckluft gereinigte aminopropylsilylierte Glasoberflächen (2.5 x 7.5 cm; Sigma, SiIane-Prep™, S4651) wurden mit der so erhaltenen 4-Acetylbenzoesäureanhydrid-Lösung überschichtet und 3 Stunden bei RT inkubiert. Anschließend wurden die so behandelten Glasoberflächen fünfmal mit jeweils 30 mL DMF je 3 Minuten bei RT gewaschen. Nach jeweils dreimaligem Waschen je drei Minuten mit jeweils 30 mL Methanol und DCM bei RT wurden die Glasoberflächen getrocknet. Die so modifizierten Glasoberflächen wurden durch einstündige Behandlung mit einer Lösung von 0.1 mL Brom in 10 mL Eisessig in die Bromacetophenon-modifizierten Glasoberflächen überführt. Nach jeweils dreimaligem Waschen je drei Minuten mit jeweils 30 mL Methanol und DCM bei RT wurden die Glasoberflächen getrocknet und bis zur weiteren Verwendung bei 4°C gelagert.

### Beispiel 14: Thiocyanato-acetophenon-Funktionalisierung aminopropylsilylierter Glasoberflächen

Bromacetophenon-modifizierte, aminopropylsilylierte Glasoberflächen (2.5 x 7.5 cm; Sigma, Silane-Prep™, S4651) (vgl. Beispiel 13) wurden mit einer 0.1 M Lösung von Natriumthiocyanat in Ethanol überschichtet und fünf Stunden bei 50°C inkubiert. Anschließend wurden die so behandelten Glasoberflächen fünfmal mit jeweils 30 mL Ethanol je 3 Minuten bei RT gewaschen. Nach dreimaligem Waschen je drei Minuten mit jeweils 30 mL DCM bei RT wurden die Glasoberflächen getrocknet und bis zur weiteren Verwendung bei 4°C gelagert.

### Beispiel 15: Immobilisierung cysteinhaltiger Peptide an maleinimido-funktionalisierten Glasoberflächen

a) Die zur Immobilisierung verwendeten Peptide wurden nach Standardmethoden der Fmoc-basierenden Chemie an der festen Phase als C-terminale Peptidamide synthetisiert. Dabei wurden entsprechend geschützte Fmoc-Aminosäuren mit einem Äquivalent HBTU und drei Äquivalenten Diisopropylethylamin in DMF aktiviert und an Rink-Amind-MBHA-Harz in DMF gekoppelt. Die Abspaltung der Fmoc-Schutzgruppe erfolgte unter Verwendung von 20% Piperidin in DMF 30 min bei RT. Die Abspaltung der permanenten Schutzgruppen(tBu für Serin, Threonin, Tyrosin, Glutaminsäure und Asparaginsäure; Boc für Lysin; Trityl für Asparagin, Glutamin, Cystein, Histidin und Pbf für Arginin) und die gleichzeitige Ablösung vom Polymer erfolgte durch zweistündige Behandlung mit 97% Trifluoressigsäure bei RT. Die resultierende Mischung wurde filtriert und das Filtrat durch Zugabe von tert.Butyl-Methyl-Ether gefällt. Der Niederschlag wurde abgetrennt und mittels HPLC an RP18-Material mit Acetonitril/Wasser Mischungen (0.1% Trifluoressigsäure) gereinigt. Die das gewünschte Produkt enthaltenden Fraktionen wurden lyophilisiert und bis zur weiteren Verwendung bei -20°C gelagert.
b) Die HPLC-gereinigten cysteinhaltigen Peptide wurden in 200 mM Natrium-Phosphatpuffer pH 7.5 gelöst (finale Konzentation der Peptide 10 mM). Anschließend wurde bei RT jeweils 1 µL dieser Lösung auf die maleinimido-funktionalisierten Glasoberflächen (vgl. Beispiel 1) mittels einer Eppendorf-Pipette aufgespottet und diese eine Stunde bei RT in einer nahezu wassergesättigten Atmosphäre inkubiert. Nach dreimaligem Waschen bei RT mit jeweils 100 mL destilliertem Wasser wurden die modifizierten Glasoberflächen mit 30 mL einer 300 mM Lösung von Mercaptoethanol in 200 mM Natrium-Phosphatpuffer pH 7.5 inkubiert, um die restlichen Maleinimidofunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 16: Immobilisierung cysteinhaltiger Peptide an bromacetylierten Glasoberflächen

Die HPLC-gereinigten cysteinhaltigen Peptide wurden in 200 mM Natrium-Phosphatpuffer pH 6.5 gelöst (finale Konzentation der Peptide 5 mM). Anschließend wurde bei RT jeweils 1 µL dieser Lösung auf die funktionalisierten Glasoberflächen (vgl. Beispiel 5) mittels einer Eppendorf-Pipette aufgespottet und diese eine Stunde bei RT in einer nahezu wassergesättigten Atmosphäre inkubiert. Nach dreimaligem Waschen bei RT mit jeweils 100 mL destilliertem Wasser wurden die modifizierten Glasoberflächen mit 30 mL einer 300 mM Lösung von Mercaptoethanol in 200 mM Natrium-Phosphatpuffer pH 7.5 inkubiert, um die restlichen Maleinimidofunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 17: Immobilisierung cysteinhaltiger Peptide an aldehyd-funktionalisierten Glasoberflächen

Die gereinigten cysteinhaltigen Peptide wurden in 200 mM Natrium-Phosphatpuffer pH 5.5 gelöst (finale Konzentation der Peptide 5 mM), der 200 mM Tris-carboxyethylphosphin enthält. Anschließend wurde bei RTjeweils 1 µL dieser Lösung auf die aldehyd-funktionalisierten Glasoberflächen (vgl. Beispiel 3) mittels einer Eppendorf-Pipette aufgespottet und diese vier Stunden bei RT in einer nahezu wassergesättigten Atmosphäre inkubiert. Nach dreimaligem Waschen bei RT mit jeweils 100 mL destilliertem Wasser wurden die modifizierten Glasoberflächen mit 30 mL einer 40%igen wässrigen Lösung Hydroxylamin 30 Minuten bei RT inkubiert, um die restlichen Aldehydfunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 18: Immobilisierung cysteinhaltiger Peptide an maleinimido-funktionalisierten Glasoberflächen

a) Die zur Immobilisierung verwendeten Peptide (43 serin/threonin-haltige Peptide und die entsprechenden 43 Kontrollpeptide) wurden nach Standard-SPOT-Synthesemethoden (R.Frank, *Tetrahedron,* ***48***, *1992*, S.9217-9232; A.Kramer und *J*. Schneider-Mergener, Methods in Molecular Biology, vol 87: Combinatorial Peptide Library Protocols, S. 25-39, edited by: S. Cabilly; Humana Press Inc., Totowa, NJ) an Cellulose als C-terminale Peptidamide synthetisiert. Dabei wurden entsprechend geschützte Fmoc-Aminosäure-pentafluorphenylester in DMF gelöst und jeweils 1µL aufgespottet. Die Kupplungsreaktion erfolgte doppelt jeweils 25 min bei RT. Die Abspaltung der Fmoc-Schutzgruppe erfolgte unter Verwendung von 20% Piperidin in DMF 20 min bei RT. Die Abspaltung der permanenten Schutzgruppen (tBu für Serin, Threonin, Tyrosin, Glutaminsäure und Asparaginsäure; Boc für Lysin; Trityl für Asparagin, Glutamin, Cystein, Histidin und Pbf für Arginin) erfolgte durch zweistündige Behandlung mit 97% Trifluoressigsäure bei RT. Anschließend wurden die cellulose-gebundenen Peptide mit DCM, MeOH und Diethylether gewaschen und im Vakuum getrocknet. Die Abspaltung der Peptide von der Cellulose erfolgte unter Verwendung von Ammoniakgas 24 Stunden bei RT. Die Spots mit den physikalisch adsorbierten Peptiden wurden ausgestanzt und in 96-well Mikrotiterplatten überführt. Nach Ablösen der Peptide mit je 200 µL 20% Methanol unter Ultraschallbedingungen wurden die Proben filtriert, in 384well Mikrotiterplatten überführt, lyophilisiert und bis zur weiteren Verwendung bei -20°C gelagert. Die nachfolgende Liste gibt eine Übersicht bezüglich der synthetisierten Peptidsequenzen und erlaubt gleichzeitig die Zuordnung der Peptidnummern in den Fig. 8 und 9 zu den entsprechenden Sequenzen (BA1=β-Alanin). 
1 Cys-BAl-Lys-Lys-Ala-Leu-Arg-Arg-Gln-Glu-Thr-Val-Asp-Ala-Leu-NH2
2 Cys-BAl-Lys-Lys-Ala-Leu-Arg-Arg-Gln-Glu-Ala-Val-Asp-Ala-Leu-NH2
3 Cys-BAl-Ala-Lys-Arg-Arg-Arg-Leu-Ser-Ser-Leu-Arg-Ala-NH2
4 Cys-BAl-Ala-Lys-Arg-Arg-Arg-Leu-Ala-Ala-Leu-Arg-Ala-NH2
5 Cys-BAl-Gly-Arg-Thr-Gly-Arg-Arg-Asn-Ser-Ile-NH2
6 Cys-BAl-Gly-Arg-Ala-Gly-Arg-Arg-Asn-Ala-Ile-NH2
7 Cys-BAl-Asp-Asp-Asp-Glu-Glu-Ser-Ile-Thr-Arg-Arg-NH2
8 Cys-BAl-Asp-Asp-Asp-Glu-Glu-Ala-Ile-Ala-Arg-Arg-NH2
9 Cys-BAl-Glu-Arg-Ser-Pro-Ser-Pro-Ser-Phe-Arg-NH2
10 Cys-BAl-Glu-Arg-Ala-Pro-Ala-Pro-Ala-Phe-Arg-NH2
11 Cys-BAl-Gly-Arg-Pro-Arg-Thr-Ser-Ser-Phe-Ala-Glu-Gly-NH2
12 Cys-BAl-Gly-Arg-Pro-Arg-Ala-Ala-Ala-Phe-Ala-Glu-Gly-NH2
13 Cys-BAl-Lys-Lys-Lys-Ala-Leu-Ser-Arg-Gln-Leu-Ser-Val-Ala-Ala-NH2
14 Cys-BAl-Lys-Lys-Lys-Ala-Leu-Ala-Arg-Gln-Leu-Ala-Val-Ala-Ala-NH2
15 Cys-BAl-Lys-Lys-Leu-Asn-Arg-Thr-Leu-Ser-Val-Ala-NH2
16 Cys-BAl-Lys-Lys-Leu-Asn-Arg-Ala-Leu-Ala-Val-Ala-NH2
17 Cys-BAl-Lys-Arg-Gln-Gln-Ser-Phe-Asp-Leu-Phe-NH2
18 Cys-BAl-Lys-Arg-Gln-Gln-Ala-Phe-Asp-Leu-Phe-NH2
19 Cys-BAl-Lys-Arg-Arg-Glu-Ile-Leu-Ser-Arg-Arg-Pro-Ser-Tyr-Arg-NH2
20 Cys-BAl-Lys-Arg-Arg-Glu-Ile-Leu-Ala-Arg-Arg-Pro-Ala-Phe-Arg-NH2
21 Cys-BAl-Leu-Arg-Ala-Pro-Ser-Trp-Ile-Asp-Thr-NH2
22 Cys-BAl-Leu-Arg-Ala-Pro-Ala-Trp-Ile-Asp-Ala-NH2
23 Cys-BAl-Pro-Leu-Ser-Arg-Thr-Leu-Ser-Val-Ala-Ala-Lys-Lys-NH2
24 Cys-BAl-Pro-Leu-Ala-Arg-Ala-Leu-Ala-Val-Ala-Ala-Lys-Lys-NH2
25 Cys-BAl-Pro-Leu-Ser-Arg-Thr-Leu-Ser-Val-Ser-Ser-NH2
 
26 Cys-BAl-Pro-Leu-Ala-Arg-Ala-Leu-Ala-Val-Ala-Ala-NH2
27 Cys-BAl-Gln-Lys-Arg-Pro-Ser-Gln-Arg-Ser-Lys-Tyr-Leu-NH2
28 Cys-BAl-Gln-Lys-Arg-Pro-Ala-Gln-Arg-Ala-Lys-Phe-Leu-NH2
29 Cys-BAl-Arg-Lys-Ile-Ser-Ala-Ser-Glu-Phe-NH2
30 Cys-BAl-Arg-Lys-Ile-Ala-Ala-Ala-Glu-Phe-NH2
31 Pro-Lys-Thr-Pro-Lys-Lys-Ala-Lys-Lys-Leu-BAl-Cys-NH2
32 Pro-Lys-Ala-Pro-Lys-Lys-Ala-Lys-Lys-Leu-BAl-Cys-NH2
33 Cys-BAl-Arg-Pro-Arg-Ala-Ala-Thr-Phe-NH2
34 Cys-BAl-Arg-Pro-Arg-Ala-Ala-Ala-Phe-NH2
35 Cys-BAl-Arg-Arg-Arg-Ala-Pro-Leu-Ser-Pro-NH2
36 Cys-BAl-Arg-Arg-Arg-Ala-Pro-Leu-Ala-Pro-NH2
37 Cys-BAl-Arg-Arg-Arg-Glu-Glu-Glu-Thr-Glu-Glu-Glu-NH2
38 Cys-BAl-Arg-Arg-Arg-Glu-Glu-Glu-Ala-Glu-Glu-Glu-NH2
39 Cys-BAl-Met-His-Arg-Gln-Glu-Thr-Val-Asp-Cys-Leu-Lys-NH2
40 Cys-BAl-Met-His-Arg-Gln-Glu-Ala-Val-Asp-Cys-Leu-Lys-NH2
41 Cys-BAl-Lys-Lys-Arg-Phe-Ser-Phe-Lys-Lys-Ser-Phe-Lys-Leu-NH2
42 Cys-BAl-Lys-Lys-Arg-Phe-Ala-Phe-Lys-Lys-Ala-Phe-Lys-Leu-NH2
43 Cys-BAl-Pro-Lys-Asp-Pro-Ser-Gln-Arg-Arg-Arg-NH2
44 Cys-BAl-Pro-Lys-Asp-Pro-Ala-Gln-Arg-Arg-Arg-NH2
45 Cys-BAl-Ile-Ala-Ala-Asp-Ser-Glu-Ala-Glu-Gln-NH2
46 Cys-BAl-Ile-Ala-Ala-Asp-Ala-Glu-Ala-Glu-Gln-NH2
47 Cys-BAl-Ser-Pro-Ala-Leu-Thr-Gly-Asp-Glu-Ala-NH2
48 Cys-BAl-Ala-Pro-Ala-Leu-Ala-Gly-Asp-Glu-Ala-NH2
49 Cys-BAl-Gly-Arg-Ile-Leu-Thr-Leu-Pro-Arg-Ser-NH2
50 Cys-BAl-Gly-Arg-Ile-Leu-Ala-Leu-Pro-Arg-Ala-NH2
51 Cys-BAl-Met-Gly-Glu-Ala-Ser-Gly-Cys-Gln-Leu-NH2
52 Cys-BAl-Met-Gly-Glu-Ala-Ala-Gly-Cys-Gln-Leu-NH2
53 Cys-BAl-Glu-Glu-Thr-Pro-Tyr-Ser-Tyr-Pro-Thr-NH2
54 Cys-BAl-Glu-Glu-Ala-Pro-Phe-Ser-Phe-Pro-Ala-NH2
55 Cys-BAl-Gly-Asn-His-Thr-Tyr-Gln-Glu-Ile-Ala-NH2
56 Cys-BAl-Gly-Asn-His-Ala-Phe-Gln-Glu-Ile-Ala-NH2
57 Leu-Arg-Ser-Pro-Ser-Trp-Glu-Pro-Phe-BAl-Cys-NH2
58 Leu-Arg-Ala-Pro-Ala-Trp-Glu-Pro-Phe-BAl-Cys-NH2
59 Ser-Ser-Pro-Val-Tyr-Gln-Asp-Ala-Val-BAl-Cys-NH2
 
60 Ala-Ala-Pro-Val-Phe-Gln-Asp-Ala-Val-BAl-Cys-NH2
61 Cys-BAl-Ser-Arg-Thr-Leu-Ser-Val-Ser-Ser-Leu-NH2
62 Cys-BAl-Ala-Arg-Ala-Leu-Ala-Val-Ala-Ala-Leu-NH2
63 Leu-Ser-Val-Ser-Ser-Leu-Pro-Gly-Leu-BAl-Cys-NH2
64 Leu-Ser-Val-Ala-Ala-Leu-Pro-Gly-Leu-BAl-Cys-NH2
65 Cys-BAl-Val-Thr-Pro-Arg-Thr-Pro-Pro-Pro-Ser-NH2
66 Cys-BAl-Val-Ala-Pro-Arg-Ala-Pro-Pro-Pro-Ala-NH2
67 Cys-BAl-Arg-Phe-Ala-Arg-Lys-Gly-Ser-Leu-Arg-Gln-Lys-Asn-Val-NH2
68 Cys-BAl-Arg-Phe-Ala-Arg-Lys-Gly-Ala-Leu-Arg-Gln-Lys-Asn-Val-NH2
69 Cys-BAl-Pro-Arg-Pro-Ala-Ser-Val-Pro-Pro-Ser-NH2
70 Cys-BAl-Pro-Arg-Pro-Ala-Ser-Ala-Val-Pro-Pro-Ala-NH2
71 Cys-BAl-Arg-Glu-Ala-Arg-Ser-Arg-Ala-Ser-Thr-NH2
72 Cys-BAl-Arg-Glu-Ala-Arg-Ala-Arg-Ala-Ala-Ala-NH2
73 Gln-Ser-Tyr-Ser-Ser-Ser-Gln-Arg-Val-BAl-Cys-NH2
74 Gln-Ser-Tyr-Ala-Ala-Ala-Gln-Arg-Val-BAl-Cys-NH2
75 Cys-BAl-Gly-Gly-Gly-Thr-Ser-Pro-Val-Phe-Pro-NH2
76 Cys-BAl-Gly-Gly-Gly-Ala-Ala-Pro-Val-Phe-Pro-NH2
77 Leu-Tyr-Ser-Ser-Ser-Pro-Gly-Gly-Ala-BAl-Cys-NH2
78 Leu-Tyr-Ala-Ala-Ala-Pro-Gly-Gly-Ala-BAl-Cys-NH2
79 Cys-BAl-Asp-Leu-Pro-Leu-Ser-Pro-Ser-Ala-Phe-NH2
80 Cys-BAl-Asp-Leu-Pro-Leu-Ala-Pro-Ala-Ala-Phe-NH2
81 Cys-BAl-Thr-Thr-Pro-Leu-Ser-Pro-Thr-Arg-Leu-NH2
82 Cys-BAl-Ala-Ala-Pro-Leu-Ala-Pro-Ala-Arg-Leu-NH2
83 Arg-Arg-Ile-Ser-Lys-Asp-Asn-Pro-Asp-Tyr-Gln-Gln-Asp-BAl-Cys-NH2
84 Arg-Arg-Ile-Ala-Lys-Asp-Asn-Pro-Asp-Tyr-Gln-Gln-Asp-BAl-Cys-NH2
85 Cys-BAl-Leu-Arg-Arg-Ala-Ser-Leu-Gly-NH2
86 Cys-BAl-Leu-Arg-Arg-Ala-Ala-Leu-Gly-NH2
b) Die Aufbringung der in Mikrotiterplatten vorliegenden cysteinhaltigen Peptide auf die maleinimido-funktionalisierten Glasoberflächen (vgl. Beispiel 1) erfolgte mittels eines NanoPlotters der Firma Gesim. Dabei betrug der Spot-zu-Spot-Abstand 1 mm. Pro Spot wurden 0.8 nL einer Peptidlösung in 100 mM PBS Puffer pH 7.8, die 20 % Glycerin enthielt, aufgebracht und diese Anordnung vier Stunden bei RT inkubiert. Nach dreimaligem Waschen bei RT mit jeweils 100 mL destilliertem Wasser wurden die modifizierten Glasoberflächen mit 30 mL einer 300 mM Lösung von Mercaptoethanol in 200 mM Natrium-Phosphatpuffer pH 7.5 inkubiert, um die restlichen Maleinimidofunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 19: Immobilisierung von Anthraniloyl-peptiden an aldehyd-funktionalisierten Glasoberflächen

a) Die zur Immobilisierung verwendeten Peptide (jeweils 13mere Peptide, die die gesamte Primärstruktur der Proteine MBP, Casein und Histon H1 repräsentieren) wurden nach Standard-SPOT-Synthesemethoden (R.Frank, *Tetrahedron,* **48**, 1992, S.9217-9232; A.Kramer und J. Schneider-Mergener, Methods in Molecular Biology, vol 87: Combinatorial Peptide Library Protocols, S. 25-39, edited by: S. Cabilly; Humana Press Inc., Totowa, NJ) an Cellulose als C-terminale Peptidamide synthetisiert. Dabei wurden entsprechend geschützte Fmoc-Aminosäure-pentafluorphenylester in DMF gelöst und jeweils 1µL aufgespottet. Die Kupplungsreaktion erfolgte doppelt jeweils 25 min bei RT. Die Abspaltung der Fmoc-Schutzgruppe erfolgte unter Verwendung von 20% Piperidin in DMF 20 min bei RT. Nach der letzten Fmoc-Abspaltung wurden die N-Termini der cellulose-gebundenen Peptide durch eine fünfstündige Inkubation mit einer gesättigten Lösung von Isatursäure in DMF bei 50°C in die entsprechenden 2-aminobezoylierten Derivate überführt. Die Abspaltung der permanenten Schutzgruppen (tBu für Serin, Threonin, Tyrosin, Glutaminsäure und Asparaginsäure; Boc für Lysin; Trityl für Asparagin, Glutamin, Cystein, Histidin und Pbf für Arginin) erfolgte durch zweistündige Behandlung mit 97% Trifluoressigsäure bei RT. Anschließend wurden die cellulose-gebundenen Peptide mit DCM, MeOH und Diethylether gewaschen und im Vakuum getrocknet. Die Abspaltung der Peptide von der Cellulose erfolgte unter Verwendung von Ammoniakgas 24 Stunden bei RT. Die Spots mit den physikalisch adsorbierten Peptiden wurden ausgestanzt und in 96-well Mikrotiterplatten überführt. Nach Ablösen der Peptide mit je 200 µL 20% Methanol unter Ultraschallbedingungen wurden die Proben filtriert, in 384well Mikrotiterplatten überführt, lyophilisiert und bis zur weiteren Verwendung bei -20°C gelagert.
b) Die in Mikrotiterplatten vorliegenden Anthraniloyl-peptide wurden in 200 mM Natrium-Phosphatpuffer pH 6.0 gelöst (finale Konzentation der Peptide 0.5 mM), der 15 Vol% DMSO enthielt. Anschließend wurde bei RT jeweils 0.01 µL dieser Lösung auf die aldehydmodifizierten Glasoberflächen (vgl. Beispiel 3) mittels eines NanoPlotters der Firma Gesim aufgebracht und diese vier Stunden bei RT inkubiert. Nach dreimaligem Waschen bei RT mit jeweils 100 mL destilliertem Wasser wurden die modifizierten Glasoberflächen mit 30 mL einer 40%igen wässrigen Lösung Hydroxylamin 30 Minuten bei RT inkubiert, um die restlichen Aldehydfunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 20: Immobilisierung aminooxyessigsäurehaltiger Peptide an aldehyd-funktionalisierten Glasoberflächen

a) Die zur Immobilisierung verwendeten Peptide (jeweils 13mere Peptide, die die gesamte Primärstruktur der Proteine MBP, Casein und Histon H1 repräsentieren) wurden nach Standard-SPOT-Synthesemethoden (R.Frank, *Tetrahedron*, **48**, 1992, S.9217-9232; A.Kramer und J. Schneider-Mergener, Methods in Molecular Biology, vol 87: Combinatorial Peptide Library Protocols, S. 25-39, edited by: S. Cabilly; Humana Press Inc., Totowa, NJ) an Cellulose als C-terminale Peptidamide synthetisiert. Dabei wurden entsprechend geschützte Fmoc-Aminosäure-pentafluorphenylester in DMF gelöst und jeweils 1µL aufgespottet. Die Kupplungsreaktion erfolgte doppelt jeweils 25 min bei RT. Die Abspaltung der Fmoc-Schutzgruppe erfolgte unter Verwendung von 20% Piperidin in DMF 20 min bei RT. Der N-Terminus wurde mit Boc-Amino-Oxy-Essigsäure acyliert. Dazu wurde diese in DMF mit 1 Äquivalent HOAT/DIC aktiviert. Jeweils 1µL dieser Mischung wurde auf jedes cellulosegebundenen Peptid aufgespottet und 30 min bei RT belassen. Die Abspaltung der permanenten Schutzgruppen (tBu für Serin, Threonin, Tyrosin, Glutaminsäure und Asparaginsäure; Boc für Lysin; Trityl für Asparagin, Glutamin, Cystein, Histidin und Pbf für Arginin) erfolgte durch zweistündige Behandlung mit 97% Trifluoressigsäure bei RT. Anschließend wurden die cellulose-gebundenen Peptide mit DCM, MeOH und Diethylether gewaschen und im Vakuum getrocknet. Die Abspaltung der Peptide von der Cellulose erfolgte unter Verwendung von Ammoniakgas 24 Stunden bei RT. Die Spots mit den physikalisch adsorbierten Peptiden wurden ausgestanzt und in 96-well Mikrotiterplatten überführt. Nach Ablösen der Peptide mit je 200 µL 20% Methanol unter Ultraschallbedingungen wurden die Proben filtriert, in 384well Mikrotiterplatten überführt, lyophilisiert und bis zur weiteren Verwendung bei -20°C gelagert.
b) Die in Mikrotiterplatten vorliegenden amino-oxy-essigsäure-haltigen Peptide wurden in DMSO gelöst. Anschließend wurde bei RT jeweils 1 nL dieser Lösung auf die aldehyd-funktionalisierten Glasoberflächen (vgl. Beispiel 3) mittels eines NanoPlotters der Firma Gesim aufgebracht und diese vier Stunden bei RT inkubiert. Dabei betrug der Spot-zu-Spot-Abstand 1.5 mm. Nach dreimaligem Waschen bei RT mit jeweils 100 mL destilliertem Wasser wurden die modifizierten Glasoberflächen mit 30 mL einer 40%igen wässrigen Lösung Hydroxylamin 30 Minuten bei RT inkubiert, um die restlichen Aldehydfunktionen zu deaktivieren. Danach wurden die Glasoberflächen jeweils fünfinal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 21: Immobilisierung aminooxyessigsäurehaltiger Peptide an bromacetylierte Glasoberflächen

a) Die zur Immobilisierung verwendeten Peptide (jeweils 13mere Peptide, die die gesamte Primärstruktur der Proteine MBP, Casein und Histon H1 repräsentieren) wurden nach Standard-SPOT-Synthesemethoden (R.Frank, Tetrahedron, 48, 1992, S.9217-9232; A.Kramer und J. Schneider-Mergener, Methods in Molecular Biology, vol 87: Combinatorial Peptide Library Protocols, S. 25-39, edited by: S. Cabilly; Humana Press Inc., Totowa, NJ) an Cellulose als C-terminale Peptidamide synthetisiert. Dabei wurden entsprechend geschützte Fmoc-Aminosäure-pentafluorphenylester in DMF gelöst und jeweils 1µL aufgespottet. Die Kupplungsreaktion erfolgte doppelt jeweils 25 min bei RT. Die Abspaltung der Fmoc-Schutzgruppe erfolgte unter Verwendung von 20% Piperidin in DMF 20 min bei RT. Der N-Terminus wurde mit Boc-Amino-Oxy-Essigsäure acyliert. Dazu wurde diese in DMF mit 1 Äquivalent HOAT/DIC aktiviert. Jeweils 1µL dieser Mischung wurde auf jedes cellulosegebundenen Peptid aufgespottet und 30 min bei RT belassen. Die Abspaltung der permanenten Schutzgruppen (tBu für Serin, Threonin, Tyrosin, Glutaminsäure und Asparaginsäure; Boc für Lysin und Amino-Oxy-Essigsäure; Trityl für Asparagin, Glutamin, Cystein, Histidin und Pbf für Arginin) erfolgte durch zweistündige Behandlung mit 97% Trifluoressigsäure bei RT. Anschließend wurden die cellulose-gebundenen Peptide mit DCM, MeOH und Diethylether gewaschen und im Vakuum getrocknet. Die Abspaltung der Peptide von der Cellulose erfolgte unter Verwendung von Ammoniakgas 24 Stunden bei RT. Die Spots mit den physikalisch adsorbierten Peptiden wurden ausgestanzt und in 96-well Mikrotiterplatten überführt. Nach Ablösen der Peptide mit je 200 µL 20% Methanol unter Ultraschallbedingungen wurden die Proben filtriert, in 384well Mikrotiterplatten übertragen, lyophilisiert und bis zur weiteren Verwendung bei -20°C gelagert.
b) Die in Mikrotiterplatten vorliegenden amino-oxy-essigsäure-haltigen Peptide wurden in 200 mM Natrium-Phosphatpuffer pH 6.0 gelöst (finale Konzentation der Peptide 0.5 mM) der 25 Vol% Glycerol enthielt. Anschließend wurde bei RT jeweils 0.01 µL dieser Lösung auf die bromacetylierten amino-funktionalisierten Glasoberflächen (vgl. Beispiel 5) mittels eines NanoPlotters der Firma Gesim aufgebracht und diese vier Stunden bei RT inkubiert. Nach dreimaligem Waschen bei RT mit jeweils 100 mL destilliertem Wasser wurden die modifizierten Glasoberflächen mit 30 mL einer 300 mM Lösung von Mercaptoethanol in 200 mM Natrium-Phosphatpuffer pH 7.5 inkubiert, um die restlichen Bromacetylfunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 22: Immobilisierung aminooxyessigsäurehaltiger Peptide an aldehydfunktionalisierte Glasoberflächen

a) Das zur Immobilisierung verwendete Peptid Dpr(Aoa)-Leu-Arg-Arg-Ala-Ser-Leu-Gly-NH₂ wurde nach Standardmethoden der Fmoc-basierenden Chemie an der festen Phase als C-terminales Peptidamid synthetisiert. Dabei wurden entsprechend geschützte Fmoc-Aminosäuren mit einem Äquivalent HBTU und drei Äquivalenten Diisopropylethylamin in DMF aktiviert und an Rink-Amind-MBHA-Harz in DMF gekoppelt. Die Abspaltung der Fmoc-Schutzgruppe erfolgte unter Verwendung von 20% Piperidin in DMF 30 min bei RT. Die Abspaltung der permanenten Schutzgruppen (tBu für Serin, Boc für die Amino-oxy-funktion und Pbf für Arginin) und die gleichzeitige Ablösung vom Polymer erfolgte durch zweistündige Behandlung mit 97% Trifluoressigsäure bei RT. Die resultierende Mischung wurde filtriert und das Filtrat durch Zugabe von tert.Butyl-Methyl-Ether gefällt. Der Niederschlag wurde abgetrennt und mittels HPLC an RP18-Material mit Acetonitril/Wasser Mischungen (0.1% Trifluoressigsäure) gereinigt. Die das gewünschte Produkt enthaltenden Fraktionen wurden lyophilisiert und bis zur weiteren Verwendung bei -20°C gelagert.
b) Das Peptid Dpr(Aoa)-Leu-Arg-Arg-Ala-Ser-Leu-Gly-NH₂ wurde in 200 mM Acetatpuffer pH 4.0 gelöst (finale Konzentation der Peptide 0.5 mM) der 25 Vol% tert. Butanol enthielt. Anschließend wurde bei RT jeweils 5 nL dieser Lösung in einer Anordnung von 20 Reihen und 48 Spalten (gesamt 960 Spots) auf die aldehyd-funktionalisierten Glasoberflächen (Telechem/ArrayIt, CSS-25 Glasträger) mittels eines NanoPlotters der Firma Gesim aufgebracht. Dabei betrug der Spot-zu-Spot-Abstand 1 mm. Anschließend wurden die so behandelten Glasoberflächen vier Stunden bei RT inkubiert. Nach dreimaligem Waschen bei RT mit jeweils 100 mL destilliertem Wasser wurden die modifizierten Glasoberflächen mit 30 mL einer 40%igen wässrigen Lösung Hydroxylamin 30 Minuten bei RT inkubiert, um die restlichen Aldehydfunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 23: Immobilisierung cysteinhaltiger Peptide an 4-bromomethylbenzoylierte Glasoberflächen

Das HPLC-gereinigte Peptid Cys-βAla-Leu-Arg-Arg-Ala-Ser-Leu-Gly-NH₂ wurde in 200 mM Natrium-Phosphatpuffer pH 6.5 gelöst (finale Konzentation 2 mM) der 20 Vol% Glycerol enthielt. Anschließend wurden bei RT jeweils 2 nL dieser Lösung in einer Anordnung von 50 Reihen und 120 Spalten (gesamt 6000 Spots) auf die bromomethylbenzoesäure-funktionalisierten Glasoberflächen (vgl. Beispiel 6) mittels eines NanoPlotters der Firma Gesim aufgebracht. Dabei betrug der Spot-zu-Spot-Abstand 0.4 mm. Anschließend wurden die so behandelten Glasoberflächen 5 Stunden bei RT inkubiert. Nach dreimaligem Waschen bei RT mit jeweils 100 mL destilliertem Wasser wurden die modifizierten Glasoberflächen mit 30 mL einer 300 mM Lösung von Mercaptoethanol in 200 mM Natrium-Phosphatpuffer pH 7.5 inkubiert, um die restlichen Bromomethylphenyl-Funktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 24: Immobilisierung thioamidhaltiger Peptide an 1-brom-2.3-diketo-butanfunktionalisierte Glasoberflächen

a) Das zur Immobilisierung verwendete Peptid Leu-Arg-Arg-Ala-Ser-Leu-Gly-thioamid wurde nach Standardmethoden der Fmoc-basierenden Chemie an der festen Phase als C-terminales Peptidthioxoamid synthetisiert. An Rink-Amid-MBHA-Harz gebundenes Fmoc-Gly-OH wurde 3 Stunden mit Lawessons Reagenz in THF am Rückfluß gekocht. Anschließend wurde das Harz mit THF und DCM gewaschen, 1 Stunde mit DMF geschüttelt und dann mit DMF, DCM und Methanol gewaschen. Nach Entfernen der Fmoc-Schutzgruppe mit 50% Morpholin in DMF (40 min) wurden die entsprechend geschützten Fmoc-Aminosäuren mit einem Äquivalent HBTU und drei Äquivalenten Diisopropylethylamin in DMF aktiviert gekoppelt. Die Abspaltung der Fmoc-Schutzgruppe erfolgte unter Verwendung von 20% Piperidin in DMF 30 min bei RT. Die Abspaltung der permanenten Schutzgruppen (tBu für Serin und Pbf für Arginin) und die gleichzeitige Ablösung vom Polymer erfolgte durch zweistündige Behandlung mit 97% Trifluoressigsäure bei RT. Die resultierende Mischung wurde filtriert und das Filtrat durch Zugabe von tert.Butyl-Methyl-Ether gefällt. Der Niederschlag wurde abgetrennt und mittels HPLC an RP18-Material mit Acetonitril/Wasser Mischungen (0.1% Trifluoressigsäure) gereinigt. Die das gewünschte Produkt enthaltenden Fraktionen wurden lyophilisiert und bis zur weiteren Verwendung bei -20°C gelagert.
b) Das Peptid Leu-Arg-Arg-Ala-Ser-Leu-Gly-thioamid wurde in 200 mM Natrium-Phosphatpuffer pH 5.5 gelöst (finale Konzentation 1 mM) der 50 Vol% Glycerol enthielt. Anschließend wurden bei RT jeweils 1 nL dieser Lösung in einer Anordnung von 70 Reihen und 168 Spalten (gesamt 11760 Spots) auf die 1-Brom-2.3-diketo-butan-funktionalisierten Glasoberflächen (vgl. Beispiel 10) mittels eines NanoPlotters der Firma Gesim aufgebracht. Dabei betrug der Spot-zu-Spot-Abstand 0.3 mm. Anschließend wurden die so behandelten Glasoberflächen 2 min mit einer Mikrowelle behandelt und dann 3 Stunden bei RT inkubiert. Nach dreimaligem Waschen bei RT mit jeweils 100 mL destilliertem Wasser wurden die modifizierten Glasoberflächen mit 30 mL einer 3%igen wässrigen Lösung von Thioacetamid 30 Minuten bei RT inkubiert, um die restlichen α-Bromketonfunktionen zu deaktivieren. Danach wurden die Glasoberflächen jeweils fünfmal je 3 Minuten mit je 50 mL Wasser bei RT und anschließend jeweils zweimal je 3 Minuten mit je 50 mL Methanol bei RT gewaschen. Die so behandelten Glasoberflächen wurden getrocknet und bis zur weiteren Verwendung bei 4°C gelagert.

### Beispiel 25: Analyse Kinase vermittelter Peptidmodifikationen an modifizierten Glasoberflächen (vgl. Fig. 4)

Eine mit dem Peptid Cys-βAla-Leu-Arg-Arg-Ala-Ser-Leu-Gly-NH₂ (sowohl als Rohpeptid als auch als mittels präp. HPLC gereinigtes Peptid) und dem Kontrollpeptid Cys-βAla-Leu-Arg-Arg-Ala-Ala-Leu-Gly-NH₂ modifizierte Glasoberfläche (maleinimido-funktionalisierte Glasoberfläche, vgl. Beispiel 1) wurde mit 10ml 100µM ATP in Kinasepuffer (50mM Tris-HCl, 150mM NaCl, 30mM MgCl₂, 4mM DTT, 2mM EGTA, pH 7,5) 10 Minuten bei RT inkubiert. Anschließend wurde auf die Peptid-modifizierten Glasoberflächen 1 µL einer Mischung aus Proteinkinase A (Sigma, P26452, U/mL), 100µM/mL ATP und 100µCi/mL γ-³²P-ATP (Amersham, 9,25mBq/250µCi/25µL, Aktivität >5000Ci/mmol) in Kinasepuffer (50mM Tris-HCl, 150mM NaCl, 30mM MgCl₂, 4mM DTT, 2mM EGTA, pH 7,5) aufgespottet und 30 min bei RT in einer nahezu wassergesättigten Atmosphäre inkubiert. Um den durch unspezifische Bindung von ATP- bzw. Kinase-Molekülen an die Glasoberflächen verursachten Hintergrund zu verringern, wurden die modifizierten Glasoberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1% SDS enthielt
- 3mal 3 Minuten bei RT mit destilliertem Wasser
- 3mal 3 Minuten bei RT mit Methanol

Nach dem Trocknen der Glasoberfläche wurde die in den Glasoberflächen-gebundenen Peptiden eingebaute Menge an radioaktivem Phosphat mittels eines PhosphorImager-Systems (FLA-3000, FUJIFILM) bestimmt (vgl. Fig. 4).

### Beispiel 26: Analyse Kinase vermittelter Peptidmodifikationen an modifizierten Glasoberflächen (vgl. Fig. 5)

Eine mit dem Peptid Cys-βAla-Leu-Arg-Arg-Ala-Ser-Leu-Gly-NH₂ und dem Kontrollpeptid Cys-βAla-Leu-Arg-Arg-Ala-Ala-Leu-Gly-NH₂ modifizierte Glasoberfläche (maleinimidofunktionalisierte Glasoberfläche; vgl. Beispiel 1) wurde mit 10ml 100µM ATP in Kinasepuffer (50mM Tris-HCl, 150mM NaCl, 30mM MgCl₂, 4mM DTT, 2mM EGTA, pH 7,5) 10 Minuten bei RT inkubiert. Die Glasoberfläche wurde getrocknet und anschließend wurde auf die Peptid-modifizierte Glasoberfläche ein Deckgläschen gelegt. Dann wurden 20 µL einer Mischung aus Proteinkinase A (Sigma, P26452, U/mL), 100µM/mL ATP und 100µCi/mL γ-³²P-ATP (Amersham, 9,25mBq/250µCi/25µL, Aktivität >5000Ci/mmol) in Kinasepuffer (50mM Tris-HCl, 150mM NaCl, 30mM MgCl₂, 4mM DTT, 2mM EGTA, pH 7,5) durch Kapillarkräfte in den Spalt, der durch das auf der modifizierte Glasoberfläche aufliegende Deckgläschen gebildet wird, aufgegeben. Anschließend wurde 30 min bei RT in einer nahezu wassergesättigten Atmosphäre inkubiert. Um den durch unspezifische Bindung von ATP- bzw. Kinase-Molekülen an die Glasoberflächen verursachten Hintergrund zu verringern, wurden die modifizierten Glasoberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1 % SDS enthielt
- 3mal 3 Minuten bei RT mit destilliertem Wasser
- 3mal 3 Minuten bei RT mit Methanol

Nach dem Trocknen der Glasoberfläche wurde die in den Glasoberflächen-gebundenen Peptiden eingebaute Menge an radioaktivem Phosphat mittels eines PhosphorImager-Systems (FLA-3000, FUJIFILM) bestimmt (vgl. Fig. 5).

### Beispiel 27: Analyse Kinase vermittelter Peptidmodifikationen an modifizierten Glasoberflächen (vgl. Fig. 7)

Eine mit dem Peptid Dpr(Aoa)-Leu-Arg-Arg-Ala-Ser-Leu-Gly-NH₂ modifizierte Glasoberfläche (vgl. Beispiel 22) wurde mit 10ml 100µM ATP in Kinasepuffer (50mM Tris-HCl, 150mM NaCl, 30mM MgCl₂, 4mM DTT, 2mM EGTA, pH 7,5) 10 Minuten bei RT inkubiert. Die Glasoberfläche wurde getrocknet und anschließend wurde auf die Peptid-modifizierte Glasoberfläche eine zweite, nichtmodifizierte Glasoberfläche gleicher Abmessungen gelegt. Dann wurden 50 µL einer Mischung aus Proteinkinase A (Sigma, P26452, U/mL), 100µM/mL ATP und 100µCi/mL γ-³²P-ATP (Amersham, 9,25mBq/250µCi/25µL, Aktivität >5000Ci/mmol) in Kinasepuffer (50mM Tris-HCl, 150mM NaCl, 30mM MgCl₂, 4mM DTT, 2mM EGTA, pH 7,5) durch Kapillarkräfte in den Spalt, der durch die auf der modifizierten Glasoberfläche aufliegende zweite Glasoberfläche gebildet wird, aufgegeben. Anschließend wurde 30 min bei RT in einer nahezu wassergesättigten Atmosphäre inkubiert. Um den durch unspezifische Bindung von ATP- bzw. Kinase-Molekülen an die Glasoberflächen verursachten Hintergrund zu verringern, wurde die modifizierte Glasoberfläche wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1% SDS enthielt
- 3mal 3 Minuten bei RT mit destilliertem Wasser
- 3mal 3 Minuten bei RT mit Methanol

Nach dem Trocknen der Glasoberfläche wurde die in den glasoberflächen-gebundenen Peptiden eingebaute Menge an radioaktivem Phosphat mittels eines PhosphorImager-Systems (FLA-3000, FUJIFILM) bestimmt (vgl. Fig. 7).

### Beispiel 28: Analyse Kinase vermittelter Peptidmodifikationen an modifizierten Glasoberflächen (vgl. Fig. 6)

Eine mit dem Peptid Leu-Arg-Arg-Ala-Ser-Leu-Gly-thioamid modifizierte Glasoberfläche (vgl. Beispiel 24) wurde mit 10ml 100µM ATP in Kinasepuffer (50mM Tris-HCl, 150mM NaCl, 30mM MgCl₂, 4mM DTT, 2mM EGTA, pH 7,5) 10 Minuten bei RT inkubiert. Die Glasoberfläche wurde getrocknet und anschließend wurde auf die Peptid-modifizierte Glasoberfläche eine zweite, nichtmodifizierte Glasoberfläche gleicher Abmessungen gelegt. Dann wurden 50 µL einer Mischung aus Proteinkinase A (Sigma, P26452, U/mL), 100µM/mL ATP und 100µCi/mL γ-³²P-ATP (Amersham, 9,25mBq/250µCi/25µL, Aktivität >5000Ci/mmol) in Kinasepuffer (50mM Tris-HCl, 150mM NaCl, 30mM MgCl₂, 4mM DTT, 2mM EGTA, pH 7,5) durch Kapillarkräfte in den Spalt, der durch die auf der modifizierten Glasoberfläche aufliegende zweite Glasoberfläche gebildet wird, aufgegeben. Anschließend wurde 30 min bei RT in einer nahezu wassergesättigten Atmosphäre inkubiert. Um den durch unspezifische Bindung von ATP- bzw. Kinase-Molekülen an die Glasoberflächen verursachten Hintergrund zu verringern, wurde die modifizierte Glasoberfläche wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1% SDS enthielt
- 3mal 3 Minuten bei RT mit destilliertem Wasser
- 3mal 3 Minuten bei RT mit Methanol

Nach dem Trocknen der Glasoberfläche wurde die in den glasoberflächen-gebundenen Peptiden eingebaute Menge an radioaktivem Phosphat mittels eines Phosphorlmager-Systems (FLA-3000, FUJIFILM) bestimmt (vgl. Fig. 6).

### Beispiel 29: Analyse Kinase vermittelter Peptidmodifikationen an modifizierten Glasoberflächen (vgl. Fig. 1)

Eine mit den Peptiden Cys-βAla-Leu-Arg-Arg-Ala-Ser-Leu-Gly-NH₂, Cys-βAla-Gln-Lys-Arg-Pro-Ser-Gln-Arg-Ser-Lys-NH₂, und Cys-βAla-Arg-Arg-Lys-Asp-Leu-His-Ap-Asp-Glu-Glu-Asp-Glu-Ala-Met-Ser-Ile-Thr-Ala-NH₂ bzw. den entsprechenden Kontrollpeptiden Cys-βAla-Leu-Arg-Arg-Ala-Ala-Leu-Gly-NH₂, Cys-βAla-Gln-Lys-Arg-Pro-Ala-Gln-Arg-Ala-Lys-NH₂, und Cys-βAla-Arg-Arg-Lys-Asp-Leu-His-Asp-Asp-Glu-Glu-Asp-Glu-Ala-Met-Ala-Ile-Ala-Ala-NH₂ modifizierte Glasoberfläche (maleinimido-funktionalisierte Glasoberfläche, vgl. Beispiel 2) wurde mit 10ml 100µM ATP in Kinasepuffer (50mM Tris-HCl, 150mM NaCl, 30mM MgCl₂, 4mM DTT, 2mM EGTA, pH 7,5) 10 Minuten bei RT inkubiert. Anschließend wurde auf die Peptid-modifizierten Glasoberflächen 1 µL einer Mischung aus Proteinkinase (Proteinkinase A, Sigma, P2645, 1.67 µg/mL für Cys-βAla-Leu-Arg-Arg-Ala-Ser-Leu-Gly-NH₂ und Cys-βAla-Leu-Arg-Arg-Ala-Ala-Leu-Gly-NH₂; Proteinkinase C, Sigma, P7956, 1.3 µg/mL für Cys-βAla-Gln-Lys-Arg-Pro-Ser-Gln-Arg-Ser-Lys-NH₂ und Cys-βAla-Gln-Lys-Arg-Pro-Ala-Gln-Arg-Ala-Lys-NH₂; Caseinkinase 1, New England Biolabs, P6030S, 2.5 µg/mL für Cys-βAla-Arg-Arg-Lys-Asp-Leu-His-Ap-Asp-Glu-Glu-Asp-Glu-Ala-Met-Ser-Ile-Thr-Ala-NH₂ und Cys-βAla-Arg-Arg-Lys-Asp-Leu-His-Asp-Asp-Glu-Glu-Asp-Glu-Ala-Met-Ala-Ile-Ala-Ala-NH₂) 100µM/mL ATP und 100µCi/mL γ-³²P-ATP (Amersham, 9,25mBq/250µCi/25µL, Aktivität >5000Ci/mmol) in Kinasepuffer (50mM Tris-HCl, 150mM NaCl, 30mM MgCl₂, 4mM DTT, 2mM EGTA, pH 7,5) aufgespottet und 30 min bei RT in einer nahezu wassergesättigten Atmosphäre inkubiert. Um den durch unspezifische Bindung von ATP- bzw. Kinase-Molekülen an die Glasoberflächen verursachten Hintergrund zu verringern, wurden die modifizierten Glasoberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1 % SDS enthielt
- 3mal 3 Minuten bei RT mit destilliertem Wasser
- 3mal 3 Minuten bei RT mit Methanol

Nach dem Trocknen der Glasoberfläche wurde die in den Glasoberflächen-gebundenen Peptiden eingebaute Menge an radioaktivem Phosphat mittels eines PhosphorImager-Systems (FLA-3000, FUJIFILM) bestimmt (vgl. Fig. 1).

### Beispiel 30: Analyse Kinase vermittelter Peptidmodifikationen an verschiedenen modifizierten Oberflächen (vgl. Fig. 2)

Mit den Peptiden Cys-βAla-Leu-Arg-Arg-Ala-Ser-Leu-Gly-NH₂ und Cys-βAla-Leu-Arg-Arg-Ala-Ala-Leu-Gly-NH₂ modifizierte Oberflächen (maleinimido-funktionalisierte Glasoberfläche, vgl. Beispiel 1; maleinimidobutyryl-β-alaninin-funktionalisierte Cellulose sowie maleinimidobutyryl-β-alaninin-funktionalisierte Polypropylen-Membranen) wurden mit 10ml 100µM ATP in Kinasepuffer (50mM Tris-HCl, 150mM NaCl, 30mM MgCl₂, 4mM DTT, 2mM EGTA, pH 7,5) 10 Minuten bei RT inkubiert. Die Oberflächen wurden anschließend mit einer Mischung aus Proteinkinase A (Sigma, P26452, U/mL), 100µM/mL ATP und 100µCi/mL γ-³²P-ATP (Amersham, 9,25mBq/250µCi/25µL, Aktivität >5000Ci/mmol) in Kinasepuffer (50mM Tris-HCl, 150mM NaCl, 30mM MgCl₂, 4mM DTT, 2mM EGTA, pH 7,5) überschichtet und 30 min bei RT in einer nahezu wassergesättigten Atmosphäre inkubiert. Um den durch unspezifische Bindung von ATP- bzw. Kinase-Molekülen an die Oberflächen verursachten Hintergrund zu verringern, wurden die modifizierten Oberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1% SDS enthielt
- 3mal 3 Minuten bei RT mit destilliertem Wasser
- 3mal 3 Minuten bei RT mit Methanol

Nach dem Trocknen der Oberflächen wurde die in den oberflächen-gebundenen Peptiden eingebaute Menge an radioaktivem Phosphat mittels eines PhosphorImager-Systems (FLA-3000, FUJIFILM) bestimmt (vgl. Fig. 2)

### Beispiel 31: Analyse der zeitabhängigkeit Kinase vermittelter Peptidmodifikationen an verschiedenen modifizierten Oberflächen (vgl. Fig. 3)

Mit dem Peptide Cys-βAla-Leu-Arg-Arg-Ala-Ser-Leu-Gly-NH₂ modifizierte Oberflächen (maleinimido-funktionalisierte Glasoberfläche, vgl. Beispiel 1 und maleinimidobutyryl-β-alanininfunktionalisierte Cellulose) wurden mit 10mi 100µM ATP in Kinasepuffer (50mM Tris-HCl, 150mM NaCl, 30mM MgCl₂, 4mM DTT, 2mM EGTA, pH 7,5) 10 Minuten bei RT inkubiert. Die Oberflächen wurden anschließend mit jeweils 1 µL einer Mischung aus Proteinkinase A (Sigma, P26452, U/mL), 100µM/mL ATP und 100µCi/mL γ-³²P-ATP (Amersham, 9,25mBq/250µCi/25µL, Aktivität >5000Ci/mmol) in Kinasepuffer (50mM Tris-HCl, 150mM NaCl, 30mM MgCl₂, 4mM DTT, 2mM EGTA, pH 7,5) versetzt und die angegebenen Zeiten bei RT in einer nahezu wassergesättigten Atmosphäre inkubiert. Um den durch unspezifische Bindung von ATP- bzw. Kinase-Molekülen an die Oberflächen verursachten Hintergrund zu verringern, wurden die modifizierten Oberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1% SDS enthielt
- 3mal 3 Minuten bei RT mit destilliertem Wasser
- 3mal 3 Minuten bei RT mit Methanol

Nach dem Trocknen der Oberflächen wurde die in den oberflächen-gebundenen Peptiden eingebaute Menge an radioaktivem Phosphat mittels eines PhosphorImager-Systems (FLA-3000, FUJIFILM) bestimmt (vgl. Fig. 3)

### Beispiel 32: Analyse Kinase vermittelter Peptidmodifikationen an verschiedenen modifizierten Oberflächen (vgl. Fig. 8)

Genau 43 serin- und/oder threoninhaltige Peptide (potentielle Substratpeptide für Kinasen) und die entsprechenden Kontrollpeptide, jeweils am N-Terminus mit dem Dipeptid Cysteinyl-β-alanin modifiziert, wurden durch eine Michael-Addition an eine maleinimidofunktionalisierte Glas-Oberfläche gekoppelt (vgl. Beispiel 18). Die Anordnung der Peptide ist in Fig. 8A dargestellt. Die Nummerierung der Spots ist in Fig. 8C ersichtlich, die Sequenzen der genutzten Peptide gehen aus Beispiel 18 hervor. Die modifizierte Glas-Oberfläche wurde nach dem Aufbringen der Peptide zunächst mit 10mL 100µM ATP-Lösung in 50 mM Natriumphosphatpuffer pH 7.5 für 10 Minuten vorinkubiert. Anschließend wurde modifizierte Glas-Oberfläche mit einem Deckgläschen abgedeckt und in den entstandenen Zwischenraum Proteinkinase C (10U/mL) zusammen mit ATP/γ³²P-ATP-Mischung (100 µM/mL; 100µCi/mL) mittels Kapillarkraft eingebracht. Danach wurde 30 min bei 25°C inkubiert. Um den durch unspezifische Bindung von ATP- bzw. Kinase-Molekülen an die Oberflächen verursachten Hintergrund zu verringern, wurden die modifizierten Oberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1 % SDS enthielt
- 3mal 3 Minuten bei RT mit destilliertem Wasser
- 3mal 3 Minuten bei RT mit Methanol

Nach dem Trocknen der Oberflächen wurde die in den oberflächen-gebundenen Peptiden eingebaute Menge an radioaktivem Phosphat mittels eines PhosphorImager-Systems (FLA-3000, FUJIFILM) bestimmt. Das resultierende Bild ist in Fig. 8B dargestellt. Die Spots mit hoher Signalintensität in allen drei Subarrays wurden den entsprechenden von der Proteinkinase C phosphorylierten Peptiden zugeordnet. Deren Primärstrukturen sind in Fig. 8D gezeigt. Es wird deutlich, daß als Proteinkinase C Substrate bekannte (Substratpeptid Nr. 3, 23, 27, 41, 43) und weitere, nicht als Substrate für Proteinkinase C beschriebene Peptide von dieser Kinase auf der modifizierten Glasoberfläche erkannt und phosphoryliert werden. Damit wird deutlich, dass eine solche Anordnung geeignet ist, die Substratspezifität einer Kinase, wie zum Beispiel Proteinkinase C, zu charakterisieren.

### Beispiel 33: Analyse Kinase vermittelter Peptidmodifikationen an verschiedenen modifizierten Oberflächen (vgl. Fig. 9)

Genau 43 serin- und/oder threoninhaltige Peptide (potentielle Substratpeptide für Kinasen) und die entsprechenden Kontrollpeptide, jeweils am N-Terminus mit dem Dipeptid Cysteinyl-β-alanin modifiziert, wurden durch eine Michael-Addition an eine maleinimidofunktionalisierte Glas-Oberfläche gekoppelt (vgl. Beispiel 18). Die Anordnung der Peptide ist in Fig. 8A dargestellt. Die Nummerierung der Spots ist in Fig. 8C ersichtlich, die Sequenzen der genutzten Peptide gehen aus Beispiel 18 hervor. Die modifizierte Glas-Oberfläche wurde nach dem Aufbringen der Peptide zunächst mit 10mL 100µM ATP-Lösung in 50 mM Natriumphosphatpuffer pH 7.5 für 10 Minuten vorinkubiert. Anschließend wurde modifizierte Glas-Oberfläche mit einem Deckgläschen abgedeckt und in den entstandenen Zwischenraum Proteinkinase A (10U/mL) zusammen mit ATP/γ³²P-ATP-Mischung (100 µM/mL; 100µCi/mL) mittels Kapillarkraft eingebracht. Danach wurde 30 min bei 25°C inkubiert. Um den durch unspezifische Bindung von ATP- bzw. Kinase-Molekülen an die Oberflächen verursachten Hintergrund zu verringern, wurden die modifizierten Oberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1% SDS enthielt
- 3mal 3 Minuten bei RT mit destilliertem Wasser
- 3mal 3 Minuten bei RT mit Methanol

Nach dem Trocknen der Oberflächen wurde die in den oberflächen-gebundenen Peptiden eingebaute Menge an radioaktivem Phosphat mittels eines PhosphorImager-Systems (FLA-3000, FUJIFILM) bestimmt. Das resultierende Bild ist in Fig. 9B dargestellt. Die Spots mit hoher Signalintensität in allen drei Subarrays wurden den entsprechenden von der Proteinkinase A phosphorylierten Peptiden zugeordnet. Deren Primärstrukturen sind in Fig. 9D gezeigt.Es wird deutlich, daß mit einer Ausnahme alle Peptide auf der modifizierten Glasoberfläche von Proteinkinase A erkannt und phosphoryliert werden, die in Position -2 und -3 (N-terminal) zum Serin zwei Argininreste tragen. Dieses Sequenzmotiv RRxS ist als bevorzugtes Substratmotiv für Proteinkinase A beschrieben (A. Kreegipuu, N. Blom, S. Brunak, J. Jarv, 1998, Statistical analysis of protein kinase specificity determinants, *FEBS Lett*., **430**, 45-50). Das Peptid 83 wird wahrscheinlich wegen der zu sehr N-terminalen Lokalisation des Substratmotives nicht phosphoryliert. Somit wird deutlich, dass eine solche Anordnung geeignet ist, die Substratspezifität einer Kinase, wie zum Beispiel Proteinkinase A, zu charakterisieren.

### Beispiel 34: Analyse Kinase vermittelter Peptidmodifikationen an verschiedenen modifizierten Oberflächen (vgl. Fig. 10)

Genau 79 Peptide, jeweils am N-Terminus mit dem Dipeptid Amino-oxyessigsäure-β-alanin modifiziert, wurden an eine aldehydfunktionalisierte Glas-Oberfläche gekoppelt (vgl. Beispiel 20). Die Primässtruktur des MBP ist in Fig. 10C dargestellt. Für die durch Fettdruck hervorgehobenen Reste wurde eine Phosphorylierung durch Proteinkinase A im Stand der Technik beschrieben (A. Kishimoto, K. Nishiyama, H. Nakanishi, Y. Uratsuji, H. Nomura, Y. Takeyama, Y. Nishizuka, 1985, Studies on the phosphorylation of myelin basic protein by protein kinase C and adenosine 3':5'-monophosphate-dependent protein kinase, *J*. *Biol*. *Chem*., **260**, 12492-12499). Die 13 meren Peptide in dem Scan weisen eine Sequenzverschiebung von 2 Aminosäuren auf, Die Anordnung der Peptide ist in Fig. 10B dargestellt. Somit repräsentiert das Peptid Nr. 1 die Aminosäuresequenz 1-13 der Primärstruktur von MBP, das Peptid Nr. 2 die Aminosäuresequenz 3-15 der Primärstruktur von MBP usw. Es wurden drei identische Subarrays auf die Glasoberfläche aufgebracht. In Fig. 10A ist eines dieser Subarrays dargestellt. Die modifizierte Glas-Oberfläche wurde nach dem Aufbringen der Peptide zunächst mit 10mL 100µM ATP-Lösung in 50 mM Natriumphosphatpuffer pH 7.5 für 10 Minuten vorinkubiert. Anschließend wurde die modifizierte Glas-Oberfläche mit einem Deckgläschen abgedeckt und in den entstandenen Zwischenraum Proteinkinase A (10U/mL) zusammen mit ATP/γ³²P-ATP-Mischung (100 µM/mL; 100µCi/mL) mittels Kapillarkraft eingebracht. Danach wurde 30 min bei 25°C inkubiert. Um den durch unspezifische Bindung von ATP- bzw. Kinase-Molekülen an die Oberflächen verursachten Hintergrund zu verringern, wurden die modifizierten Oberflächen wie folgt gewaschen:
- 3mal 3 Minuten bei RT mit Waschpuffer (1% SDS und 1% Tween20 in 50mM TRIS-Puffer, pH 7.5, 200 mM NaCl)
- 2mal 3 Minuten bei RT mit 1M NaCl-Lösung
- 2mal 3 Minuten bei RT mit destilliertem Wasser
- 2mal 3 Minuten bei RT mit 80%iger Ameisensäure in Ethanol
- 2mal 3 Minuten bei RT mit destilliertem Wasser
- 2mal 5 Minuten bei 50°C mit einer Lösung, die 6M Harnstoff, 2M Thioharnstoff und 1 % SDS enthielt
- 3mal 3 Minuten bei RT mit destilliertem Wasser
- 3mal 3 Minuten bei RT mit Methanol

Nach dem Trocknen der Oberflächen wurde die in den oberflächen-gebundenen Peptiden eingebaute Menge an radioaktivem Phosphat mittels eines PhosphorImager-Systems (FLA-3000, FUJIFILM) bestimmt. Das resultierende Bild ist in Fig. 10A dargestellt. Die Spots mit hoher Signalintensität in allen drei Subarrays wurden den entsprechenden von der Proteinkinase A phosphorylierten Peptiden zugeordnet. Deren Primärstrukturen sind in Fig. 10D gezeigt. Es wird deutlich, daß die meisten Peptide auf der modifizierten Glasoberfläche von Proteinkinase A erkannt und phosphoryliert werden, die auch in dem in Lösung durchgeführten Experiment gefunden werden konnten (A. Kishimoto, K. Nishiyama, H. Nakanishi, Y. Uratsuji, H. Nomura, Y. Takeyama, Y. Nishizuka, 1985, Studies on the phosphorylation of myelin basic protein by protein kinase C and adenosine 3':5'-monophosphate-dependent protein kinase, *J*. *Biol. Chem*., **260**, 12492-12499).

Die in der vorstehenden Beschreibung, den Beispielen, den Ansprüchen, den Zeichnungen und dem Sequenzprotokoll offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verfahren zur Bestimmung der Substratspezifität einer enzymatischen Aktivität umfassend die folgenden Schritte:
- Bereitstellen einer Anordnung umfassend eine Vielzahl von Aminosäuresequenzen auf einer planaren Oberfläche eines Trägermaterials, wobei die Aminosäuresequenzen gerichtet immobilisiert sind,
- Kontaktieren und/oder Inkubieren einer enzymatischen Aktivität mit der Anordnung, und
- Nachweis einer Reaktion zwischen einer oder mehreren der auf der Anordnung immobilisierten Aminosäuresequenzen und der enzymatischen Aktivität,
**dadurch gekennzeichnet, dass** bei der Umsetzung der enzymatischen Aktivität mit der Anordnung eine Änderung des Molekulargewichts mindestens einer der Aminosäuresequenzen erfolgt und die Oberfläche eine nicht-poröse Oberfläche ist.

2. Verfahren nach Anspruch 1, **dadurch gekennzeichnet, dass** der Nachweis der Reaktion an der oder unter Verwendung der auf der Oberfläche des Trägermaterials immobilisierten Aminosäuresequenz erfolgt.

3. Verfahren nach Anspruch 1 oder 2, **dadurch gekennzeichnet, dass** die Änderung des Molekulargewichts durch Ausbildung oder Spaltung einer kovalenten Bindung an einer der Aminosäuresequenzen erfolgt, bevorzugterweise an derjenigen Aminosäuresequenz, die mit der enzymatischen Aktivität reagiert.

4. Verfahren nach einem der Ansprüche 1 bis 3, **dadurch gekennzeichnet, dass** der Nachweis der Reaktion durch Detektion der Änderung des Molekulargewichts erfolgt.

5. Verfahren nach einem der Ansprüche 1 bis 4, **dadurch gekennzeichnet, dass** der Nachweis durch ein. Nachweisverfahren erfolgt, das ausgewählt ist aus der Gruppe, die Autoradiographie, Plasmonresonanzspektroskopie und Fluoreszenzspektroskopie umfasst.

6. Verfahren nach einem der Ansprüche 1 bis 5, **dadurch gekennzeichnet, dass** mindestens eine der Aminosäuresequenzen ein Substrat für eine enzymatische Aktivität ist.

7. Verfahren nach einem der Ansprüche 1 bis 6, **dadurch gekennzeichnet, dass** die Anordnung von Aminosäuresequenzen für mindestens zwei verschiedene enzymatische Aktivitäten jeweils mindestens ein Substrat aufweist.

8. Verfahren nach einem der Ansprüche 1 bis 7, **dadurch gekennzeichnet, dass** die enzymatische Aktivität ausgewählt ist aus der Gruppe, die Oxidoreduktasen, Transferasen, Hydrolasen, Lyasen und Ligasen umfasst, und insbesondere ausgewählt ist aus der Gruppe, die Kinasen, Sulfotransferasen, Glycosyltransferasen, Acetyltransferasen, Farnesyltransferasen, Palmityltransferasen, Phosphatasen, Sulfatasen, Esterasen, Lipasen, Acetylasen und Proteasen umfasst.

9. Verfahren nach einem der Ansprüche 1 bis 8, **dadurch gekennzeichnet, dass** der Nachweis einer Reaktion zwischen den auf der Anordnung immobilisierten Aminosäuresequenzen und der enzymatischen Aktivität mehrfach, bevorzugterweise in zeitlichen Abständen, wiederholt wird.

10. Verfahren nach einem der Ansprüche 1 bis 9, **dadurch gekennzeichnet, dass** die enzymatische Aktivität in einer Probe bestimmt wird und die Probe bevorzugterweise ausgewählt ist aus der Gruppe, die Urin, Liquor, Sputum, Stuhl, Lymphflüssigkeit, andere Körperflüssigkeiten Zellysate, Gewebelysate, Organlysate, Extrakte, Rohextrakte, gereinigte Präparate und ungereinigte Präparate umfasst.

11. Verfahren nach einem der Ansprüche 1 bis 10, **dadurch gekennzeichnet, dass** das Trägermaterial Glas ist.

12. Verfahren nach einem der Ansprüche 1 bis 11, **dadurch gekennzeichnet, dass** die Aminosäuresequenz über eine Schwefel urnfassende Gruppe an der Oberfläche immobilisiert ist

13. Anordnung von einer Vielzahl von Aminosäuresequenzen auf einer Oberfläche, wobei die Aminosäuresequenzen gerichtet auf der planaren Oberfläche eines Trägermaterials immobilisiert sind, **dadurch gekennzeichnet, dass** mindestens eine der Aminosäuresequenzen ein Substrat für eine enzymatische Aktivität darstellt, wobei am Substrat durch die enzymatische Aktivität eine Änderung des Molekulargewichts erfolgt und die Oberfläche eine nicht-poröse Oberfläche ist.

14. Anordnung nach Anspruch 13, **dadurch gekennzeichnet, dass** die Änderung des Molekulargewichts durch Ausbildung oder Spaltung einer kovalenten Bindung am Substrat erfolgt ist.

15. Anordnung nach Anspruch 13 oder 14, **dadurch gekennzeichnet, dass** die Anordnung von Aminosäuresequenzen für mindestens zwei verschiedene enzymatische Aktivitäten jeweils mindestens ein Substrat aufweist.

16. Anordnung nach Anspruch 13 bis 15, **dadurch gekennzeichnet, dass** das Trägermaterial ausgewählt ist aus der Gruppe, die Silikate, Keramik, Glas, Metalle und organische Trägermaterialien umfasst.

17. Anordnung nach einem der Ansprüche 13 bis 16, **dadurch gekennzeichnet, dass** die Aminosäuresequenzen ausgewählt sind aus der Gruppe, die Peptide, Oligopeptide, Polypeptide und Proteine sowie deren jeweilige Derivate umfasst.

18. Anordnung nach einem der Ansprüche 13 bis 17, **dadurch gekennzeichnet, dass** eine jede Aminosäuresequenz oder Gruppe von Aminosäuresequenzen relativ zu einer anderen Aminosäuresequenz oder Gruppen von Aminosäuresequenzen definiert angeordnet ist.

19. Träger umfassend eine Anordnung nach einem der vorhergehenden Ansprüche.

20. Träger nach Anspruch 19, **dadurch gekennzeichnet, dass** der Träger ein Basisträgermaterial umfasst.

21. Träger nach Anspruch 19 oder 20, **dadurch gekennzeichnet, dass** die Anordnung einer Vielzahl von Aminosäuresequenzen auf einer oder mehreren der Oberflächen des Trägers angeordnet ist.

22. Trägeranordnung umfassend mindestens zwei Träger nach einem der Ansprüche 19 bis 21, wobei jeweils zwei Träger durch einen Spalt getrennt sind.

23. Trägeranordnung nach Anspruch 22, **dadurch gekennzeichnet, dass** zumindest eine Anordnung auf einem ersten Träger mindestens einer Anordnung auf einem zweiten Träger zugewandt ist.

24. Trägeranordnung nach Anspruch 22 oder 23, **dadurch gekennzeichnet, dass** der Spalt eine Breite etwa von 0,01 mm bis 10 mm, bevorzugterweise etwa von 0,1 mm bis 2 mm und bevorzugtererweise etwa von 0,5 mm bis 1 mm aufweist.

25. Verwendung einer Anordnung nach einem der Ansprüche 13 bis 18 und/oder eines Trägers nach einem der Ansprüche 19 bis 21 und/oder einer Trägeranordnung nach einem der Ansprüche 22 bis 24 in einem Verfahren nach einem der Ansprüche 1 bis 12.

## Claims

1. A method for determining the substrate specifity of an enzymatic activity, comprising the following steps:
- providing an arrangement comprising a plurality of amino acid sequences on a planar surface of a support material, the amino acid sequences being directionally immobilised,
- contacting and/or incubating an enzymatic activity with the arrangement, and
- detecting a reaction between one or more of the amino acid sequences immobilised on the arrangement and the enzymatic activity,
**characterised in that** during the reaction of the enzymatic activity with the arrangement a change in the molecular weight of at least one of the amino acid sequences takes place, and that the surface is a non-porous surface.

2. A method according to Claim 1, **characterised in that** the detection of the reaction takes place on or using the amino acid sequence immobilised on the surface of the support material.

3. A method according to Claim 1 or 2, **characterised in that** the change in the molecular weight takes place by forming or cleaving a covalent bond on one of the amino acid sequences, preferably on the amino acid sequence which reacts with the enzymatic activity.

4. A method according to one of Claims 1 to 3, **characterised in that** the detection of the reaction takes place by detecting the change in the molecular weight.

5. A method according to one of Claims 1 to 4, **characterised in that** the detection takes place by a detection method selected from the group comprising autoradiography, plasmon resonance spectroscopy and fluorescence spectroscopy.

6. A method according to one of Claims 1 to 5, **characterised in that** at least one of the amino acid sequences is a substrate for an enzymatic activity.

7. A method according to one of Claims 1 to 6, **characterised in that** the arrangement of amino acid sequences for at least two different enzymatic activities in each case has at least one substrate.

8. A method according to one of Claims 1 to 7, **characterised in that** the enzymatic activity is selected from the group comprising oxidoreductases, transferases, hydrolases, lyases and ligases, and in particular is selected from the group comprising kinases, sulphotransferases, glycosyltransferases, acetyltransferases, famesyltransferases, palmityltransferases, phosphatases, sulphatases, esterases, lipases, acetylases and proteases.

9. A method according to one of Claims 1 to 8, **characterised in that** the detection of a reaction between the amino acid sequences immobilised on the arrangement and the enzymatic activity is repeated several times, preferably at intervals of time.

10. A method according to one of Claims 1 to 9, **characterised in that** the enzymatic activity is determined in a sample and the sample is preferably selected from the group comprising urine, liquor, sputum, stools, lymphatic fluid, other bodily fluids, cell Iysates, tissue Iysates, organ Iysates, extracts, raw extracts, purified preparations and non-purified preparations.

11. A method according to one of Claims 1 to 10, **characterised in that** the support material is glass.

12. A process according to one of Claims 1 to 11, **characterised in that** the amino acid sequence is immobilised on the surface via a group comprising sulphur.

13. The arrangement of a plurality of amino acid sequences on a surface, the amino acid sequences being directionally immobilised on the planar surface of a support material, **characterised in that** at least one of the amino acid sequences represents a substrate for an enzymatic activity, with a change in the molecular weight taking place on the substrate due to the enzymatic activity and the surface being a non-porous surface.

14. An arrangement according to Claim 13, **characterised in that** the change in the molecular weight is obtained by forming or splitting a covalent bond on the substrate.

15. An arrangement according to Claims 13 to 14, **characterised in that** the arrangement of amino acid sequences for at least two different enzymatic activities in each case has at least one substrate.

16. An arrangement according to Claims 13 to 15, **characterised in that** the support material is selected from the group comprising silicates, ceramics, glass, metals and organic support materials.

17. An arrangement according to one of Claims 13 to 16, **characterised in that** the amino acid sequences are selected from the group comprising peptides, oligopeptides, polypeptides and proteins and their respective derivatives.

18. An arrangement according to one of Claims 13 to 17, **characterised in that** each amino acid sequence or group of amino acid sequences is arranged definedly relative to another amino acid sequence or groups of amino acid sequences.

19. A support comprising an arrangement according to one of the preceding claims.

20. A support according to Claim 19, **characterised in that** the support comprises a base support material.

21. A support according to Claim 19 or 20, **characterised in that** the arrangement of a plurality of amino acid sequences is arranged on one or more of the surfaces of the support.

22. A support arrangement comprising at least two supports according to one of Claims 19 to 21, wherein in each case two supports are separated by a gap.

23. A support arrangement according to Claim 22, **characterised in that** at least one arrangement on a first support faces at least one arrangement on a second support.

24. A support arrangement according to Claim 22 or 23, **characterised in that** the gap has a width of approximately 0.01 mm to 10 mm, preferably of approximately 0.1 mm to 2 mm, and more preferably of approximately 0.5 mm to 1 mm.

25. The use of an arrangement according to one of Claims 13 to 18 and/or of a support according to one of Claims 19 to 21 and/or of a support arrangement according to one of Claims 22 to 24 in a process according to one of Claims 1 to 12.

## Revendications

1. Procédé de détermination de la spécificité de substrat d'une activité enzymatique comprenant les étapes suivantes consistant à :
- préparer un arrangement comprenant une pluralité de séquences d'acides aminés sur une surface plane d'un matériau de support, les séquences d'acides aminés étant immobilisées de manière adressée,
- mettre en contact et/ou incuber une activité enzymatique avec l'arrangement, et
- détecter une réaction entre une ou plusieurs des séquences d'acides aminés immobilisées sur l'arrangement et l'activité enzymatique,
**caractérisé en ce que** sous l'action de l'activité enzymatique avec l'arrangement, une modification du poids moléculaire d'au moins une des séquences d'acides aminés a lieu et la surface est une surface non poreuse.

2. Procédé selon la revendication 1, **caractérisé en ce que** la détection de la réaction a lieu au niveau de ou par l'utilisation des séquences d'acides aminés immobilisées sur la surface du matériau porteur.

3. Procédé selon la revendication 1 ou 2, **caractérisé en ce que** la modification du poids moléculaire a lieu par formation ou scission d'une liaison covalente au niveau d'une des séquences d'acides aminés, de préférence au niveau de la séquence d'acide aminé qui réagit avec l'activité enzymatique.

4. Procédé selon l'une des revendications 1 à 3, **caractérisé en ce que** la détection de la réaction a lieu par détection de la modification du poids moléculaire.

5. Procédé selon l'une des revendications 1 à 4, **caractérisé en ce que** la détection a lieu par un procédé de détection qui est choisi dans le groupe comprenant l'autoradiographie, la spectroscopie par résonance plasmon et la spectroscopie de fluorescence.

6. Procédé selon l'une des revendications 1 à 5, **caractérisé en ce qu'**au moins une des séquences d'acides aminés est un substrat pour une activité enzymatique.

7. Procédé selon l'une des revendications 1 à 6, **caractérisé en ce que** l'arrangement de séquences d'acides aminés présente pour au moins deux activités enzymatiques différentes respectivement au moins un substrat.

8. Procédé selon l'une des revendications 1 à 7, **caractérisé en ce que** l'activité enzymatique est choisie dans le groupe comprenant les oxydoréductases, les transférases, les hydrolases, les lysases et les ligases, et en particulier est choisie dans le groupe comprenant les kinases, les sulfotransférases, les glycosyltransférases, les acétyltransférases, les farnésyltransférases, les palmityltransférases, les phosphatases, les sulfatases, les estérases, les lipases, les acétylases et les protéases.

9. Procédé selon l'une des revendications 1 à 8, **caractérisé en ce que** la détection d'une réaction entre les séquences d'acides aminés immobilisées sur l'arrangement et l'activité enzymatique est répétée plusieurs fois, de préférence de manière espacée dans le temps.

10. Procédé selon l'une des revendications 1 à 9, **caractérisé en ce que** l'activité enzymatique est évaluée dans un échantillon et l'échantillon est de préférence choisi dans le groupe comprenant l'urine, le liquide céphalo-rachidien, la salive, les selles, le liquide lymphatique, d'autres liquides organiques, des lysats cellulaires, des lysats tissulaires, des lysats d'organe, des extraits, des extraits bruts, des préparations purifiées et des préparations non purifiées.

11. Procédé selon l'une des revendications 1 à 10, **caractérisé en ce que** le matériau de support est du verre.

12. Procédé selon l'une des revendications 1 à 11, **caractérisé en ce que** la séquence d'acides aminés est immobilisée sur la surface par un groupe comprenant un soufre.

13. Arrangement d'une pluralité de séquences d'acides aminés sur une surface, les séquences d'acides aminés étant immobilisées de manière dirigée sur la surface plane d'un matériau de support, **caractérisé en ce qu'**au moins une des séquences d'acides aminés représente un substrat pour une activité enzymatique, dans lequel a lieu sur le substrat, par l'activité enzymatique, une modification du poids moléculaire, et la surface étant une surface non poreuse.

14. Arrangement selon la revendication 13, **caractérisé en ce que** la modification du poids moléculaire a lieu par formation ou scission d'une liaison covalente au niveau du substrat.

15. Arrangement selon la revendication 13 ou 14, **caractérisé en ce que** l'arrangement de séquences d'acides aminés présente pour au moins deux activités enzymatiques différentes respectivement au moins un substrat.

16. Arrangement selon les revendications 13 à 15, **caractérisé en ce que** le matériau de support est choisi dans le groupe comprenant le silicate, la céramique, le verre, des métaux et des matériaux de support organiques.

17. Arrangement selon l'une des revendications 13 à 16, **caractérisé en ce que** les séquences d'acides aminés sont choisies dans le groupe comprenant des peptides, des oligopeptides, des polypeptides et des protéines et leurs dérivés.

18. Arrangement selon l'une des revendications 13 à 17, **caractérisé en ce qu'**une séquence d'acides aminés ou le groupe de séquences d'acides aminés sont arrangés de manière définie par rapport à une autre séquence d'acides aminés ou à un autre groupe de séquences d'acides aminés.

19. Support comprenant un arrangement selon l'une des revendications précédentes.

20. Support selon la revendication 19, **caractérisé en ce que** le support comprend un matériau de support de base.

21. Support selon la revendication 19 ou 20, **caractérisé en ce que** l'arrangement porte une pluralité de séquences d'acides aminés sur une ou plusieurs surfaces du support.

22. Arrangement de supports comprenant au moins deux supports selon l'une des revendications 19 à 21, dans lequel deux supports sont à chaque fois séparés par un espace.

23. Arrangement de supports selon la revendication 22, **caractérisé en ce qu'**au moins un arrangement sur un premier support est tourné vers au moins un arrangement sur un second support.

24. Arrangement de supports selon la revendication 22 ou 23, **caractérisé en ce que** l'espace présente une largeur d'environ 0,01 mm à 10 mm, de préférence d'environ 0,1 mm à 2 mm et de préférence d'environ 0,5 mm à 1 mm.

25. Utilisation d'un arrangement selon l'une des revendications 13 à 18 et/ou d'un support selon l'une des revendications 19 à 21 et/ou d'un arrangement de supports selon l'une des revendications 22 à 24 dans un procédé selon l'une des revendications 1 à 12.
